# EUROPÄISCHE PATENTSCHRIFT

(11) **EP 1 888 754 B1**
(45) Veröffentlichungstag und Bekanntmachung des Hinweises auf die Patenterteilung: **18.05.2011**
(21) Anmeldenummer: 06763391.7
(22) Anmeldetag: 30.05.2006
(51) Int. Cl.: C12N 15/82, A01H 5/00

(54) **PROMOTOR ZUR EPIDERMISSPEZIFISCHEN, PATHOGENINDUZIERBAREN TRANSGENEXPRESSION IN PFLANZEN**
PROMOTER FOR EPIDERMIS-SPECIFIC, PATHOGEN-INDUCIBLE TRANSGENIC EXPRESSION IN PLANTS
PROMOTEUR DE L'EXPRESSION DE TRANSGÈNES SPÉCIFIQUE À L'ÉPIDERME, POUVANT ÊTRE INDUITE PAR DES PATHOGÈNES DANS DES PLANTES

(30) Priorität: 03.06.2005 DE 102005025656
(43) Veröffentlichungstag der Anmeldung: 20.02.2008
(73) Patentinhaber: Leibniz-Institut für Pflanzengenetik und Kulturpflanzenforschung (IPK), 06466 Gatersleben (DE)
(72) Erfinder: SCHWEIZER, Patrick, 06493 Ballenstedt (DE); HIMMELBACH, Axel, 06502 Thale (DE); ALTSCHMIED, Lothar, 06484 Quedlinburg (DE); MAUCHER, Helmut, 06184 Kabelsketal (DE)
(74) Vertreter: Neuefeind, Regina
(86) Internationale Anmeldenummer: PCT/EP2006/062747
(87) Internationale Veröffentlichungsnummer: WO 2006/128882

(56) Entgegenhaltungen:
- EP-A- 0 368 167
- WO-A-02/057412
- WO-A-2005/035766
- WEI YANGDOU ET AL: "An epidermis/papilla-specific oxalate oxidase-like protein in the defence response of barley attacked by the powdery mildew fungus" PLANT MOLECULAR BIOLOGY, Bd. 36, Nr. 1, 1. Januar 1998 (1998-01-01), Seiten 101-112, XP002398590 ISSN: 0167-4412 -& DATABASE EMBL [Online] 21. November 1995 (1995-11-21), "H.vulgare mRNA for oxalate oxidase-like protein" XP002398649 gefunden im EBI accession no. EM_PRO:X93171 Database accession no. X93171
- DATABASE EMBL [Online] 2. April 2003 (2003-04-02), "Hordeum vulgare germin-like 8 (GL8) gene, complete cds." XP002398541 gefunden im EBI accession no. EM_PRO:AF493980 Database accession no. AF493980
- DATABASE EMBL [Online] 2. April 2003 (2003-04-02), "Hordeum vulgare germin-like 12 (GL12) gene, complete cds." XP002398540 gefunden im EBI accession no. EM_PRO:AF493981 Database accession no. AF493981
- DATABASE EMBL [Online] 20. Februar 2001 (2001-02-20), "Hordeum vulgare partial glp2 gene for germin-like protein 2" XP002398542 gefunden im EBI accession no. EM_PRO:AJ310534 Database accession no. AJ310534
- GJETTING TORBEN ET AL: "Differential gene expression in individual papilla-resistant and powdery mildew-infected barley epidermal cells" MOLECULAR PLANT-MICROBE INTERACTIONS, Bd. 17, Nr. 7, Juli 2004 (2004-07), Seiten 729-738, XP002398625 ISSN: 0894-0282
- CHRISTENSEN ANDERS B ET AL: "The germinlike protein GLP4 exhibits superoxide dismutase activity and is an important component of quantitative resistance in wheat and barley." MOLECULAR PLANT-MICROBE INTERACTIONS, Bd. 17, Nr. 1, Januar 2004 (2004-01), Seiten 109-117, XP002398626 ISSN: 0894-0282
- DRUKA ARNIS ET AL: "Physical and genetic mapping of barley (Hordeum vulgare) germin-like cDNAs" PROCEEDINGS OF THE NATIONAL ACADEMY OF SCIENCES OF THE UNITED STATES OF AMERICA, Bd. 99, Nr. 2, 22. Januar 2002 (2002-01-22), Seiten 850-855, XP002398629 ISSN: 0027-8424
- BERNA ANNE ET AL: "Regulation by biotic and abiotic stress of a wheat germin gene encoding oxalate oxidase, a H2O2-producing enzyme" PLANT MOLECULAR BIOLOGY, Bd. 39, Nr. 3, Februar 1999 (1999-02), Seiten 539-549, XP002398636 ISSN: 0167-4412
- ALTPETER FREDY ET AL: "Stable expression of a defense-related gene in wheat epidermis under transcriptional control of a novel promoter confers pathogen resistance" PLANT MOLECULAR BIOLOGY, Bd. 57, Nr. 2, Januar 2005 (2005-01), Seiten 271-283, XP002398634 ISSN: 0167-4412
- CANEVASCINI STEFANO ET AL: "Tissue-specific expression and promoter analysis of the tobacco Itp1 gene" PLANT PHYSIOLOGY (ROCKVILLE), Bd. 112, Nr. 2, 1996, Seiten 513-524, XP002398628 ISSN: 0032-0889

## Beschreibung

Die vorliegende Erfindung betrifft Promotorregionen, unter deren Kontrolle Transgene in Pflanzen epidermisspezifisch und pathogeninduzierbar exprimiert werden können. Weiterhin betrifft die Erfindung rekombinante Nukleinsäuremoleküle, die diese Promotorregionen umfassen und transgene Pflanzen und Pflanzenzellen, die mit diesen Nukleinsäuremolekülen transformiert wurden, sowie Verfahren zu deren Herstellung. Außerdem betrifft die vorliegende Erfindung Nukleinsäuremoleküle umfassend einen erfindungsgemäßen Promotor und Nukleinsäuresequenzen bzw. Transgene, die Pathogenresistenz vermitteln können sowie mit diesen Nukleinsäuremolekülen transformierte Pflanzen und Pflanzenzellen und Verfahren zu deren Herstellung. Die Erfindung betrifft auch die Verwendung der erfindungsgemäßen Promotorregion zur Untersuchung von pathogeninduzierten Signaltransduktionswegen in Pflanzenzellen.

Als Promotoren werden allgemein diejenigen DNA-Bereiche eines Gens bezeichnet, die stromaufwärts des Transkriptionsstartpunktes liegen und durch die der Initiationspunkt und die Initiationshäufigkeit der Transkription und somit die Expressionsstärke und das Expressionsmuster des kontrollierten Gens festgelegt werden. An die Promotoren binden die RNA-Polymerase und spezifische, die RNA-Polymerase aktivierende Transkriptionsfaktoren, um die Transkription zusammen mit dem basalen Transkriptionskomplex zu initiieren. Die Wirksamkeit der Promotoren wird häufig durch zusätzliche DNA-Sequenzen, die Enhancer-Sequenzen, gesteigert und reguliert, deren Position im Gegensatz zu der der Promotoren nicht festgelegt ist. Diese regulatorischen Elemente können stromaufwärts, stromabwärts oder in einem Intron des zu exprimierenden Gens liegen.

In der rekombinanten DNA-Technologie werden Promotoren in Expressionsvektoren eingesetzt, um die Expression eines Transgens zu steuern, das in der Regel nicht das natürlicherweise durch den Promotor regulierte Gen ist. Dabei kommt es wesentlich auf die Spezifität des Promotors an, die bestimmt, zu welchem Zeitpunkt, in welchen Gewebetypen und in welcher Intensität ein gentechnisch übertragenes Gen exprimiert wird.

In der Pflanzenzucht wird die rekombinante DNA-Technologie häufig eingesetzt, um bestimmte nützliche Eigenschaften auf Nutzpflanzen zu übertragen, was zu einem höheren Ertrag, z. B. durch erhöhte Pathogenresistenz, oder zu verbesserten Eigenschaften der Ernteprodukte führen soll. Dabei ist es häufig wünschenswert, dass das übertragene Gen nicht ubiquitär exprimiert wird, sondern nur in den Geweben, in denen die Transgenaktivität gewünscht wird, da sich die Anwesenheit des Transgenprodukts in manchen Geweben negativ auf normale physiologische Prozesse auswirken kann. So konnte etwa gezeigt werden, dass die Überexpression einer anionischen Peroxidase unter der Kontrolle des 35S-Promotors zum Welken von transgenen Tabakpflanzen führt, weil weniger Wurzelwachstum stattfindet und sich daher auch weniger Wurzelmasse bildet (Lagrimini et al. (1997) Plant Mol Biol. 33 (5): 887-895). Die Überexpression der spi2-Peroxidase unter der Kontrolle des Ubiquitin-Promotors führt zu einer reduzierten Epicotylbildung und einem reduzierten Längenwachstum verglichen mit Kontrollpflanzen (Elfstrand, M. et al. (2001) Plant Cell Reports 20 (7): 596-603). Abgesehen von negativen Effekten auf physiologische Prozesse soll in der Resistenzzüchtung häufig vermieden werden, dass das Transgenprodukt auch in den geernteten Pflanzenteilen vorliegt.

Deshalb wurden in den vergangenen Jahren Promotoren isoliert, die entweder gewebespezifisch oder induzierbar wirken. Zu den gewebespezifischen Promotoren gehören etwa samen-, knollen- und fruchtspezifische Promotoren. Die induzierbaren Promotoren können beispielsweise durch chemische Induktion, durch Lichtinduktion oder andere Stimuli aktiviert werden.

Es ist auch wünschenswert, die Genexpression spezifisch in der Epidermis, zu modulieren. Die Epidermis stellt das Abschlussgewebe der oberirdischen Organe höherer Pflanzen dar. Als solches bestehen die Aufgaben der Epidermis darin, einerseits den Wasser- und Stoffaustausch der Pflanze zu ermöglichen und andererseits das Eindringen von Pathogenen in die Pflanze zu verhindern. Durch eine veränderte Genexpression in der Epidermis mit Hilfe geeigneter Promotoren und von ihnen gesteuerter Gene könnten diese Funktionen gezielt moduliert werden.

Epidermisspezifische Promotoren wurden in dikotyledonen Pflanzen bereits beschrieben. So konnte gezeigt werden, dass der Promotor des CER6- (CUT1-) Gens aus Arabidopsis, das für ein kondensierendes Enzym bei der Wachssynthese kodiert, die epidermisspezifische Expression eines β-Glucuronidase-Reportergens bewirken kann (Hooker et al. (2002) Plant Physiol. 129(4): 1568-1580; Kunst et al. (2000) Biochem. Soc. Trans. 28(6): 651-654).

Jedoch sind bisher nur wenige geeignete epidermisspezifische Promotoren in monokotyledonen Pflanzen identifiziert worden, die sich für die Expression von Transgenen in Monokotyledonen, insbesondere Poaceen (Süßgräsern), besonders gut eignen. Kürzlich wurde ein Promotor beschrieben, der aus dem Promotor des GSTA1-Gens und dem Intron des WIR1a-Gens zusammengesetzt ist und eine konstitutive epidermisspezifische Aktivität aufweist (DE 103 46 611 A1).

Die internationale Patentanmeldung WO 02/057412 A2 offenbart verschiedene Germingene, die für Oxalatoxidasen kodieren, sowie die Promotoren dieser Gene.

Da bisher nur wenige geeignete Promotoren für die epidermisspezifische Transgenexpression bekannt waren, wurden meist konstitutive Promotoren wie der Ubiquitin-Promotor aus Mais verwendet, um Proteine in der Epidermis zu exprimieren (siehe z. B. Oldach et al. (2001) Mol Plant Microbe Interact. 14(7): 832-838). Dies kann aber, wie oben beschrieben, zu unerwünschten Nebeneffekten bei den transgenen Pflanzen aufgrund der Anwesenheit des Transgenprodukts in anderen Geweben bzw. Organen als der Epidermis führen.

Aufgabe der Erfindung ist es daher, Mittel bereitzustellen, die eine epidermisspezifische und pathogeninduzierbare Genexpression in Monokotyledonen, bevorzugt in Getreidepflanzen, ermöglichen.

Diese Aufgabe wird gelöst durch die Bereitstellung der in den Patentansprüchen charakterisierten Ausführungsformen.

Somit betrifft die vorliegende Erfindung eine pathogeninduzierbare Promotorregion mit Spezifität für die pflanzliche Epidermis, die ausgewählt ist aus der Gruppe bestehend aus
a) Promotorregionen, die die unter SEQ ID Nr. 1 oder 16 angegebene Nukleinsäuresequenz umfassen,

Bevorzugt umfasst die erfindungsgemäße Promotorregion eine zusätzliche Sequenz, die aus dem Intron des WIR1a-Gens stammt und ausgewählt ist aus der Gruppe bestehend aus
a) Sequenzen, die die unter SEQ ID Nr. 2 angegebene Nukleinsäuresequenz umfassen, und
b) Sequenzen, die unter stringenten Bedingungen mit der unter SEQ ID Nr. 2 angegebenen Nukleinsäuresequenz hybridisieren.

Die zusätzliche Sequenz mit der SEQ ID Nr. 2 kann sowohl am 5'- als auch am 3'-Ende der in SEQ ID Nr. 1 oder 16 angegebenen Nukleinsäuresequenz liegen. Bevorzugt liegt sie am 3'-Ende der in SEQ ID Nr. 1 oder 16 angegebenen Nukleinsäuresequenz. Wenn die zusätzliche Sequenz vorhanden ist, wird die Aktivität des erfindungsgemäßen Promotors erhöht.

Zwischen der ersten und der zweiten Sequenz können weitere, untranslatierte Sequenzen liegen, die eine Länge von 10bp bis 1000bp, bevorzugt von 20bp bis 800bp, besonders bevorzugt von 30bp bis 500bp und am meisten bevorzugt zwischen 40bp und 300bp aufweisen.

Besonders bevorzugt handelt es sich bei der erfindungsgemäßen Promotorregion um eine Promotorregion, ausgewählt aus der Gruppe bestehend aus
a) Promotorregionen, die die unter SEQ ID Nr. 3 oder 17 angegebene Nukleinsäuresequenz umfassen;
b) Promotorregionen, die einen funktionalen Teil der unter SEQ ID Nr. 3 oder 17 angegebenen Nukleinsäuresequenz umfassen oder
c) Promotorregionen, die eine Sequenz aufweisen, die unter stringenten Bedingungen mit der unter SEQ ID Nr. 3 oder 17 angegebenen Nukleinsäuresequenz hybridisiert.

Im Rahmen der vorliegenden Erfindung wird unter einer "Promotorregion" eine Nukleinsäuresequenz verstanden, die die zur Expression einer kodierenden Sequenz (Transgen) notwendigen regulatorischen Sequenzen umfasst. Regulatorische Sequenzen bilden denjenigen Teil eines Gens, der die Expression einer kodierenden Sequenz bestimmt, also vor allem das Expressionsniveau und -muster. Die regulatorischen Sequenzen besitzen mindestens ein Sequenzmotiv, an das spezifische Transkriptionsfaktoren und die RNA-Polymerase binden, zum Transkriptionskomplex assemblieren und die Transkription der von der Promotorregion kontrollierten Nukleinsäuresequenz wirksam initiieren.

Unter dem Begriff "epidermisspezifisch" wird im Rahmen der vorliegenden Erfindung verstanden, dass eine unter der Kontrolle der erfindungsgemäßen Promotorregion stehende Nukleinsäuresequenz bevorzugt in der Sprossepidermis von Pflanzen exprimiert wird. Insbesondere ist Epidermisspezifität im Sinne der vorliegenden Erfindung auch dann gegeben, wenn die erfindungsgemäße Promotorregion die Expression eines Fremdgens in der Epidermis im Vergleich zu anderen Zelltypen begünstigt und in der Epidermis im Vergleich zu anderen Zelltypen eine signifikant, wie mindestens 2-fach, bevorzugt mindestens 5- fach und besonders bevorzugt mindestens 10- und am meisten bevorzugt 50-fach erhöhte Expression bewirkt. Die Expressionshöhe kann mit üblichen *in situ*-Nachweistechniken bestimmt werden.

Der Begriff "pflanzliche Epidermis" ist dem Fachmann geläufig. Ergänzende Informationen sind in jedem Pflanzenanatomie oder -physiologiebuch zu finden, wie etwa in Strasburger, Lehrbuch der Botanik, 35. Auflage 2002, Spektrum Akademischer Verlag.

Der Begriff "pathogeninduzierbar" bedeutet im Rahmen der Erfindung, dass durch Kontakt der Pflanze bzw. der Pflanzenzelle mit einem Pathogen die Expression einer unter der Kontrolle der erfindungsgemäßen Promotorregion stehenden Nukleinsäuresequenz induziert wird. Insbesondere ist die Pathogeninduzierbarkeit im Sinne der vorliegenden Erfindung auch dann gegeben, wenn die erfindungsgemäße Promotorregion die Expression eines Fremdgens in der Pflanze/Pflanzenzelle im Vergleich zu Pflanzen/Pflanzenzellen, die nicht im Kontakt mit einem Pathogen sind, signifikant, wie mindestens 2-fach, bevorzugt mindestens 5-fach, besonders bevorzugt mindestens 10- und am meisten bevorzugt 50-fach erhöht. Die Expressionshöhe kann mit üblichen *in situ*-Nachweistechniken bestimmt werden.

Bei den Pathogenen, die die Aktivität der erfindungsgemäßen Promotorregion steigern, kann es sich prinzipiell um jedes Pathogen handeln, bevorzugt handelt es sich um *Blumeria graminis, Rynchosporium secalis* oder *Cochliobolus sativus.*

Die vorliegende Erfindung betrifft auch Promotorregionen, die die funktionalen Teile der erfindungsgemäßen Sequenz aufweisen und die in Pflanzen eine epidermisspezifische und pathogeninduzierbare Expression einer von ihnen kontrollierten kodierenden Nukleinsäuresequenz bewirken.

Unter einem "funktionalen Teil" werden in diesem Zusammenhang Sequenzen verstanden, an die trotz leicht abweichender Nukleinsäuresequenz noch der Transkriptionskomplex binden und die epidermisspezifische und pathogeninduzierbare Expression bewirken kann. Funktionale Teile einer Promotorsequenz umfassen auch solche Promotorvarianten, deren Promotoraktivität, verglichen mit dem Wildtyp, abgeschwächt oder verstärkt ist. Unter einem funktionalen Teil versteht man auch natürliche oder künstliche Varianten der in SEQ ID Nr. 3 oder 17 angegebenen Sequenz der Promotorregion. Mutationen umfassen Substitutionen, Additionen, Deletionen, Vertauschungen und/oder Insertionen eines oder mehrerer Nukleotidreste. Funktionale Teile der Promotorregionen umfassen im Rahmen der vorliegenden Erfindung natürlich vorkommende Varianten der SEQ ID Nr. oder 17 sowie künstliche, z. B. durch chemische Synthese erhaltene Nukleotidsequenzen. Der "funktionale Teil" weist eine epidermisspezifische und pathogeninduzierbare Promotoraktivität von 10%, 20%, 30% oder 40%, bevorzugt 50%, 60% oder 70%, besonders bevorzugt 80%, 90% oder 100% und am meisten bevorzugt von 120%, 150% oder 200% oder mehr der Aktivität der Wildtyp-Promotorsequenz auf.

Durch Deletionsexperimente wurde herausgefunden, dass die für die Pathogeninduzierbarkeit verantwortlichen Sequenzmotive über die gesamte Promotorsequenz gemäß SEQ ID No. 1 verteilt liegen, wobei vermutet wird, dass im 5'-Bereich vor allem Enhancermotive liegen.

Gemessen werden kann die Promotoraktivität von Varianten der Promotorregion mit Hilfe von Reportergenen, deren kodierende Sequenz unter der Kontrolle der zu untersuchenden Promotorregion steht. Geeignete Reportergene sind beispielsweise das β-Glucuronidase-(GUS)-Gen aus *E. coli*, ein Fluoreszenzgen wie etwa das Green-Fluorescence-Protein (GFP)-Gen aus *Aequoria victoria*, das Luziferase-Gen aus *Photinus pyralis* oder das β-Galaktosidase-(lacZ)-Gen aus E. coli. Die absolute Promotoraktivität wird bestimmt durch Vergleich mit einer Wildtyp-Pflanze. Die Gewebe- bzw. Zellspezifität lässt sich leicht durch Vergleich der Expressionsraten der oben genannten Reportergene in den jeweiligen Geweben bzw. Zellen bestimmen. Die Induzierbarkeit lässt sich durch Vergleich der Expressionsraten der oben genannten Reportergene in behandelten und nicht-behandelten Pflanzen bestimmen.

Descrieben werden ebenfalls Promotorregionen mit einer Nukleinsäuresequenz, die mit den unter SEQ ID Nr. 1, 3, 16 oder 17 angegebenen Nukleinsäuresequenzen unter stringenten Bedingungen hybridisiert. Der Begriff "Hybridisierung unter stringenten Bedingungen" bedeutet im Zusammenhang dieser Erfindung, dass die Hybridisierung *in vitro* unter Bedingungen durchgeführt wird, die stringent genug sind, um eine spezifische Hybridisierung zu gewährleisten. Solche stringenten Hybridisierungsbedingungen sind dem Fachmann bekannt und können der Literatur entnommen werden (Sambrook et al. (2001), Molecular Cloning: A Laboratory Manual, 3. Auflage, Cold Spring Harbor Laboratory Press, Cold Spring Harbor, New York).

Allgemein bedeutet "spezifisch hybridisieren", dass ein Molekül unter stringenten Bedingungen präferenziell an eine bestimmte Nukleotidsequenz bindet, wenn diese Sequenz in einem komplexen Gemisch von (z. B. Gesamt-) DNA oder RNA vorliegt. Der Begriff "stringente Bedingungen" steht allgemein für Bedingungen, unter denen eine Nukleinsäuresequenz präferenziell an ihre Zielsequenz hybridisieren wird, und zu einem deutlich geringeren Ausmaß oder gar nicht an andere Sequenzen. Stringente Bedingungen sind z. T. Sequenz-abhängig und werden unter verschiedenen Umständen unterschiedlich sein. Längere Sequenzen hybridisieren spezifisch bei höheren Temperaturen. Im Allgemeinen werden stringente Bedingungen so ausgewählt, dass die Temperatur etwa 5°C unter dem thermischen Schmelzpunkt (Tₘ) für die spezifische Sequenz bei einer definierten Ionenstärke und einem definierten pH liegt. Die Tₘ ist die Temperatur (unter definierter Ionenstärke, pH und Nukleinsäurekonzentration), bei der 50% der zu der Zielsequenz komplementären Moleküle zu der Zielsequenz im Gleichgewichtszustand hybridisieren. Typischerweise sind stringente Bedingungen solche, bei denen die Salzkonzentration mindestens ungefähr 0,01 bis 1,0 M Natriumionen-Konzentration (oder ein anderes Salz) bei einem pH zwischen 7,0 und 8,3 beträgt und die Temperatur mindestens 30 °C für kurze Moleküle (also z. B. 10-50 Nukleotide) beträgt. Zusätzlich können stringente Bedingungen durch Zugabe destabilisierender Agenzien, wie beispielsweise Formamid, erreicht werden.

Geeignete stringente Hybridisierungsbedingungen sind beispielsweise auch beschrieben in Sambrook et al.,vide supra. So kann die Hybridisierung etwa unter den folgenden Bedingungen stattfinden:
- Hybridisierungspuffer: 2x SSC, 10x Denhardt's Lösung (Fikoll 400 + PEG + BSA; Verhältnis 1:1:1), 0,1% SDS, 5mM EDTA, 50mM Na₂HPO₄, 250µg/ml Heringssperma-DNA; 50µg/ml tRNA oder 0,25M Natriumphosphatpuffer pH 7,2, 1mM EDTA, 7% SDS bei einer Hybridisierungstemperatur von 65°C bis 68°C
- Waschpuffer: 0,2x SSC, 0,1% SDS bei einer Waschtemperatur von 65°C bis 68°C

Derartige Promotorvarianten weisen eine Sequenzidentität von mindestens 90% und am meisten bevorzugt mindestens 95% zu der unter SEQ ID Nr. 1, 3, 16 oder 17 angegebenen Promotorsequenz auf, bezogen auf die gesamte in SEQ ID Nr. 1, 3, 16 oder 17 gezeigte DNA-Sequenz. Vorzugsweise wird die Sequenzidentität derartiger Promotorsequenzen durch Vergleich mit der unter SEQ ID Nr. 1, 3, 16 oder 17 angegebenen Nukleinsäuresequenz bestimmt. Wenn zwei unterschiedlich lange Nukleinsäuresequenzen miteinander verglichen werden, bezieht sich die Sequenzidentität vorzugsweise auf den prozentualen Anteil der Nukleotidreste der kürzeren Sequenz, die identisch sind mit den entsprechenden Nukleotidresten der längeren Sequenz.

Sequenzidentitäten werden üblicherweise über verschiedene Alignment-Programme, wie z. B. CLUSTAL festgestellt. Allgemein stehen dem Fachmann zur Bestimmung der Sequenzidentität geeignete Algorithmen zur Verfügung, z. B. auch das Programm, das unter http://www.ncbi.nlm.nih.gov/BLAST (z. B. der Link "Standard nucleotide-nucleotide BLAST [blastn]") zugänglich ist.

Die vorliegende Erfindung betrifft auch chimäre Gene aus der erfindungsgemäßen Promotorregion und einer kodierenden Sequenz, deren Expression, die natürlicherweise nicht durch die erfindungsgemäße Promotorregion reguliert wird, durch die erfindungsgemäße Promotorregion reguliert wird, in operativer Verknüpfung sowie rekombinante Nukleinsäuremoleküle, die diese chimären Gene enthalten.

Der Begriff "Nukleinsäuresequenz, deren Expression durch die erfindungsgemäße Promotorregion reguliert wird" bedeutet, dass die Expression der Nukleinsäuresequenz unter der Kontrolle der erfindungsgemäßen Promotorregion in den Zellen und unter den Bedingungen, in denen die Promotorregion aktiv ist, um mindestens den Faktor fünf, bevorzugt mindestens den Faktor 10 und besonders bevorzugt mindestens den Faktor 50 gegenüber Wildtyp-Zellen gesteigert werden kann.

Bei der Nukleinsäuresequenz, deren Expression durch die erfindungsgemäße Nukleinsäuresequenz reguliert wird, kann es sich um die kodierende Region eines Transgens handeln, z. B. eines Resistenzgens, dessen Genprodukt in der Epidermis erwünscht ist. Durch die Expression des Transgens kann der Gehalt des von ihm kodierten Genprodukts mindestens um den Faktor 2, bevorzugt mindestens um den Faktor 5, besonders bevorzugt mindestens um den Faktor 10 und am meisten bevorzugt mindestens um den Faktor 50 erhöht werden.

Die erfindungsgemäße Promotorregion kann aber auch in RNAi-Konstrukten zur RNA-Interferenz eingesetzt werden, um das epidermisspezifische und pathogeninduzierbare Silencing bestimmter Gene zu erreichen, deren Genprodukte in der Epidermis nicht oder in geringerem Ausmaß als üblich anwesend sein sollen und deren Expression durch Befall der Pflanze mit einem Pathogen nicht induziert werden soll. Letzteres kann natürlich auch mit klassischen antisense- oder Kosuppressionskonstrukten unter Einsatz der erfindungsgemäßen Promotorregionen erreicht werden. Die Expression des endogenen Gens wird durch die Silencing-Konstrukte um mindestens 50%, bevorzugt um mindestens 70%, besonders bevorzugt um mindestens 90% und am meisten bevorzugt um mindestens 95% verringert.

In einem Konstrukt, das zur RNA-Interferenz verwendet werden soll, liegen üblicherweise palindromische DNA-Sequenzen vor, die nach der Transkription doppelsträngige RNA bilden. Diese doppelsträngige RNA wird durch das Dicer-Enzym zu kürzeren RNA-Stücken prozessiert, die an eine endogene RNA binden und deren Abbau mit Hilfe des RISC (RNA-induced silencing complex) bewirken (Hannon (2002) Nature 418: 244-251).

Der Effekt der Gen-Silencing-Konstrukte auf die Expression des endogenen Gens kann mit Hilfe gängiger molekularbiologischer Methoden nachgewiesen werden, die dem Fachmann bekannt sind. So stehen zur Untersuchung des RNA-Levels Northern-Blot- und RT-PCR-Verfahren zur Verfügung, das Protein kann durch Western-Blot-Analysen, Immunfluoreszenzen oder, sofern es sich bei dem Protein um ein Enzym handelt, Enzymassays nachgewiesen werden.

Unter dem Begriff "Transgen" werden im Rahmen der vorliegenden Erfindung diejenigen Gene zusammengefasst, deren Genprodukte in der Epidermis pathogeninduzierbar bereitgestellt werden sollen, bzw. beim Gen-Silencing unterdrückt werden sollen.

Bei der Nukleinsäuresequenz, deren Expression unter der Kontrolle des erfindungsgemäßen Promotors steht, handelt es sich bevorzugt um eine Nukleinsäuresequenz, die Pathogenresistenz vermittelt, da die Epidermis die erste Barriere darstellt, die von einem Pathogen beim Eindringen in die Pflanze überwunden werden muss.

In einem chimären Gen oder einem rekombinanten Expressionsvektor bedeutet "operativ daran gebunden", dass die Nukleotidsequenz von Interesse derart an die Regulationssequenz(en) gebunden ist, dass die Expression der Nukleotidsequenz möglich ist und die beiden Sequenzen derart aneinander gebunden sind, dass sie die vorhergesagte, der Sequenz zugeschriebene Funktion erfüllen.

Im Rahmen der vorliegenden Erfindung wird unter dem Begriff "rekombinantes Nukleinsäuremolekül" ein Vektor verstanden, der ein erfindungsgemäßes chimäres Gen oder eine erfindungsgemäße Promotorregion enthält und die promotorabhängige Expression der unter der Kontrolle der erfindungsgemäßen Promotorregion stehenden Nukleinsäuresequenz in Pflanzenzellen und Pflanzen bewirken kann. In einer bevorzugten Ausführungsform enthält ein erfindungsgemäBes rekombinantes Nukleinsäuremolekül zusätzlich transkriptionelle Terminationssequenzen. Unter "transkriptionellen Terminationssequenzen" werden dabei DNA-Sequenzen verstanden, die am stromabwärts-Ende einer kodierenden Sequenz liegen und die RNA-Polymerase zum Stoppen der Transkription veranlassen.

Weiterhin betrifft die Erfindung Verfahren zur Herstellung transgener Pflanzen mit epidermis spezifischer und pathogeninduzierbarer Expression einer durch die erfindungsgemäße Promotorregion regulierten Nukleinsäuresequenz, umfassend die Schritte:
a) Herstellung eines rekombinanten Nukleinsäuremoleküls, in der die erfindungsgemäße Promotorregion in operativer Verknüpfung mit einer kodierenden Sequenz vorliegt,
b) Übertragung des Nukleinsäuremoleküls aus a) auf pflanzliche Zellen und
c) Regeneration vollständig transformierter Pflanzen und, falls erwünscht, Vermehrung der Pflanzen.

Zur Vorbereitung der Einführung fremder Gene in höhere Pflanzen bzw. deren Zellen stehen eine große Anzahl von Klonierungsvektoren zur Verfügung, die ein Replikationssignal für *E. coli* und ein Markergen zur Selektion transformierter Bakterienzellen enthalten. Beispiele für derartige Vektoren sind pBR322, pUC-Serien, M13mp-Serien, pACYC184 usw. Das chimäre Gen kann an einer passenden Restriktionsschnittstelle in den Vektor eingeführt werden. Das erhaltene Plasmid wird dann für die Transformation von *E. coli*-Zellen verwendet. Transformierte E. coli-Zellen werden in einem geeigneten Medium gezüchtet und anschließend geerntet und lysiert, und das Plasmid wird wiedergewonnen. Als Analysemethoden zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid-DNA gespalten und daraus gewonnene DNA-Fragmente mit anderen DNA-Sequenzen verknüpft werden.

Wie bereits erwähnt, stehen für die Einführung von DNA in eine pflanzliche Wirtszelle eine Vielzahl von Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von *Agrobacterium tumefaciens* oder *Agrobacterium rhizogenes* als Transformationsmedium, die Fusion von Protoplasten, die Injektion, die Elektroporation, den direkten Gentransfer isolierter DNA in Protoplasten, die Einbringung von DNA mittels biolistischer Methoden sowie weitere Möglichkeiten, die bereits seit mehreren Jahren gut etabliert sind und zum üblichen Repertoire des Fachmanns in der pflanzlichen Molekularbiologie bzw. Ptlanzenbiotechnologie gehören. Die biolistische Gentransfermethode wird vor allem bei monokotyledonen Pflanzen verwendet. Hier findet der Fachmann nützliche Informationen zur Durchführung z.B. in Vasil et al. (1992) Bio/Technology 10: 667-674; Vasil et al. (1993) Bio/Technology 11: 1153-1158; Nehra et al. (1994) Plant J. 5: 285-297; Becker et al. (1994) Plant J. 5: 299-307; Altpeter et al. (1996) Plant Cell Reports 16: 12-17; Ortiz et al. (1996) Plant Cell Reports 15: 877-81; Rasco-Gaunt et al. (2001) J. Exp. Bot. 52: 865-874.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide, wie z.B. pUC-Derivate verwendet werden.

Sollen aber aus derartig transformierten Zellen ganze Pflanzen regeneriert werden, ist die Anwesenheit eines selektierbaren Markergens empfehlenswert. Dem Fachmann sind die gängigen Selektionsmarker bekannt, und es stellt für ihn kein Problem dar, einen geeigneten Marker auszuwählen.

Je nach Einführungsmethode der gewünschten Gene in die Pflanzenzelle können weitere DNA-Sequenzen erforderlich sein. Wird z.B. zur Transformation der Pflanzenzelle das Ti- oder Ri-Plasmid verwendet, so muss mindestens die rechte Begrenzung, häufig jedoch die rechte und linke Begrenzung der im Ti- bzw. Ri-Plasmid enthaltenen T-DNA als Flankenbereich mit den einzuführenden Genen verbunden werden. Werden zur Transformation Agrobakterien verwendet, muss die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können allerdings nicht in Agrobakterien replizieren. Mittels eines Helferplasmids kann der intermediäre Vektor auf *Agrobacterium tumefaciens* übertragen werden (Konjugation). Binäre Vektoren dagegen können sowohl in *E. coli* als auch in Agrobakterien replizieren. Sie enthalten ein Selektionsmarkergen und einen Linker oder Polylinker, welche von der rechten und linken T-DNA-Grenzregion eingerahmt werden. Sie können direkt in die Agrobakterien transformiert werden. Das als Wirtszelle dienende Agrobakterium soll ein Plasmid enthalten, welches das chimäre Gen innerhalb der T-DNA trägt, welche in die Pflanzenzelle übertragen wird. Zusätzliche T-DNA kann vorhanden sein. Das derartig transformierte Agrobakterium wird zur Transformation von Pflanzenzellen verwendet. Die Verwendung von T-DNA für die Transformation von Pflanzenzellen ist intensiv untersucht und ausreichend in allseits bekannten Übersichtsartikeln und Handbüchern zur Pflanzentransformation beschrieben worden.

Für monokotyledone Pflanzen müssen für einen effektiven Agrobakteriumvermittelten Gentransfer abgewandelte Protokolle angewandt werden, wie sie etwa in Cheng et al. (1997) Plant Physiol. 115: 971-980; Khanna and Daggard (2003) Plant Cell Reports 21: 429-436; Wu et al. (2003) Plant Cell Reports 21: 659-668; Hu et al. (2003) Plant Cell Reports 21: 1010-1019, beschrieben sind. Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden.

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin, Methotrexat, Glyphosat, Streptomycin, Sulfonylharnstoff, Gentamycin oder Phosphinotricin u.a. vermittelt. Der individuell gewählte Marker sollte daher die Selektion transformierter Zellen gegenüber Zellen, denen die eingeführte DNA fehlt, gestatten. Hierzu sind auch alternative Marker geeignet, wie nutritive Marker oder Screeningmarker (wie GFP, green fluorescent protein). Selbstverständlich kann auch vollkommen auf Selektionsmarker verzichtet werden, was allerdings mit einem ziemlich hohen Screeningbedarf einhergeht. Falls markerfreie transgene Pflanzen erwünscht sind, stehen dem Fachmann auch Strategien zur Verfügung, die eine nachträgliche Entfernung des Markergens erlauben, z.B. Cotransformation oder Sequenzspezifische Rekombinasen.

Die Regeneration der transgenen Pflanzen aus transgenen Pflanzenzellen erfolgt nach üblichen Regenerationsmethoden unter Verwendung bekannter Nährmedien. Die so erhaltenen Pflanzen können dann mittels üblicher Verfahren, einschließlich molekularbiologischer Methoden, wie PCR, Blot-Analysen, auf Anwesenheit und Gewebespezifität der eingeführten Nukleinsäuresequenz, deren Expression von dem erfindungsgemäßen Promotor kontrolliert wird, bzw. der von ihr beeinflussten endogenen RNAs und Proteine untersucht werden.

Ferner betrifft die Erfindung transgene Pflanzen, die eine durch die erfindungsgemäße Promotorregion regulierte Nukleinsäuresequenz enthalten und diese epidermisspezifisch und pathogeninduzierbar exprimieren.

Bei den erfindungsgemäßen Pflanzen handelt es sich bevorzugt um Monokotyledonen, insbesondere Getreidepflanzen wie Roggen, Mais und Hafer, besonders bevorzugt um Weizen oder Gerste, sowie transgene Teile dieser Pflanzen und deren transgenes Vermehrungsmaterial, wie Protoplasten, Pflanzenzellen, Kalli, Samen, Knollen oder Stecklinge, sowie die transgenen Nachkommen dieser Pflanze. Aber auch für andere Poaceen (Süßgräser) wie z. B. Futtergräser kann die erfindungsgemäße Promotorregion zur Herstellung entsprechender Pflanzen mit epidermisspezifischer und pathogeninduzierbarer Expression von Transgenen eingesetzt werden.

Unter der Kontrolle des erfindungsgemäßen, epidermisspezifischen Promotors werden bevorzugt Pathogen-Resistenzgene unter der Kontrolle des erfindungsgemäßen Promotors exprimiert.

Als Pflanzenpathogene werden unter anderem Bakterien, Viren, Tierpathogene und Pilze bezeichnet, die Pflanzen infizieren und dadurch den Stoffwechsel der Pflanze negativ beeinflussen. Bevorzugt vermittelt ein unter der Kontrolle der erfindungsgemäßen Promotorsequenz stehendes Resistenzgen eine Resistenz gegen Pilzpathogene wie beispielsweise Mehltau. Es ist aber anzunehmen, dass die unter der Kontrolle der erfindungsgemäßen Sequenz exprimierten Gene auch eine Resistenz gegen weitere Pathogene bewirken.

Pilzpathogene oder pilz-ähnliche Pathogene (wie z.B. Chromista) stammen vorzugsweise aus der Gruppe umfassend Plasmodiophoramycota, Oomycota, Ascomycota, Chytridiomyceten, Zygomyceten, Basidiomycota und Deuteromyceten (Fungi imperfecti). Beispielhaft, jedoch nicht einschränkend, seien die in Tabelle 1 genannten Pilzpathogene und die mit ihnen in Zusammenhang gebrachten Erkrankungen zu nennen.

**Tabelle 1: Pflanzliche Pilzerkrankungen**

| **Erkrankung** | **Pathogen** |
|---|---|
| Braunrost | *Puccinia recondita* |
| Gelbrost | *P. striiformis* |
| Echter Mehltau | *Erysiphe graminis* /*Blumeria graminis* |
| Spelzenbräune | *Septoria nodorum* |
| Blattdürre | *Septoria tritici* |
| Ährenfusariosen | *Fusarium spp.* |
| Hahnbruchkrankheit | *Pseudocercosporella herpotrichoides* |
| Flugbrand | *Ustilago spp.* |
| Weizensteinbrand | *Tilletia caries* |
| Schwarzbeinigkeit | *Gaeumannomyces graminis* |
| Anthracnose leaf blight | *Colletotrichum graminicola* (teleomorph: *Glomerella graminicola Politis*); *Glomerella* |
| Anthracnose stalk rot | *tucumanensis* (anamorph: *Glomerella falcatum Went*) |
| Aspergillus ear and kernel rot | *Aspergillus flavus* |
| Banded leaf and sheath spot ("Wurzeltöter") | *Rhizoctonia solani Kuhn = Rhizoctonia microsclerotia J. Matz* (telomorph: *Thanatephorus cucumeris*) |
| Black bundle disease | *Acremonium strictum W. Gams = Cephalosporium acremonium Auct. non Corda* |
| Black kernel rot | *Lasiodiplodia theobromae = Botryodiplodia theobromae* |
| Borde blanco | *Marasmiellus sp.* |
| Brown spot (black spot, stalk rot) | *Physoderma maydis* |
| Cephalosporium kernel rot | *Acremonium strictum = Cephalosporium acremonium* |
| Charcoal rot | *Macrophomina phaseolina* |
| Corticium ear rot | *Thanatephorus cucumeris = Corticium sasakii* |
| Curvularia leaf spot | *Curvularia clavata, C. eragrostidis*, *= C. maculans* (teleomorph: *Cochliobolus eragrostidis*), *Curvularia inaequalis, C. intermedia* (teleomorph: *Cochliobolus intermedius*), *Curvularia lunata* (teleomorph: *Cochliobolus lunatus*), *Curvularia pallescens* (teleomorph: *Cochliobolus pallescens*), *Curvularia senegalensis, C. tuberculata* (teleomorph: *Cochliobolus tuberculatus)* |
| Didymella leaf spot | *Didymella exitalis* |
| Diplodia ear rot and stalk rot | *Diplodia frumenti* (teleomorph: *Botryosphaeria festucae*) |
| Diplodia ear rot, stalk rot, seed rot and seedling blight | *Diplodia maydis* = *Stenocarpella maydis* |
| Diplodia leaf spot or streak | *Stenocarpella macrospora* = |
| | *Diplodia macrospora* |
| Brown stripe downy mildew | *Sclerophthora rayssiae var. zeae* |
| Crazy top downy mildew | *Sclerophthora macrospora = Sclerospora macrospora* |
| Green ear downy mildew (graminicola downy mildew) | *Sclerospora graminicola* |
| Java downy mildew | *Peronosclerospora maydis = Sclerospora maydis* |
| Philippine downy mildew | *Peronosclerospora philippinensis = Sclerospora philippinensis* |
| Sorghum downy mildew | *Peronosclerospora sorghi = Sclerospora sorghi* |
| Spontaneum downy mildew | *Peronosclerospora spontanea = Sclerospora spontanea* |
| Sugarcane downy mildew | *Peronosclerospora sacchari = Sclerospora sacchari* |
| Dry ear rot (cob, kernel and stalk rot) | *Nigrospora oryzae* (teleomorph: *Khuskia oryzae*) |
| Ear rots, minor | *Alternaria alternata = A. tenuis*, |
| | *Aspergillus glaucus, A. niger,* |
| | *Aspergillus spp., Botrytis cinerea* (teleomorph: |
| | *Botryotinia fuckeliana), Cunninghamella sp.,* |
| | *Curvularia pallescens,* |
| | *Doratomyces stemonitis =* |
| | *Cephalotrichum stemonitis,* |
| | *Fusarium culmorum,* |
| | *Gonatobotrys simplex,* |
| | *Pithomyces maydicus,* |
| | *Rhizopus microsporus Tiegh:,* |
| | *R. stolonifer = R. nigricans,* |
| | *Scopulariopsis brumptii* |
| Ergot (horse's tooth) | *Claviceps gigantea* (anamorph: *Sphacelia sp.*) |
| Eyespot | *Aureobasidium zeae = Kabatiella zeae* |
| Fusarium ear and stalk rot | *Fusarium subglutinans = F. moniliforme varsubglutinans* |
| Fusarium kernel, root and stalk rot, seed rot and seedling blight | *Fusarium moniliforme* (teleomorph: *Gibberella fujikuroi*) |
| Fusarium stalk rot, seedling root rot | *Fusarium avenaceum* (teleomorph: *Gibberella avenacea*) |
| Gibberella ear and stalk rot (Ähren- u. Stengelfäule) | *Gibberella zeae* (anamorph: *Fusarium graminearum*) |
| Gray ear rot | *Botryosphaeria zeae = Physalospora zeae* (anamorph: *Macrophoma zeae*) |
| Gray leaf spot (Cercospora leaf spot) | *Cercospora sorghi = C. sorghi var. maydis*, *C. zeae-maydis* |
| Helminthosporium root rot | *Exserohilum pedicellatum = Helminthosporium pedicellatum* (teleomorph: *Setosphaeria pedicellata)* |
| Hormodendrum ear rot (Cladosporium rot) | *Cladosporium cladosporioides = Hormodendrum cladosporioides, C. herbarum* (teleomorph: *Mycosphaerella tassiana)* |
| Hyalothyridium leaf spot | *Hyalothyridium maydis* |
| Late wilt | *Cephalosporium maydis* |
| Leaf spots, minor | *Alternaria alternata, Ascochyta maydis, A. tritici, A. zeicola, Bipolaris victoriae = Helminthosporium victoriae* (teleomorph: *Cochliobolus victoriae), C. sativus* (anamorph: *Bipolaris sorokiniana = H. sorokinianum = H. sativum), Epicoccum nigrum, Exserohilum prolatum = Drechslera prolata* (teleomorph: *Setosphaeria prolata*) *Graphium penicillioides, Leptosphaeria maydis, Leptothyrium zeae, Ophiosphaerella herpotricha,* (anamorph: *Scolecosporiella sp.), Paraphaeosphaeria michotii, Phoma sp., Septoria zeae, S. zeicola, S. zeina* |
| Northern corn leaf blight (white blast, crown stalk rot, stripe) | *Setosphaeria turcica* (anamorph: *Exserohilum turcicum = Helminthosporium turcicum*) |
| Northern corn leaf spot Helminthosporium ear rot (race 1) | *Cochliobolus carbonum* (anamorph: *Bipolaris zeicola = Helminthosporium carbonum*) |
| Penicillium ear rot (blue eye, blue mold) | *Penicillium spp., P. chrysogenum, P. expansum, P. oxalicum* |
| Phaeocytostroma stalk rot and root rot | *Phaeocytostroma ambiguum = Phaeocytosporella zeae* |
| Phaeosphaeria leaf spot | *Phaeosphaeria maydis = Sphaerulina maydis* |
| Physalospora ear rot (Botryosphaeria ear rot) | *Botryosphaeria festucae = Physalospora zeicola* (anamorph: *Diplodia frumenti*) |
| Purple leaf sheath | Hemiparasitic bacteria and fungi |
| Pyrenochaeta stalk rot and root rot | *Phoma terrestris = Pyrenochaeta terrestris* |
| Pythium root rot | *Pythium spp., P. arrhenomanes*, *P. graminicola* |
| Pythium stalk rot | *Pythium aphanidermatum = P. butleri L.* |
| Red kernel disease (ear mold, leaf and seed rot) | *Epicoccum nigrum* |
| Rhizoctonia ear rot (sclerotial rot) | *Rhizoctonia zeae* (teleomorph: *Waitea circinata*) |
| Rhizoctonia root rot and stalk rot | *Rhizoctonia solani, Rhizoctonia zeae* |
| Root rots, minor | *Alternaria alternata, Cercospora sorghi, Dictochaeta fertilis, Fusarium acuminatum* (teleomorph: *Gibberella acuminata*), *F. equiseti* (teleomorph: *G. intricans*), *F. oxysporum, F. pallidoroseum, F. poae, F. roseum, G. cyanogena* (anamorph: F. *sulphureum), Microdochium bolleyi, Mucor sp., Periconia circinata, Phytophthora cactorum, P. drechsleri, P. nicotianae var. parasitica, Rhizopus arrhizus* |
| Rostratum leaf spot (Helminthosporium leaf disease, ear and stalk rot) | *Setosphaeria rostrata* (anamorph: *Exserohilum rostratum = Helminthosporium rostratum*) |
| Rust, common corn | *Puccinia sorghi* |
| Rust, southern corn | *Puccinia polysora* |
| Rust, tropical corn | *Physopella pallescens, P. zeae Angiopsora zeae* |
| Sclerotium ear rot (southern blight) | *Sclerotium rolfsii Sacc.* (teleomorph: *Athelia rolfsii*) |
| Seed rot-seedling blight | *Bipolaris sorokiniana, B. zeicola = Helminthosporium carbonum, Diplodia maydis, Exserohilum pedicillatum, Exserohilum turcicum = Helminthosporium turcicum, Fusarium avenaceum, F. culmorum, F. moniliforme, Gibberella zeae ( anamorph: F. graminearum*), *Macrophomina phaseolina, Penicillium spp., Phomopsis sp., Pythium spp., Rhizoctonia solani, R. zeae, Sclerotium rolfsii, Spicaria sp.* |
| Selenophoma leaf spot | *Selenophoma sp.* |
| Sheath rot | *Gaeumannomyces graminis* |
| Shuck rot | *Myrothecium gramineum* |
| Silage mold | *Monascus purpureus, Mruber* |
| Smut, common | *Ustilago zeae = U. maydis* |
| Smut, false | *Ustilaginoidea virens* |
| Smut, head | *Sphacelotheca reiliana = Sporisorium holcisorghi* |
| Southern corn leaf blight and stalk rot | *Cochliobolus heterostrophus* (anamorph: *Bipolaris maydis = Helminthosporium maydis*) |
| Southern leaf spot | *Stenocarpella macrospora = Diplodia macrospora* |
| Stalk rots, minor | *Cercospora sorghi, Fusarium episphaeria, F. merismoides, F. oxysporum Schlechtend, F. poae, F. roseum, F. solani* (teleomorph: *Nectria haematococca), F. tricinctum, Marlannaea elegans, Mucor sp., Rhopographus zeae, Spicaria sp.* |
| Storage rots | *Aspergillus spp., Penicillium spp.* and other fungi |
| Tar spot | *Phyllachora maydis* |
| Trichoderma ear rot and root rot | *Trichoderma viride = T. lignorum* (teleomorph: *Hypocrea sp.)* |
| White ear rot, root and stalk rot | *Stenocarpella maydis = Diplodia zeae* |
| Yellow leaf blight | *Ascochyta ischaemi, Phyllosticta maydis* (teleomorph: *Mycosphaerella zeae-maydis*) |
| Zonate leaf spot | *Gloeocercospora sorghi* |

Besonders bevorzugt wird eine Resistenz gegen
- Plasmodiophoromycota wie *Plasmodiophora brassicae* (Kohlhernie, clubroot of crucifers), *Spongospora subterranea* (powdery scab of potato tubers), *Polymyxa graminis* (root disease of cereals and grasses);
- Oomycota wie *Bremia lactucae* (Falscher Mehltau an Salat), *Peronospora* (Falscher Mehltau) bei snapdragon (*P. antirrhini*), Zwiebel (*P. destructor*), Spinat (*P. effusa*), Sojabohne (*P. manchurica*), Tabak ("blue mold" = Blauschimmel; *P. tabacina*) Alfalfa und Klee (*P. trifolium*), *Pseudoperonospora humuli* (Falscher Mehltau an Hopfen), *Plasmopara* (Falscher Mehltau bei Trauben) (*P. viticola*) und Sonnenblume (*P. halstedii*), *Sclerophtohra macrospora* (Falscher Mehltau bei Cerealien und Gäsern), *Pythium* (seed rot, seedling damping-off, and root rot and all types of plants, z.B. Wurzelbrand an Beta-Rübe durch *P. debaryanum*), *Phytophthora infestans* (Kraut- und Knollenfäule bei Kartoffel, Braunfäule bei Tomate etc.), *Albugo spec.* (white rust on cruciferous plants);
- Ascomycota wie *Microdochium nivale* (Schneeschimmel an Roggen und Weizen), *Fusarium graminearum, Fusarium culmorum* (Ährenfäule v.a. bei Weizen), *Fusarium oxysporum* (Fusarium-Welke an Tomate), *Blumeria graminis* (Echter Mehltau an Gerste (f.sp. hordei) und Weizen (f.sp. tritici)), *Erysiphe pisi* (Erbsenmehltau), *Nectria galligena* (Obstbaumkrebs), *Unicnula necator* (Echter Mehltau der Weinrebe), *Pseudopeziza tracheiphila* (Roter Brenner der Weinrebe), *Claviceps purpurea* (Mutterkorn an z.B. Roggen und Gräsern), *Gaeumannomyces graminis* (Schwarzbeinigkeit an Weizen, Roggen u.a. Gräsern), *Magnaporthe grisea* (rice blast disease), *Pyrenophora graminea* (Streifenkrankheit an Gerste), *Pyrenophora teres* (Netzfleckenkrankheit an Gerste), *Pyrenophora tritici-repentis* (Blattfleckenkrankheit (Blattdürre) an Weizen), *Venturia inaequalis* (Apfelschorf), *Sclerotinia sclerotium* (Weißstengeligkeit, Rapskrebs), *Pseudopeziza medicaginis* (Klappenschorf an Luzerne, Weiß- und Rotklee);
- Basidiomyceten wie *Typhula incarnata* (Typhula-Fäule an Gerste, Roggen, Weizen), *Ustilago maydis* (Beulenbrand an Mais), *Ustilago nuda* (Flugbrand an Gerste), *Ustilago tritici* (Flugbrand an Weizen, Dinkel), *Ustilago avenae* (Flugbrand an Hafer), *Rhizoctonia solani* (Wurzeltöter an Kartoffeln), *Sphacelotheca spp.* (head smut of sorghum), *Melampsora lini* (rust of flax), *Puccinia graminis* (Schwarzrost an Weizen, Gerste, Roggen, Hafer), *Puccinia recondita* (Braunrost an Weizen), *Puccinia dispersa* (Braunrost an Roggen), *Puccinia hordei* (Braunrost an Gerste), *Puccinia coronata* (Kronenrost an Hafer), *Puccinia striiformis* (Gelbrost an Weizen, Gerste, Roggen sowie zahlreichen Gräsern), *Uromyces appendiculatus* (Bohnenrost), *Sclerotium rolfsii* (root and stem rots of many plants);
- Deuteromyceten (Fungi imperfecti) wie *Septoria nodorum* (Spelzenbräune) an Weizen (*Septoria tritici), Pseudocercosporella herpotrichoides* (Halmbruchkrankheit an Weizen, Gerste, Roggen), *Rynchosporium secalis* (Blattfleckenkrankheit an Roggen und Gerste), *Alternaria solani* (Dürrfleckenkrankheit an Kartoffel, Tomate), *Phoma betae* (Wurzelbrand an Beta-Rübe), *Cercospora beticola* (Cercospora-Blattfleckenkrankheit an Beta-Rübe), *Alternaria brassicae* (Rapsschwärze an Raps, Kohl u.a. Kreuzblütlern), *Verticillium dahliae* (Rapswelke und -stengelfäule), *Colletotrichum lindemuthianum* (Brennfleckenkrankheit an Bohne), *Phoma lingam -* Umfallkrankheit (Schwarzbeinigkeit an Kohl; Wurzelhals- oder Stengelfäule an Raps), *Botrytis cinerea* (Grauschimmel an Weinrebe, Erdbeere, Tomate, Hopfen etc.). bewirkt

Am meisten bevorzugt sind die durch das erfindungsgemäße Verfahren hergestellten Pflanzen resistent gegen *Phytophthora infestans* (Kraut- und Knollenfäule, Braunfäule bei Tomate etc.), *Microdochium nivale* (vormals *Fusarium nivale*; Schneeschimmel an Roggen und Weizen), *Fusarium graminearum, Fusarium culmorum* (Ährenfäule an Weizen), *Fusarium oxysporum* (Fusarium-Welke an Tomate), *Blumeria graminis* (Echter Mehltau an Gerste (f. sp. hordei) und Weizen (f. sp. tritici)), *Magnaporthe grisea* (rice blast disease), *Sclerotinia sclerotium* (Weißstengeligkeit, Rapskrebs), *Septoria nodorum* und *Septoria tritici* (Spelzenbräune an Weizen), *Alternaria brassicae* (Rapsschwärze an Raps, Kohl u.a. Kreuzblütlern), *Phoma lingam* (Umfallkrankheit, Schwarzbeinigkeit an Kohl; Wurzelhals- oder Stengelfäule an Raps).

Beispielhaft, jedoch nicht einschränkend, für bakterielle Pathogene seien die in Tabelle 2 aufgeführten Pathogene und die mit ihnen in Zusammenhang gebrachten Erkrankungen genannt.

**Tabelle 2: Bakterielle Erkrankungen**

| **Erkrankung** | **Pathogen** |
|---|---|
| Bacterial leaf blight and stalk rot | *Pseudomonas avenae subsp. avenae* |
| Bacterial leaf spot | *Xanthomonas campestris pv. holcicola* |
| Bacterial stalk rot | *Enterobacter dissolvens = Erwinia dissolvens* |
| Schwarzbeinigkeit | *Erwinia carotovora subsp. carotovora, Erwinia* |
| ("Bacterial stalk and top rot") | *chrysanthemi pv. zeae* |
| Bacterial stripe | *Pseudomonas andropogonis* |
| Chocolate spot | *Pseudomonas syringae pv. coronafaciens* |
| Goss's bacterial wilt and blight (leaf freckles and wilt) | *Clavibacter michiganensis subsp. nebraskensis* = *Corynebacterium michiganense pv.andnebraskense* |
| Holcus spot | *Pseudomonas syringae pv. syringae* |
| Purple leaf sheath | Hemiparasitic bacteria |
| Seed rot-seedling blight | *Bacillus subtilis* |
| Stewart's disease (bacterial wilt) | *Pantoea stewartii = Erwinia stewartii* |
| Corn stunt (achapparramiento,maize stunt, Mesa Central or Rio Grande maize stunt) | *Spiroplasma kunkelii* |

Besonders bevorzugt sind die erfindungsgemäß hergestellten transgenen Pflanzen resistent gegen nachfolgende pathogene Bakterien:
*Corynebacterium sepedonicum* (Bakterienringfäule an Kartoffel), *Erwinia carotovora* (Schwarzbeinigkeit an Kartoffel), *Erwinia amylovora* (Feuerbrand an Birne, Apfel, Quitte), *Streptomyces scabies* (Kartoffelschorf), *Pseudomonas syringae pv. tabaci* (Wildfeuer an Tabak), *Pseudomonas syringae pv. phaseolicola* (Fettfleckenkrankheit an Buschbohne), *Pseudomonas syringae pv. tomato* ("bacterial speck" an Tomate), *Xanthomonas campestris pv. malvacearum* (Blattfleckenkrankheit an Baumwolle) und *Xanthomonas campestris pv. oryzae* (Bakterienfäule an Reis und anderen Gräsern).

Der Begriff "virale Pathogene" schließt sämtliche Pflanzenviren ein wie beispielsweise Tabak- oder oder Cucumber-Mosaiv Virus, Ringspot-Virus, Nehose-Virus, Mais Dwarf-Mosaic Virus etc.

Beispielhaft, jedoch nicht einschränkend, für virale Pathogene seien die in Tabelle 3 aufgeführten Pathogene und die mit ihnen in Zusammenhang gebrachten Erkrankungen genannt.

**Tabelle 3: Virale Erkrankungen**

| **Krankheit** | **Pathogen** |
|---|---|
| American wheat striate (wheat striate mosaic) | American wheat striate mosaic virus (AWSMV) |
| Barley stripe mosaic | Barley stripe mosaic virus (BSMV) |
| Barley yellow dwarf | Barley yellow dwarf virus (BYDV) |
| Brome mosaic | Brome mosaic virus (BMV) |
| Cereal chlorotic mottle | Cereal chlorotic mottle virus (CCMV) |
| Corn chlorotic vein banding (Braizilian maize mosaic) | Corn chlorotic vein banding virus (CCVBV) |
| Corn lethal necrosis | Viruskomplex aus Maize chlorotic mottle virus (MCMV) und Maize dwarf mosaic virus (MDMV) A oder B oder Wheat streak mosaic virus(WSMV) |
| Cucumber mosaic | Cucumber mosaic virus (CMV) |
| Cynodon chlorotic streak | Cynodon chlorotic streak virus (CCSV) |
| Johnsongrass mosaic | Johnsongrass mosaic virus (JGMV) |
| Maize bushy stunt | Mycoplasma-like organism (MLO) associated |
| Maize chlorotic dwarf | Maize chlorotic dwarf virus (MCDV) |
| Maize chlorotic mottle | Maize chlorotic mottle virus (MCMV) |
| Maize dwarf mosaic | Maize dwarf mosaic virus (MDMV) strains A, D, E and F |
| Maize leaf fleck | Maize leaf fleck virus (MLFV) |
| Maize line | Maize line virus (MLV) |
| Maize mosaic (corn leaf stripe, enanismo rayado) | Maize mosaic virus (MMV) |
| Maize mottle and chlorotic stunt | Maize mottle and chlorotic stunt virus |
| Maize pellucid ringspot | Maize pellucid ringspot virus (MPRV) |
| Maize raya gruesa | Maize raya gruesa virus (MRGV) |
| maize rayado fino (fine striping disease) | Maize rayado fino virus (MRFV) |
| Maize red leaf and red stripe | Mollicute |
| Maize red stripe | Maize red stripe virus (MRSV) |
| Maize ring mottle | Maize ring mottle virus (MRMV) |
| Maize rio IV | Maize rio cuarto virus (MRCV) |
| Maize rough dwarf (nanismo ruvido) | Maize rough dwarf virus (MRDV) (Cereal tillering disease virus) |
| Maize sterile stunt | Maize sterile stunt virus (strains of barley yellow striate virus) |
| Maize streak | Maize streak virus (MSV) |
| Maize stripe (maize chlorotic stripe, maize hoja blanca) | Maize stripe virus |
| Maize stunting | Maize stunting virus |
| Maize tassel abortion | Maize tassel abortion virus (MTAV) |
| Maize vein enation | Maize vein enation virus (MVEV) |
| Maize wallaby ear | Maize wallaby ear virus (MWEV) |
| Maize white leaf | Maize white leaf virus |
| Maize white line mosaic | Maize white line mosaic virus (MWLMV) |
| Millet red leaf | Millet red leaf virus (MRLV) |
| Northern cereal mosaic | Northern cereal mosaic virus (NCMV) |
| Oat pseudorosette (zakuklivanie) | Oat pseudorosette virus |
| Oat sterile dwarf | Oat sterile dwarf virus (OSDV) |
| Rice black-streaked dwarf | Rice black-streaked dwarf virus (RBSDV) |
| Rice stripe | Rice stripe virus (RSV) |
| Sorghum mosaic | Sorghum mosaic virus (SrMV) (auch: sugarcane mosaic virus (SCMV) Stämme H, I and M) |
| Sugarcane Fiji disease | Sugarcane Fiji disease virus (FDV) |
| Sugarcane mosaic | Sugarcane mosaic virus (SCMV) strains A, B, D, E, SC, BC, Sabi and MB (formerly MDMV-B) |
| Wheat spot mosaic | Wheat spot mosaic virus (WSMV) |

Die erfindungsgemäß hergestellten transgenen Pflanzen können auch gegen tierische Schädlinge wie Insekten und Nematoden resistent sein. Beispielhaft, jedoch nicht einschränkend, seien Insekten wie beispielsweise Käfer, Raupen, Läuse oder Milben zu nennen.

Bevorzugt sind die erfindungsgemäß hergestellten transgenen Pflanzen resistent gegen Insekten der Gattungen Coleoptera, Diptera, Hymenoptera, Lepidoptera, Mallophaga, Homoptera, Hemiptera, Orthoptera, Thysanoptera. Dermaptera, Isoptera, Anoplura, Siphonaptera, Trichoptera, etc. Besonders bevorzugt sind Insekten der Gattungen Coleoptera and Lepidoptera, wie beispielsweise der Maiszünsler (European Corn Borer (ECB)), *Diabrotica barberi* ("northern corn rootworm"), *Diabrotica undecimpunctata* ("southern corn rootworm"), *Diabrotica virgifera* ("Western corn rootworm"), *Agrotis ipsilon* ("black cutworm"), *Crymodes devastator* ("glassy cutworm"), *Feltia ducens* ("dingy cutworm"), *Agrotis gladiaria* ("claybacked cutworm"), *Melanotus spp., Aeolus mellillus* ("wireworm"), *Aeolus mancus* ("wheat wireworm"), *Horistonotus uhlerii* ("sand wireworm"), *Sphenophorus maidis* ("maize billbug"), *Sphenophorus zeae* ("timothy billbug"), *Sphenophorus parvulus* ("bluegrass billbug"), *Sphenophorus callosus* ("southern corn billbug"), *Phyllogphaga* spp.("white grubs"), *Anuraphis maidiradicis* ("corn root aphid"), *Delia platura* ("seedcorn maggot"), *Colaspis brunnea* ("grape colaspis"), *Stenolophus lecontei* ("seedcorn beetle") und *Clivinia impressifrons* ("lender seedcorn beetle").

Ferner sind zu nennen: Das Getreidehähnchen (*Oulema melanopus*), die Fritfliege (*Oscinella frit*), Drahtwürmer (*Agrotis lineatus*) und Blattläuse (wie z.B. Haferblattlaus *Rhopalosiphum padi,* Grosse Getreideblattlaus *Sitobion avenae*).

Beispielhaft, jedoch nicht einschränkend, für Nematodenschädlinge seien die in Tabelle 4 aufgeführten Pathogene und die mit ihnen in Zusammenhang gebrachten Erkrankungen genannt.

**Tabelle 4: Parasitäre Nematoden**

| **Schädigung** | **Pathogene Nematode** |
|---|---|
| Awl | *Dolichodorus spp., D. heterocephalus* |
| Stengel- oder Stockälchen, Rübenkopfalchen ("Bulb and stem"; Europe) | *Ditylenchus dipsaci* |
| Burrowing | *Radopholus similis* |
| Haferzystenälchen ("Cyst") | *Heterodera avenae, H. zeae, Punctodera chalcoensis* |
| Dagger | *Xiphinema spp., X. americanum, X. mediterraneum* |
| False root-knot | *Nacobbus dorsalis* |
| Lance, Columbia | *Hoplolaimus columbus* |
| Lance | *Hoplolaimus spp., H. galeatus* |
| Lesion | *Pratylenchus spp., P. brachyurus, P. crenatus, P. hexincisus, P. neglectus, P. penetrans, P. scribneri, P. thornei, P. zeae* |
| Needle | *Longidorus spp., L. breviannulatus* |
| Ring | *Criconemella spp., C. ornata* |
| Wurzelgallenälchen ("Root-knot") | *Meloidogyne spp., M. chitwoodi, M. incognita, M. javanica* |
| Spiral | *Helicotylenchus spp.* |
| Sting | *Belonolaimus spp., B. longicaudatus* |
| Stubby-root | *Paratrichodorus spp., P. christiei, P. minor, Quinisulcius acutus, Trichodorus spp.* |
| Stunt | *Tylenchorhynchus dubius* |

Traditionell werden die oben genannten sowie weitere pflanzliche Pilzerkrankungen durch den Einsatz von Fungiziden bekämpft, die die bekannten Nachteile, wie Grundwassergängigkeit und Akkumulation in der Nahrungskette, besitzen.

In den letzten Jahren wurden aber auch einige Gene identifiziert, die Resistenz gegen einen bestimmten oder gegen mehrere Erreger vermitteln können. Der Begriff "Vermittlung von Pathogenresistenz", wie er hier verwendet wird, bedeutet, dass Pflanzen, in denen die Expression der besagten Gene erhöht ist, gegenüber Pflanzen, in denen die Expression der besagten Gene normal ist, weniger empfänglich für die Infektion mit bestimmten Pathogenen sind. Zu den Genen, die Pathogenabwehr vermitteln, gehören auch solche Gene, deren Expression durch Infektion mit einem Pathogen angeschaltet wird.

Zu diesen Resistenzgenen gehören Peroxidasen und Oxalat-Oxidasen. Die Oxalat-Oxidasen, die zu der Familie der germinartigen Proteine gehören, katalysieren die Oxidation von Oxalat, wodurch Wasserstoffperoxid entsteht. Das Wasserstoffperoxid wirkt mikrobizid und kann die Lignifizierung der Zellwände fördern, wodurch das Eindringen von Schädlingen verhindert wird. Außerdem kann es in geringen Konzentrationen hypersensitiven Zelltod hervorrufen. Die Peroxidasen verwenden entweder molekularen Sauerstoff oder Wasserstoffperoxid, um zelluläre Substrate zu oxidieren und dadurch zu entgiften. Eine zur Überexpression in Weizen und Gerste geeignete Peroxidase ist TaPERO (Accession Number X56011; Altpeter et al. (2005) Plant Molecular Biology 57: 271-283; DE 103 46 611 A1).

Pathogene, gegenüber denen die Expression der Oxalat-Oxidasen und Peroxidasen in der Epidermis von Pflanzen Resistenz vermitteln kann, schließen zum Beispiel ein: Echter Mehltau, Fusarium spp., *Rynchosporium secalis* und *Pyrenophora teres.*

Weitere Gene, die in der Lage sind, Resistenz gegen Pathogene zu vermitteln, sind Chitinasen, Ag-AFP, GSTA1 und WIR1a.

Durch die pathogeninduzierbare Expression der für diese Enzyme kodierenden Nukleinsäuresequenz in der Epidermis von transgenen Pflanzen mit Hilfe der erfindungsgemäßen Promotorregion können Pflanzen mit erhöhter Pathogenresistenz erhalten werden.

Im Gegensatz zu den Pathogenresistenz vermittelnden Genen gibt es auch pflanzeneigene Gene, die das Eindringen eines Pathogens fördern. Zu diesen gehört das Mlo-Gen (Accession Number AF361933), das für einen Sieben-Transmembran-Rezeptor kodiert, der das Eindringen des Mehltau-Pilzes in die Epidermis zu fördern scheint. In diesem Fall ist es sinnvoll, mit der Expression des Mlo-Gens zu interferieren, um das Eindringen von Pilzen in die Pflanze zu verhindern. Dies kann z. B. mit Hilfe der oben beschriebenen RNAi-Methode erfolgen. Dass die Interferenz mit der Expression des Mlo-Gens geeignet ist, das Eindringen des Mehltaupilzes in die Pflanze zu verhindern, wurde in vitro an Blattsegmenten aus Gerste gezeigt, die mit Wolfram-Teilchen beschossen wurden, die mit Mlo-dsRNA beschichtet worden waren (Schweizer et al. (2000) The Plant Journal 24 (6): 895-903).

Weitere pflanzliche Gene, die die Interaktion eines Pathogens mit der Pflanze vermitteln können und dadurch das Eindringen des Pathogens in die Pflanze fördern können, sind beispielsweise Aminosäuren- oder Zuckertransporter oder Invertasen. Diese Gene eignen sich ebenfalls als Angriffspunkte für das Gen-Silencing.

Somit betrifft die vorliegende Erfindung ein Verfahren zur Herstellung pathogenresistenter Pflanzen, umfassend die Schritte:
a) Herstellung eines rekombinanten Nukleinsäuremoleküls, in dem der erfindungsgemäße Promotor in operativer Verknüpfung mit einer Nukleinsäuresequenz, die Pathogenresistenz vermittelt, vorliegt,
b) Übertragung des rekombinanten Nukleinsäuremoleküls aus a) auf pflanzliche Zellen und
c) Regeneration vollständig transformierter Pflanzen und, falls erwünscht, Vermehrung der Pflanzen.

Bevorzugt handelt es sich bei der Pathogenresistenz vermittelnden Nukleinsäuresequenz um die kodierende Region eines Peroxidase- oder Oxalat-Oxidase-Gens oder um eine Sequenz, die mit der endogenen Mlo-RNA interferiert.

Die erfindungsgemäße Promotorsequenz kann auch dazu verwendet werden, pathogeninduzierte Signaltransduktionswege in Pflanzen zu untersuchen. Dazu kann man ein rekombinantes Nukleinsäuremolekül umfassend ein unter der Kontrolle der erfindungsgemäßen Promotorregion stehendes Reportergen zusammen mit einem weiteren rekombinanten Nukleinsäuremolekül in Pflanzenzellen einbringen, das die Expression eines endogenen Gens hemmt oder eine Mutante des endogenen Gens exprimiert, und die transformierten Pflanzenzellen mit einem geeigneten Pathogen inokulieren. Nach einer geeigneten Inkubationszeit kann man die Expression des Reportergens in den Pflanzenzellen bestimmen und mit der Expression des Reportergens in einer Pflanzenzelle vergleichen, die zur Kontrolle nur mit dem Reporterkonstrukt transformiert wurde. Ist die Expression des Reportergens gegenüber der Kontrolle gehemmt, handelt es sich bei dem gehemmten endogenen Gen um einen positiven Regulator des pathogeninduzierten Signalweges. Dagegen handelt es sich bei dem gehemmten Gen um einen negativen Regulator des Signaltransduktionsweges, wenn die Expression des Reportergens gegenüber der Kontrolle erhöht ist.

Geeignete Verfahren zur Hemmung der Expression des endogenen Gens schließen ein Antisense-Verfahren, RNAi oder das TILLING. Das TILLING-Verfahren ist eine Strategie der so genannten reversen Genetik, das die Produktion hoher Dichten von Punktmutationen in mutagenisierten Pflanzenkollektionen, z.B. durch chemische Mutagenese mit Ethylmethansulfonat (EMS), mit der schnellen systematischen Identifizierung von Mutationen in Zielsequenzen kombiniert. Zunächst wird die Zielsequenz über PCR in DNA-Pools mutagenisierter M2-Populationen amplifiziert. Denaturierungs- und Annealingreaktionen der heteroallelischen PCR-Produkte erlauben die Ausbildung von Heteroduplexen, bei denen ein DNA-Strang von dem mutierten und der andere vom "Wildtyp" PCR-Produkt stammt. An der Stelle der Punktmutation erfolgt ein sogenannter Mismatch, der entweder über denaturierende HPLC (DHPLC, McCallum et al. (2000) Plant Physiol. 123: 439-442) oder mit dem CelI Mismatch-Detektionssystem (Oleykowsky et al. (1998) Nucl. Acids Res. 26: 4597-4602) identifiziert werden kann. CelI ist eine Endonuklease, die Mismatches in Heteroduplex-DNA erkennt und spezifisch an diesen Stellen die DNA spaltet. Die Spaltungsprodukte können dann über automatisierte Sequenzierungs-Gelelektrophorese aufgetrennt und detektiert werden (Colbert et al. (2001) Plant Physiol. 126(2): 480-484). Nach Identifizierung von Zielgen-spezifischen Mutationen in einem Pool werden individuelle DNA-Proben entsprechend analysiert, um die Pflanze mit der Mutation zu isolieren.

Geeignete Proteine, deren Funktion bei der Pathogeninduktion mit Hilfe der erfindungsgemäßen Promotorregion untersucht werden kann, sind zum Beispiel MAP-Kinasen oder Phosphatasen.

Ebenso ist es natürlich möglich, die pathogeninduzierte Signaltransduktion mit Hilfe von chemischen Substanzen zu untersuchen, deren Zielproteine bekannt sind. Dazu kann man Pflanzenzellen mit einem rekombinanten Nukleinsäuremolekül umfassend die erfindungsgemäße Promotorregion transfizieren und mit einem geeigneten Pathogen inokulieren. Entweder vor, während oder nach der Inokulation kann den Pflanzen die chemische Substanz zugegeben werden, deren Wirkung auf die Signaltransduktion untersucht werden soll. Zur Auswertung wird die Aktivität des Reportergens in Zellen, die mit der chemischen Substanz behandelt worden waren, mit der Aktivität des Reportergens in unbehandelten Zellen verglichen.

Die nachfolgenden Beispiele dienen zur Erläuterung der Erfindung und sollten nicht einschränkend verstanden werden.

### Abbildungen

1. Vektorkarte von pGLP4GUS
2. Vektorkarte von pGLP4IntronGUS
3. Vektorkarte von pGUS
4. Vektorkarte von pIntronGUS
5. Schematische Darstellung der zur transienten Transformation verwendeten Reporterkonstrukte
   Die Reporterkonstrukte bestehen aus Fusionen der angegebenen Promotorsequenzen (schwarzer Pfeil) mit dem β-Glucuronidase-Reporter (GUS) und dem transkriptionellen Terminator des GSTA1- bzw. 35S-Gens (GSTA1 bzw. 35S). Zur Modulation der Promotoraktivität wurde eine Kassette bestehend aus dem Exon1-Intron1 aus dem Weizen WIR1 Gen (I) eingesetzt. Die Reporterkonstrukte sind schematisch zusammen mit dem Standardisierungskonstrukt (p35SGFP) dargestellt, welches die Expression des "green fluorescent protein" aus *Aequorea victoria* unter Kontrolle des CaMV 35S Promotors ermöglicht.
6. Vektorkarte von p6UGLP4GUS
7. Vektorkarte von p6UGLP4IntronGUS
8. Pathogeninduzierbare GUS-Expression in den Blättern transgener Gerstepflanzen

### Beispiele

### 1. Subklonierung

Zur Identifizierung von HvGLP4 Genen inkl. Promotor wurde der BAC-Klon 418E01 (Druka et al. PNAS 99(2): 850-55) aus einer Gersten-BAC-Bibliothek (Yu et. al. (2000) TAG 101: 1093-99) entnommen und der entsprechende Genbereich zur Sequenzierung und Annotation subkloniert.

Für die Gen- und Promotoridentifizierung wurden Einzelklone in zwei Stufen subkloniert. Zunächst wurde die BAC-DNA eines Einzelklones mittels Qiagen-Säule isoliert (Maxi-Kit; Qiagen; Isolierung nach Herstellerprotokoll). Durch Scherung (Hydroshear: Genomic Solutions) wurden von dieser BAC-DNA 5 - 10 kbp Fragmente erzeugt und die entstandenen Enden mit Klenow zu glatten Enden aufgefüllt (Reaktion nach Protokoll des Herstellers). Die Selektion der Fragmentlängen erfolgte über ein 0,8%iges Agarosegel in 0,5 % TBE. Aus dem Gel wurde der entsprechende Fragmentlängenbereich herausgeschnitten und die DNA aus dem Agarosegel mit Hilfe von Qiagen Gel Extraction Kit eluiert (Elution nach Protokoll des Herstellers). Die eluierten 5-10 kbp Fragmente wurden in einen mit EcoRV linearisierten pBluescript II SK(-) Vektor mit glatten dephosphorylierten Enden ligiert (Restriktion und Dephosphorylierung entsprechend den Herstellerangaben) und in hochkompetente *E*. *coli* Zellen chemisch-thermisch transformiert. Anschließend wurden die Transformanten mit Hilfe eines Picking-Roboters (Qpick, Genetix) willkürlich geordnet und in Mikrotiterplatten mit LB-Medium überführt.

Mittels PCR (siehe unter 2.) wurde das Subfragtnent ausgewählt, welche das interessierende Gen trägt und die Länge des potenziellen Promotors maximiert. Das gewählte Subfragment wurde erneut in 1-2 kbp Fragmente geschert, ligiert, transformiert und die Klone in Microtiterplatten gelagert (s.o.). Von den gepickten Klonen wurden 96 Kolonien willkürlich ausgewählt und mit dem TempliPhi Protokoll (Amersham Biosciences) nach Herstellerangaben sequenziert. Die Sequenzen wurden assembliert. Die erhaltene Sequenzinformation wurde für die Annotation der kodierenden Exons im Vergleich zu cDNA Sequenzen von HvGLP4 genutzt, um die auf dem BAC-Subklon befindlichen HvGLP4 Gene und ihre potenziellen Promotoren zu bestimmen.

### 2. PCR-Amplifikation der interessierenden Sequenzen aus dem BAC-Klon

Die PCR wurde aufgrund ihrer hohen Sensitivität zum Nachweis der gesuchten DNA-Sequenz gewählt. Die Analyse wurde in 20 µl Reaktionsvolumen durchgeführt. Der Reaktionsansatz bestand aus 10 mM Tris-HCl, pH 9,0; 50 mM KCl; 0,1 % Triton X-100, 0,2 mM dNTP; 2 mM MgCl₂, je 0,6 µM Oligonukleotide und Taq-Polymerase (Konzentration im Reaktionsansatz: ∼ 1U µl⁻¹). Pro Reaktionsansatz wurden entweder 10 ng BAC-Pool-DNA oder 2 µl Bakterienkultur (für Kolonie-PCR) verwendet. Vorhandene HvGLP4 cDNA-Sequenzen dienten als Grundlage zur Ableitung der Oligonukleotide
5' - GGA TTT GTC ACG TCC AAC CT - 3' und
5' - ATT GGC AAT TGT GAT AGC CC - 3'.
Die zu amplifizierende BAC-DNA und die Primer wurden vorgelegt und anschließend mit dem PCR-Reaktionsansatz vermischt. Zum Abtöten und Aufschließen der Bakterien in einer Kolonie-PCR wurde die Vorlage vor der Zugabe des PCR-Reaktionsgemisches für 5 min auf 95°C erhitzt. Zur Denaturierung der doppelsträngigen DNA wurde ein initialer Schritt von 5 min bei 95°C benutzt. Die Touch-Down PCR-Reaktion erfolgte in den Abschnitten 30 s 95°C; 30 s 60 nach 55°C und 60 s 72°C für die ersten 10 Zyklen. Dabei wurde mit jedem Zyklus die Temperatur um 0,5 °C (60 nach 55°C) gesenkt. Weitere 30 Zyklen erfolgten in den Abschnitten 30 s 95°C; 30 s 55°C und 60 s 72°C. Zur abschließenden Kettenverlängerung wurde 5 min bei 72°C inkubiert, bevor das Reaktionsgemisch auf eine Temperatur von 20°C abgekühlt wurde, die konstant gehalten wurde. Die Analyse der PCR-Amplifikate erfolgte aufgrund des erwarteten kurzen Reaktionsproduktes von 189 bp mit 2,5 %igen Agarosegelen in 0,5x TBE-Puffer.

### 3. Herstellung der verwendeten Konstrukte

### a) Zwischenvektor pSKGLP4 (Klonierung des erfindungsmäßigen Promotors in pBluescript II SK (+))

Die erfindungsgemäße Promotorsequenz (3334 bp) wurde mit den Primern
5' - CGTGCGTAAATTAAGGGCAT - 3' (forward) und
5' - CAGCTCCTTTGGGTCTTG - 3' (reverse) aus dem genomischen GLP4 Klon (418e1-c9) mittels PCR amplifiziert. Das 5' Ende des reverse Primers war 5 bp stromaufwärts vom GLP4-Startkodon positioniert. Das GLP4-Promotorfragment wurde über Agarose-Gelelektrophorese gereinigt und durch T4 Polynukleotid-Kinase phosphoryliert. Der Vektor pBluescript II SK (+) wurde in der "multiple cloning site" mit *Eco*RV geschnitten und mit den "stumpfen Enden" (blunt ends) des GLP4-Promotorfragments ligiert. Bei dieser ungerichteten Ligation werden Produkte erhalten, die das GLP4-Promotorfragment in beiden Orientierungen tragen. Das resultierende Plasmid (pSKGLP4), welches die forward Primersequenz benachbart zur *Hind*III Schnittstelle des pBluescript II SK (+) Vektors trug, wurde aufgrund der Lage von Restriktionsschnittstellen (*Kpn*I, *Pst*I und *Sma*I) für die Konstruktion der Reportervektoren pGLP4GUS und pGLP4IntronGUS eingesetzt.

### b) Reportervektor pGLP4GUS (GUS-Expression unter der Kontrolle des erfindungsgemäßen Promotors)

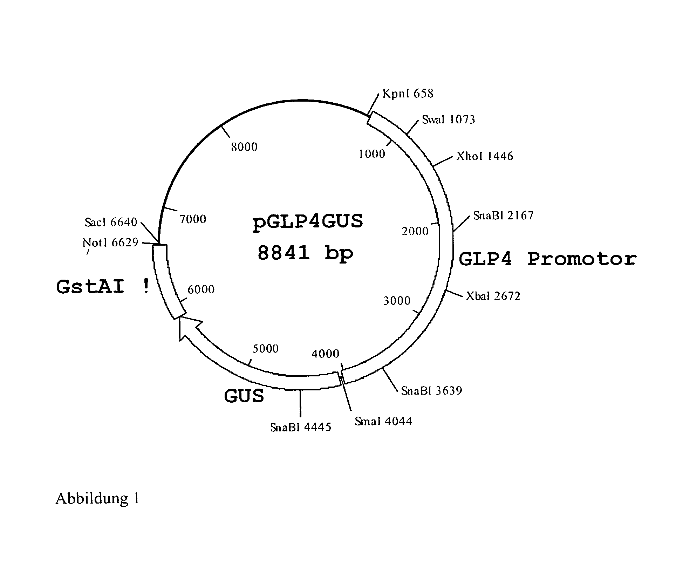

Die erfindungsgemäße GLP4-Promotorsequenz wurde aus dem Zwischenvektor pSKGLP4 mittels *Kpn*I und *Sma*I Restriktionsspaltung herausgeschnitten. Das 3396 bp lange Fragment wurde in den *Kpn*I/*Sma*I geschnittenen und über Agarose-Gelelektrophorese gereinigten pPS18-Vektor (DE 103 46 611 A1) ligiert. Durch Austausch des GstAI Promotor- und intronenthaltenden WIR1a-Genfragmentes durch die GLP4-Promotorsequenz entstand eine transkriptionelle Fusion, in der die Expression des GUS-Reportergens durch den GLP4-Promotor kontrolliert wird. Die Sequenz des resultierenden Plasmids ist in SEQ ID Nr. 4 angegeben und die Struktur des Plasmids in Abb. 1 dargestellt.

### c) Reportervektor pGLP4IntronGUS (GUS-Expression unter der Kontrolle des erfindungsgemäßen Promotors und moduliert durch ein intronenthaltendes WIR1a-Genfragment)

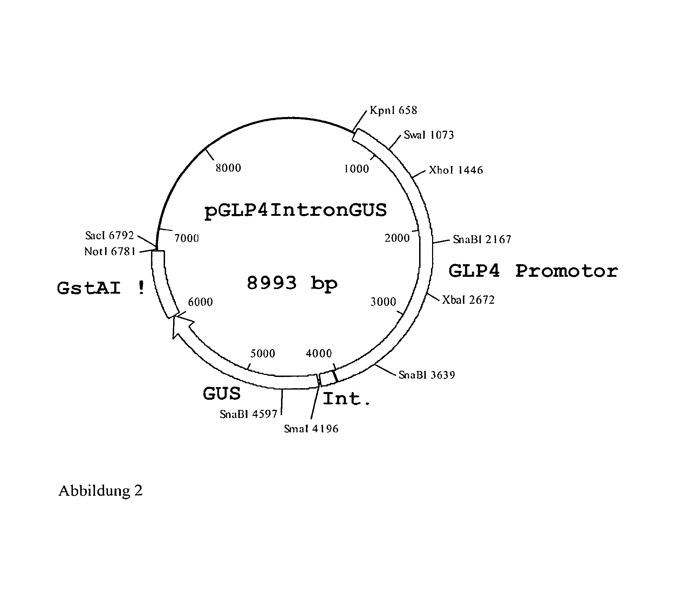

Die erfindungsgemäße GLP4 Promotorsequenz wurde aus dem Zwischenvektor pSKGLP4 mittels *Kpn*I und *Pst*I Restriktionsspaltung herausgeschnitten. Das 3392 bp lange Fragment wurde in den KpnI/PstI geschnittenen und über Agarose-Gelelektrophorese gereinigten pPS18-Vektor ligiert. Durch den Austausch der GstAI- durch die GLP4-Promotorsequenz wurde eine transkriptionelle Fusion des intronenthaltenden WIR1a-Genfragmentes mit dem GUS-Reportergen erzeugt, die durch den GLP4 Promotor.kontrolliert wird Die Sequenz des resultierenden Plasmids ist in SEQ ID Nr. 5 angegeben und die Struktur des Plasmids in Abb. 2 dargestellt.

### d) Reportervektor pGUS (GUS-Reportergen ohne Promotor, Kontrollplasmid)

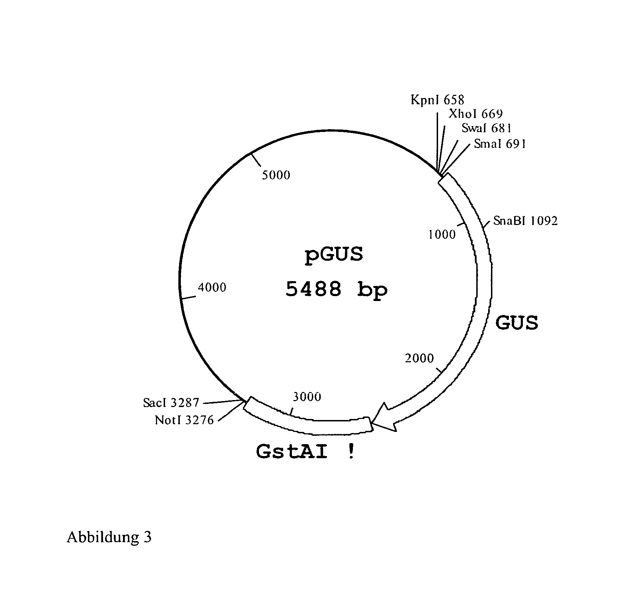

Der mit *Xho*I/*Xma*I geöffnete pPS 18-Vektor wurde über Agarose-Gelelektrophorese gereinigt und mit einem doppelsträngigen Oligonukleotid
(5' - TCGAGCACATTTAAATCAAC - 3' plus
5' - CCGGGTTGATTTAAATGTGC - 3') ligiert. In dem Kontrollplasmid (pGUS) liegt das GUS-Reportergen ohne Promotor vor. Die Sequenz des resultierenden Plasmids ist in SEQ ID Nr. 6 angegeben und die Struktur des Plasmids in Abb. 3 dargestellt.

### e) Reportervektor pIntronGUS (GUS-Reportergen ohne Promotor mit intronenthaltendem WIR1a-Genfragment, Kontrollplasmid)

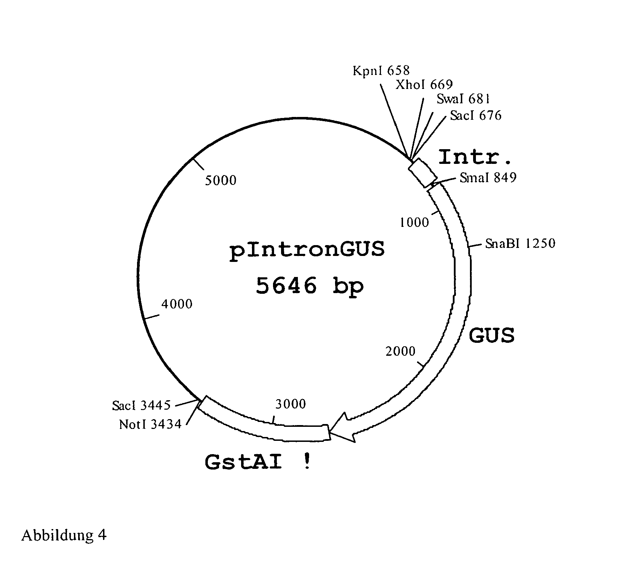

Der mit *Xho*I/*Pst*I geöffnete pPS 18-Vektor wurde über Agarose-Gelelektrophorese gereinigt und mit einem doppelsträngigen Oligonukleotid
(5' - TCGAGCTCATTTAAATCCTCTGCA - 3' plus
5' - GAGGATTTAAATGAGC - 3') ligiert. In dem resultierenden Kontrollplasmid (pIntronGUS) ist die intronenthaltende WIR1a-Gensequenz ohne GstAI Promotor mit dem GUS-Reportergen fusioniert. Die Sequenz des resultierenden Plasmids ist in SEQ ID Nr. 7 angegeben und die Struktur des Plasmids in Abb. 4 dargestellt.

### 4. Transiente Expression der Reportervektoren in Gerstenblättern durch Partikelbeschuss

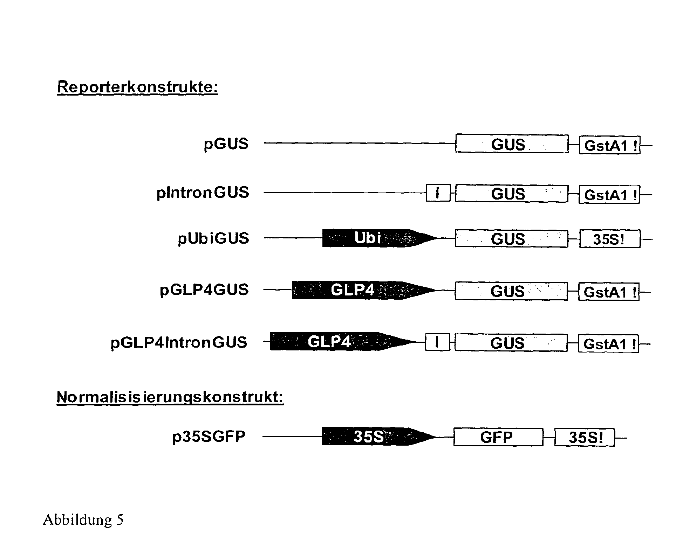

Die zur Transformation verwendeten Reporterkonstrukte sind in Abb. 5 schematisch dargestellt. Sie wurden mit einer "Genkanone" (Bio-Rad, Modell PDS-1000/He, Hepta-Adapter) mittels biolistischer Transformation in Gerstenblätter eingebracht, gemäss Douchkov et al. (2005) Mol. Plant Microbe Interact. 18: 755-761. Für die Beschichtung mit DNA wurden pro Schuß 2,18 mg Goldpartikel (1,0 µm Durchmesser, Partikeldichte 25 mg ml⁻¹ in 50% (v/v) Glyzerin) mit 7 µg "supercoiled" DNA gemischt und mit 1 M Ca(NO₃)₂ pH 10 versetzt, sodass die Endkonzentration von Ca(NO₃)₂ 0,5 M betrug. Nach Zentrifugieren und Waschen mit 70 % (v/v) Ethanol wurden die Partikel in 96 % (v/v) Ethanol resuspendiert und auf den 7 Makrocarriern verteilt. Die Partikel wurden in einem Vakuum (3,6x10³ Pa) durch eine Heliumdruckwelle von 7,6x10⁶ Pa auf jeweils 7 Blattsegmente von 7 Tage alten Wildtypgerstenpflanzen (Golden Promise) eingebracht. Zum Beschuß wurden die Blattsegmente in eine Petrischale auf 0,5% (w/v) Phytoagar gelegt, der mit 20 µg ml⁻¹ Benzimidazol versetzt war. Die Blätter wurden nach dem Beschuss für 4 h bei +20°C und indirektem Tageslicht inkubiert.

### 5. Inokulation der Blattsegmente mit Mehltau-Sporen

Die bombardierten Blätter wurden auf Polycarbonat-Schalen mit 1 % (w/v) Phytoagar enthaltend 20 µg ml⁻¹ Benzimidazol transferriert. Die Infektion mit Mehltau-Sporen erfolgte in einem Inokulationsturm, indem Sporen von stark infizierten Weizenblättern in den Turm geschüttelt wurden. Nach 5 min wurden die Schalen aus dem Turm entfernt, geschlossen und bei +20°C und indirektem Tageslicht für 60 h inkubiert.

### 6. Nachweis von grün-fluoreszierendem Protein (GFP)

Die Anzahl GFP-exprimierender Epidermiszellen bombardierter Blätter wurde 24 Stunden nach Transformation mittels Auflicht-Fluoreszenzmikroskopie bestimmt. Zu diesem Zweck wurde ein Zeiss Axioplan Imaging 2 Mikroskop mit Filtersatz Nr. 10 und 100-facher Vergrösserung verwendet: Anregungsfenster 450-490 nm; Emissionsfenster bypass 515-565 nm. Die Expression des konstitutiv exprimierten GFP diente der Standardisierung der Expression.

### 7. Histochemischer Nachweis der GUS-Expression

Die Blätter wurden unter Vakuum mit der GUS-Nachweislösung (10 mM EDTA, 1,4 mM K₃[Fe(CN)₆], 1,4 mM K₄[Fe(CN)₆], 0,1 % (v/v) Triton X-100, 20 % (v/v) Methanol, 1 mg/ml 3-Bromo-4-chlom-3-indolyl-β-D-glucuronsäure, 100 mM Na-Phosphatpuffer, pH 7,0) infiltriert und über Nacht bei +37°C inkubiert. Nach Entfernen der Nachweislösung wurden die Blätter mit einer Lösung aus 7,5 % (w/v) Trichloressigsäure und 50 % (v/v) Methanol für 15 min bei +20°C entfärbt. Der lichtmikroskopische Nachweis der GUS-Expression erfolgte mit einem Zeiss Axiolab Mikroskop bei 100-facher Vergrösserung. Zellen mit GUS-Expression besitzen einen blau gefärbten Zellinhalt.

Ein quantitativer Vergleich der GUS-Expression in den mit den verschiedenen Reporterkonstrukten transformierten Blättern, die entweder mit Mehltau infiziert worden waren oder nicht infiziert worden waren, ist in nachfolgender Tabelle 5 dargestellt, die die Ergebnisse aus zwei unabhängigen Experimenten mit 14 Blättern zeigt.

**Tabelle 5**

| | **GUS-positive Epidermiszellen** | |
|---|---|---|
| **Konstrukt** | **Kontrolle** | **Inokuliert** |
| **pGUS** | **0** | **0** |
| **pIntronGUS** | **0** | **0** |
| **pUbiGUS** | **197** | **804** |
| **pGLP4GUS** | **0** | **35** |
| **pGLP4IntronGUS** | **0** | **79** |

Es zeigt sich, dass der erfindungsgemäße Promotor im Gegensatz zum Ubiquitinpromotor nur in Epidermiszellen aktiv ist, die mit Mehltau inokuliert worden waren, jedoch nicht in nicht-inokulierten Kontrollzellen. Durch die Fusion der erfindungsgemäßen Promotorregion mit dem Intron des WIR1a-Gens kann die Expression des Reportergens um mehr als das doppelte gesteigert werden.

### 8. Herstellung von binären Vektoren für die Transformation von Pflanzen

### a) p6UGLP4GUS (GUS-Expression unter der Kontrolle des erfindungsgemäßen Promotors)

Die vom erfindungsgemäßen Promotor kontrollierte Expressionskassette aus dem Vektor pGLP4GUS wurde mittels *Sfi*I Restriktionsschnittstellen in den binären Vektor (p6U der Firma "DNA-Cloning-Service", Hamburg, Deutschland) kloniert. Da die Kassette aus pGLP4GUS keine *Sfi*I Restriktionsschnittstellen besitzt, wurde sie zunächst zwischen die zwei *Sfi*I Restriktionsschnittstellen des Zwischenvektors pNOS-AB-M (Firma "DNA-Cloning-Service", Hamburg, Deutschland) kloniert. Zur Herstellung des Zwischenvektors pNOS-AB-M GLP4GUS wurde die 5945 bp lange Expressionskassette bestehend aus der erfindungsgemäßen Promotorsequenz, der GUS Reportersequenz und dem GstA1 Transkriptionsterminator aus dem Reportervektor pGLP4GUS mittels *Hind*III/*Not*I Restriktionsspaltung herausgeschnitten und über Agarose-Gelelektrophorese gereinigt. Die Kassette wurde in den mit *Hind*III/*Not*I geschnittenen und über Agarose-Gelelektrophorese gereinigten Zwischenvektor pNOS-AB-M (Firma "DNA-Cloning-Service", Hamburg, Deutschland) ligiert. Das resultierende Plasmid pNOS-AB-M GLP4GUS trug die Expressionskassette flankiert von zwei *Sfi*I Schnittstellen und wurde für die Konstruktion des binären Vektors p6UGLP4GUS eingesetzt.

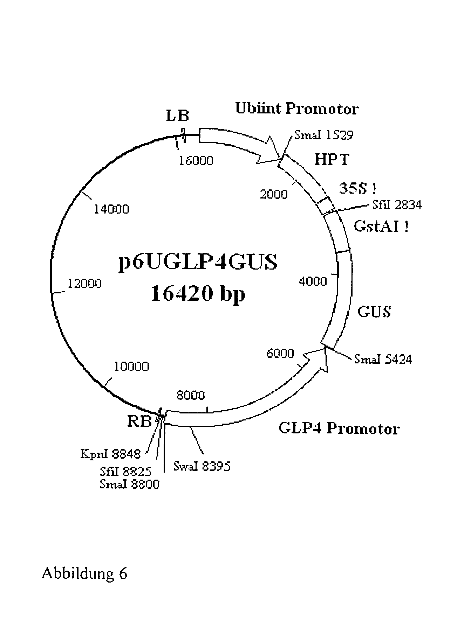

Die 5991 bp lange Expressionskassette bestehend aus der erfindungsgemäßen Promotorsequenz, der GUS-Reportersequenz und dem GstA1-Transkriptionsterminator wurde mit *Sfi*I aus dem Zwischenvektor pNOS-AB-M GLP4GUS herausgeschnitten und gerichtet mit den *Sfi*I Schnittstellen des binären Vektors p6U ligiert. Die Sequenz des resultierenden Plasmids p6UGLP4GUS ist in SEQ ID Nr. 18 angegeben, und die Struktur des Plasmides in Abb. 6 dargestellt.

### b) p6UGLP4IntronGUS (GUS-Expression unter der Kontrolle des erfindungsgemäßen Promotors und moduliert durch ein intronenthaltendes WIR1a-Genfragment)

Die Expressionskassette aus pGLP4IntronGUS (erfindungsgemäße Promotorsequenz, intronenthaltendes WIR1a-Genfragment, GUS Reportersequenz und GstA1 Transkriptionsterminator) wurde zunächst in den Zwischenvektor pNOS-AB-M (Firma "DNA-Cloning-Service", Hamburg, Deutschland) kloniert, um die Expressionskassette durch zusätzliche *Sfi*I Restriktionsschnittstellen zu flankieren. Die Kassette wurde dann als *Sfi*I Fragment in die *Sfi*I Schnittstelle des binären Vektors p6U (Firma "DNA-Cloning-Service", Hamburg, Deutschland) ligiert.

Zur Herstellung des Zwischenvektors pNOS-AB-M GLP4IntronGUS wurde die 6097 bp lange Expressionskassette (erfindungsgemäße Promotorsequenz, intronenthaltendes WIR1a-Genfragment, GUS Reportersequenz und GstA1-Transkriptionsterminator) aus pGLP4IntonGUS mittels *Hind*IIIl*Not*I Restriktionsspaltung herausgeschnitten und über Agarose-Gelelektrophorese gereinigt. Die Kassette wurde in den mit *Hind*IIIl*Not*I geschnittenen und über Agarose-Gelelektrophorese gereinigten Zwischenvektor pNOS-AB-M (Firma "DNA-Cloning-Service", Hamburg, Deutschland) ligiert. Das resultierende Plasmid pNOS-AB-M GLP4IntronGUS trug die Expressionskassette flankiert von zwei *Sfi*I Schnittstellen und wurde für die Konstruktion des binären Vektors p6UGLP4IntronGUS eingesetzt

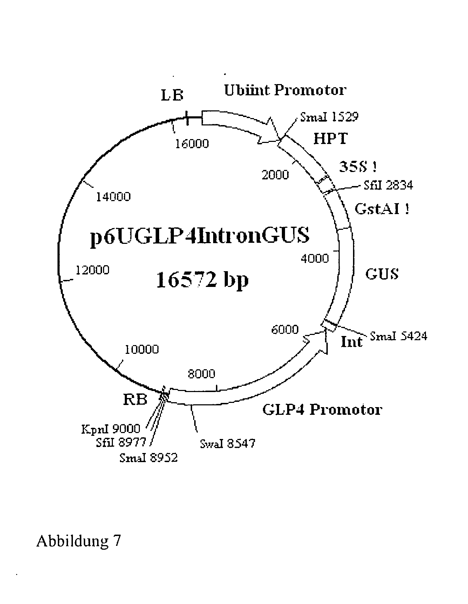

Die 6143 bp lange Expressionskassette (erfindungsgemäße Promotorsequenz, intronenthaltendes WIR1a-Genfragment, GUS Reportersequenz und GstA1 Transkriptionsterminator) wurde mit *Sfi*I aus dem Zwischenvektor pNOS-AB-M GLP4IntronGUS herausgeschnitten und gerichtet mit den *Sfi*I Schnittstellen des binären Vektors p6U ligiert. Die Sequenz des resultierenden Plasmids p6UGLP4IntronGUS ist in SEQ ID Nr. 19 angegeben, und die Struktur des Plasmids in Abb. 7 dargestellt.

### 9. Astrobacterium-vermittelte Transformation von Gerstepflanzen

Unreife Embryonen wurden in 2,5 ml CIM (Tingay et al. (1997) Plant J. 11: 1369-1376) enthaltend 800 mg/l Cystein und 500 µM Acetosyringon gesammelt Das Medium wurde entfernt und 600 µl Agrobacterium, das mit dem jeweiligen Plasmid transformiert worden war zugegeben. Anschließend fand eine Vakuuminfiltration für eine Minute bei 500 mbar statt, bevor die Embryonen 10 Minuten ruhen gelassen wurden. Danach wurden sie zweimal mit 2,5 ml CIM enthaltend 800 mg/l Cystein und 500 µM Acetosyringon gewaschen, bevor sie für 2-3 Tage bei 21 °C im Dunkeln in 2,5 ml CIM enthaltend 800 mg/l Cystein und 500 µM Acetosyringon kultiviert wurden. Anschließend wurden die Embryonen auf festes CIM (Trifinova et al. (2001) Plant Sci. 162: 871-880) enthaltend 150 mg/l Timentin und 50 mg/l Hygromycin übertragen. Die Kallusinduktion erfolgte für vier Wochen bei 24°C im Dunkeln. Anschließend erfolgte die Regeneration auf K4N (Kumlehn et al. (2006) Plant Biotechnology Journal 4: 251-261) mit 150 mg/l Timentin und 25 mg/l Hygromycin. Weitere Einzelheiten zur Transformation von Gerste können der Veröffentlichung von Hensel und Kumlehn, Genetic transformation ofbarley (Hordeum vulgare L.) by coculture of immature embryos with Agrobacteria. In: Curtis, I. S. (Ed.) Transgenic crops of the world- Essential protocols (2004), Kluwer, Dordrecht, Seiten 35-44 entnommen werden.

### 10. Inokulation der Blattsegmente der transgenen Gerstenpflanzen Golden Promise::GLP4GUS und Golden Promise::GLP4IntronGUS) mit Mehltau-Sporen

Blattsegmente der transgenen Gerstepflanzen (Golden Promise::GLP4GUS und Golden Promise::GLP4IntronGUS) wurden auf Phytoagar (1 % w/v) mit Benzimidazol (20 µg/ml) in Polycarbonat-Schalen gelegt. Die Infektion mit Mehltau-Sporen erfolgte in einem Inokulationsturm durch Abschütteln von Sporen stark infizierter Gerstenblätter. Nach 5 min wurden die Schalen geschlossen und bei +20°C und indirektem Tageslicht für 60 h inkubiert.

### 11. Histochemischer Nachweis der GUS-Expression in transgenen Gerstenpflanzen (Golden Promise::GLP4GUS und Golden Promise::GLP4IntronGUS)

Die Blätter der transgenen Gerstenpflanzen (Golden Promise::GLP4GUS und Golden Promise::GLP4IntronGUS) wurden unter Vakuum mit der GUS-Nachweislösung (10 mM EDTA, 1.4 mM K₃[Fe(CN)₆], 1.4 mM K₄[Fe(CN)₆], 0.1% (v/v) Triton X-100, 20 % (v/v) Methanol, 1 mg/ml 3-Bromo-4-chloro-3-indoxyl-β-D-glucuronsäure, 100 mM Na-Phosphatpuffer, pH 7.0) infiltriert und 24 h bei +37°C inkubiert. Nach Entfernen der Nachweislösung wurden die Blätter mit 7.5 % (w/v) Trichloressigsäure in 50 % (v/v) Methanol (15 min bei +20°C) und mit Ethanollösungen (20%, 40%, 60% und 80%) jeweils für 30 min bei 50°C entfärbt. Die Lichtmikroskopie erfolgte mit einem Zeiss Axiolab bei 100-facher Vergrösserung. Zellen mit GUS-Expression besitzen einen blau gefärbten Zellinhalt.

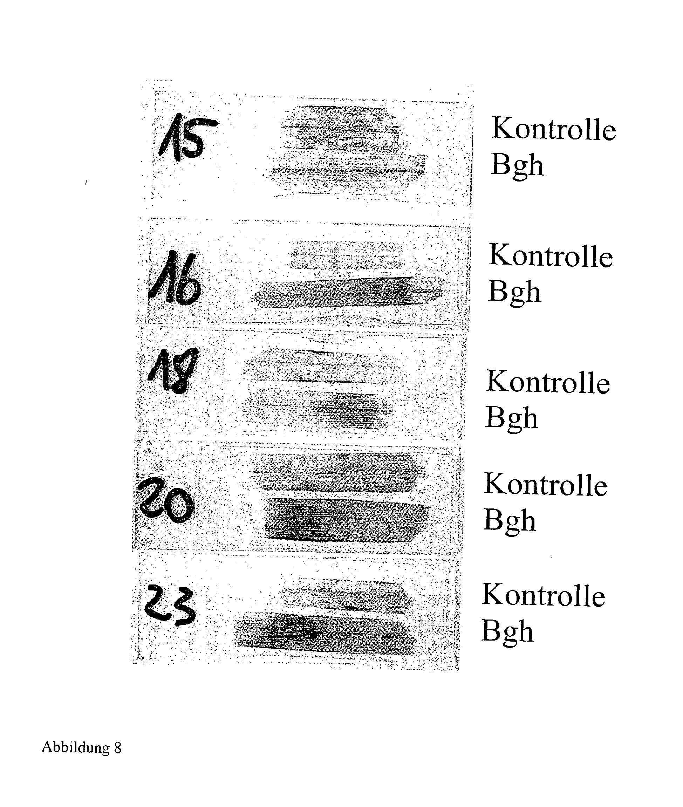

Die Ergebnisse der GUS-Färbung von Blättern transgener Pflanzen sind in Abbildung 8 dargestellt. Alle fünf analysierten transgenen Linien zeigten eine durch Mehltau induzierte GUS-Expression im Blatt, die in ihrer Stärke variierte.

## Patentansprüche

1. Pathogeninduzierbare Promotorregion mit Spezifität für die pflanzliche Epidermis, umfassend die unter SEQ ID Nr. 1 oder 16 angegebene Nukleinsäuresequenz.

2. Promotorregion nach Anspruch 1, oder Promotorregionen, die eine Sequenz aufweisen, die unter stringenten Bedingungen mit der unter SEQ ID Nr. 1 oder 16 angegebenen Nukleinsäuresequenz hybridisiert, wobei die Promotorregion eine Sequenzidentität von mindestens 90% zu der gesamten in SEQ ID Nr. 1 oder 16 angegebenen Nukleinsäuresequenz aufweist,
zusätzlich umfassend eine Sequenz ausgewählt aus der Gruppe bestehend aus
a) Sequenzen, die die unter SEQ ID Nr. 2 angegebene Nukleinsäuresequenz umfassen, und
b) Sequenzen, die unter stringenten Bedingungen mit der unter SEQ ID Nr. 2 angegebenen Nukleinsäuresequenz hybridisieren.

3. Promotorregion nach Anspruch 2, umfassend eine Sequenz ausgewählt aus der Gruppe bestehend aus
a) Promotorregionen, die die unter SEQ ID Nr. 3 oder 17 angegebene Nukleinsäuresequenz umfassen,
b) Promotorregionen, die einen funktionalen Teil der unter SEQ ID Nr. 3 oder 17 angegebenen Nukleinsäuresequenz umfassen, und
c) Promotorregionen, die eine Sequenz aufweisen, die unter stringenten Bedingungen mit der unter SEQ ID Nr. 3 oder 17 angegebenen Nukleinsäuresequenz hybridisiert.

4. Chimäres Gen,
**dadurch gekennzeichnet, dass** es eine Promotorregion nach einem der Ansprüche 1 bis 3 in operativer Verknüpfung mit einer kodierenden Sequenz enthält.

5. Chimäres Gen nach Anspruch 4,
**dadurch gekennzeichnet, dass** seine Expression zu einem erhöhten Gehalt des von der kodierenden Sequenz kodierten Proteins in der Epidermis führt.

6. Chimäres Gen nach Anspruch 4 oder 5,
**dadurch gekennzeichnet, dass** die kodierende Sequenz aus einem Reportergen stammt.

7. Chimäres Gen nach Anspruch 4 oder 5,
**dadurch gekennzeichnet, dass** die kodierende Sequenz aus einem Resistenzgen stammt.

8. Chimäres Gen,
**dadurch gekennzeichnet, dass** es eine Promotorregion nach einem der Ansprüche 1 bis 3 in operativer Verknupfung mit einer Nukleinsäuresequenz, deren Expression durch die Promotorregion reguliert wird, enthält, wobei eine Expression die Expression des entsprechenden endogenen Gens in der Epidermis unterdrückt.

9. Chimäres Gen nach Anspruch 8,
**dadurch gekennzeichnet, dass** die kodierende Sequenz in antisense-Orientierung vorliegt.

10. Chimäres Gen nach Anspruch 8,
**dadurch gekennzeichnet, dass** die Unterdrückung der Expression des endogenen Gens durch RNA-Interferenz erfolgt.

11. Rekombinantes Nukleinsäuremolekül, umfassend eine Promotorregion nach einem der Ansprüche 2 oder 3 oder ein chimäres Gen nach einem der Ansprüche 4 bis 10.

12. Rekombinantes Nukleinsäuremolekül nach Anspruch 11, zusätzlich umfassend transkriptionelle Terminationssequenzen.

13. Verfahren zur Herstellung transgener Pflanzen mit epidermisspezifischer, pathogeninduzierbarer Expression eines Transgens, umfassend die Schritte:
a) Herstellung eines rekombinanten Nukleinsäuremoleküls nach Anspruch 11 oder 12,
b) Übertragung des rekombinanten Nukleinsäuremoleküls aus a) auf pflanzliche Zellen und
c) Regeneration vollständig transformierter Pflanzen und, falls erwünscht, Vermehrung der Pflanzen

14. Transgene Pflanzen sowie transgene Teile dieser Pflanzen und deren transgenes Vermehrungsmaterial, wie Protoplasten, Pflanzenzellen, Kalli, Samen, Knollen oder Stecklinge, sowie die transgenen Nachkommen dieser Pflanze enthaltend ein
chimäres Gen nach einem der Ansprüche 4-10.

15. Transgene Pflanzen nach Anspruch 14, bei denen es sich um monokotyledone Pflanzen handelt.

16. Transgene Pflanzen nach Anspruch 15, bei denen es sich um Poaceen handelt.

17. Transgene Pflanzen nach Anspruch 16, bei denen es sich um Weizen oder Gerste handelt.

18. Verwendung einer Promotorregion nach einem der Ansprüche 1 bis 3 zur epidermisspezifischen Expression von Transgenen in Pflanzen.

19. Verwendung nach Anspruch 18, wobei das Transgen ein Resistenzgen ist.

20. Transgene Pflanzen nach einem der Ansprüche 14 bis 17,
**dadurch gekennzeichnet, dass** sie ein rekombinantes Nukleinsäuremolekül umfassend ein chimäres Gen nach Anspruch 7 enthalten und eine erhöhte Resistenz gegen Echten Mehltau zeigen.

21. Verwendung eines chimären Gens nach Anspruch 6 zur Untersuchung von pathogeninduzierten Signaltransduktionswegen in Pflanzenzellen.

## Claims

1. Pathogen-inducible promoter region having specificity for the plant epidermis, comprising the nucleic acid sequence depicted in SEQ ID NO: 1 or 16.

2. Promoter region according to claim 1, or promoter regions having a sequence which hybridises under stringent conditions with the nucleic acid sequence depicted in SEQ ID NO: 1 or 16, wherein the promoter region has a sequence identity of at least 90% to the total nucleic acid sequence depicted in SEQ ID NO: 1 or 16,
additionally comprising a sequence selected from the group consisting of
a) sequences comprising the nucleic acid sequence depicted in SEQ ID NO: 2, and
b) sequences hybridising under stringent conditions with the nucleic acid sequence depicted in SEQ ID NO: 2.

3. Promoter region according to claim 2 comprising a sequence selected from the group consisting of
a) promoter regions comprising the nucleic acid sequence depicted in SEQ ID NO: 3 or 17,
b) promoter regions comprising a functional part of the nucleic acid sequence depicted in SEQ ID NO: 3 or 17, and
c) promoter regions having a sequence hybridising under stringent conditions with the nucleic acid sequence depicted in SEQ ID NO: 3 or 17.

4. Chimeric gene,
**characterized in that** it contains a promoter region according to any one of claims 1 to 3 in operative linkage with a coding sequence.

5. Chimeric gene according to claim 4,
**characterized in that** its expression leads to an increased content of the protein encoded by the coding sequence in the epidermis.

6. Chimeric gene according to claim 4 or 5,
**characterized in that** the coding sequence originates from a reporter gene.

7. Chimeric gene according to claim 4 or 5,
**characterized in that** the coding sequence originates from a resistance gene.

8. Chimeric gene,
**characterized in that** it contains a promoter region according to any one of claims 1 to 3 in operative linkage with a nucleic acid sequence, the expression of which is regulated by the promoter region, wherein its expression inhibits the expression of the corresponding endogenous gene in the epidermis.

9. Chimeric gene according to claim 8,
**characterized in that** the coding sequence is present in antisense orientation.

10. Chimeric gene according to claim 8,
**characterized in that** inhibiting the expression of the endogenous gene is performed by means of RNA interference.

11. Recombinant nucleic acid molecule comprising a promoter region according to claim 2 or 3 or a chimeric gene according to any one of claims 4 to 10.

12. Recombinant nucleic acid molecule according to claim 11, additionally comprising transcriptional termination sequences.

13. Method for the generation of transgenic plants with epidermis-specific, pathogen-inducible expression of a transgene, comprising the steps:
a) generating a recombinant nucleic acid molecule according to claim 11 or 12,
b) transferring the recombinant nucleic acid molecule from a) to plant cells and
c) regenerating entirely transformed plants and, if desired, propagating the plants.

14. Transgenic plants as well as transgenic parts of said plants and their transgenic propagation material, like protoplasts, plant cells, calli, seeds, tubers or seedlings, as well as the transgenic progeny of said plant containing a chimeric gene according to any one of claims 4 to 10.

15. Transgenic plants according to claim 14 which are monocotyledonous plants.

16. Transgenic plants according to claim 15 which are poaceae.

17. Transgenic plants according to claim 16, which are wheat or barley.

18. Use of a promoter region according to any one of claims 1 to 3 for epidermis-specific expression of transgenes in plants.

19. Use according to claim 18, wherein the transgene is a resistance gene.

20. Transgenic plants according to any one of claims 14 to 17, **characterized in that** they contain a recombinant nucleic acid molecule comprising a chimeric gene according to claim 7 and exhibit an increased resistance to powdery mildew.

21. Use of a chimeric gene according to claim 6 for assaying pathogen-induced signal transduction pathways in plant cells.

## Revendications

1. Région promotrice inductible par pathogène présentant une spécificité pour l'épiderme végétal, comportant la séquence d'acide nucléique indiquée dans SEQ ID N° 1 ou 16.

2. Région promotrice selon la revendication 1, ou régions promotrices présentant une séquence qui s'hybride en conditions stringentes avec la séquence d'acide nucléique indiquée dans SEQ ID N° 1 ou 16, la région promotrice présentant une identité de séquence d'au moins 90 % à la séquence d'acide nucléique totale indiquée dans SEQ ID n° 1 ou 16,
comportant en outre une séquence sélectionnée dans le groupe constitué de
a) séquences comportant la séquence d'acide nucléique indiquée dans SEQ ID N° 2, et
b) séquences s'hybridant en conditions stringentes avec la séquence d'acide nucléique indiquée dans SEQ ID N° 2.

3. Région promotrice selon la revendication 2, comportant une séquence sélectionnée dans le groupe constitué de
a) régions promotrices comportant la séquence d'acide nucléique indiquée dans SEQ ID N° 3 ou 17, et
b) régions promotrices comportant une partie fonctionnelle de la séquence d'acide nucléique indiquée dans SEQ ID N° 3 ou 17, et
b) régions promotrices présentant une séquence qui s'hybride en conditions stringentes avec la séquence d'acide nucléique indiquée dans SEQ ID N° 3 ou 17.

4. Gène chimère,
**caractérisé en ce qu'**il contient une région promotrice selon l'une des revendications 1 à 3 en liaison opérante avec une séquence codante.

5. Gène chimère selon la revendication 4,
**caractérisé en ce que** son expression entraîne une teneur accrue de la protéine codée par la séquence codante dans l'épiderme.

6. Gène chimère selon la revendication 4 ou 5,
**caractérisé en ce que** la séquence codante provient d'un gène rapporteur.

7. Gène chimère selon la revendication 4 ou 5,
**caractérisé en ce que** la séquence codante provient d'un gène de résistance.

8. Gène chimère,
**caractérisé en ce qu'**il contient une région promotrice selon l'une des revendications 1 à 3 en liaison opérante avec une séquence d'acide nucléique dont l'expression est régulée par la région promotrice, son expression inhibant l'expression du gène endogène correspondant dans l'épiderme.

9. Gène chimère selon la revendication 8,
**caractérisé en ce que** la séquence codante est présente en orientation antisens.

10. Gène chimère selon la revendication 8,
**caractérisé en ce que** l'inhibition de l'expression du gène endogène se fait par interférence d'ARN.

11. Molécule d'acide nucléique recombinante, comportant une région promotrice selon l'une des revendications 2 ou 3 ou un gène chimère selon l'une des revendications 4 à 10.

12. Molécule d'acide nucléique recombinante selon la revendication 11, comportant en outre des séquences de terminaison transcriptionnelles.

13. Procédé de fabrication de plantes transgéniques à expression inductible par pathogène et spécifique à l'épiderme d'un transgène, comportant les étapes :
a) fabrication d'une molécule d'acide nucléique recombinante selon la revendication 11 ou 12,
b) transmission de la molécule d'acide nucléique recombinante issue de a) à des cellules végétales et
c) regénération de plantes complètement transformées et, si souhaité, multiplication des plantes.

14. Plantes transgéniques ainsi que parties transgéniques de ces plantes et leur matériau de multiplication transgénique, telles que protoplastes, cellules végétales, kalli, semences, bulbes ou boutures, ainsi que les descendants transgéniques de ces plantes contenant un gène chimère selon l'une des revendications 4 à 10.

15. Plantes transgéniques selon la revendication 14, où il s'agit de plantes monokotylédones.

16. Plantes transgéniques selon la revendication 15, où il s'agit de poacées.

17. Plantes transgéniques selon la revendication 16, où il s'agit de blé ou d'orge.

18. Utilisation d'une région promotrice selon l'une des revendications 1 à 3 pour l'expression spécifique à l'épiderme de transgènes dans des plantes.

19. Utilisation selon la revendication 18, le transgène étant un gène de résistance.

20. Plantes transgéniques selon l'une des revendications 14 à 17,
**caractérisées en ce qu'**elles contiennent une molécule d'acide nucléique recombinante comportant un gène chimère selon la revendication 7 et présentent une résistance accrue contre l'oïdium.

21. Utilisation d'un gène chimère selon la revendication 6 en vue d'examiner des voies de transduction de signal inductibles par pathogène dans des cellules végétales.


### SEQUENCE LISTING

<110> Institut für Pflanzengenetik und Kulturpflanzenforschung
<120> Promotor zur epidermisspezifischen, pathogeninduzierbaren
   Transgenexpression in Pflanzen
<130> I7646/RN
<150> DE 10 2005 025 656
   <151> 2005-06-03
<160> 19
<170> PatentIn version 3.3
<210> 1
   <211> 3334
   <212> DNA
   <213> Hordeum vulgare
<400> 1
   cgtgcgtaaa ttaagggcat gattgagttc ctagatatgc atcctaattt ataaaataaa      60
   aggagcatat gtaattgtgt gcctgtaatc agtgaatgta tttctacctt ttaaataatc     120
   aaaaatattg ttttttatca cactgatatg atctcacatg cttttttttg gttctactgt     180
   gtcgtatttc tcataattga tcaatatttt gggttctttt ccccttattc tgaaataata     240
   tatgattagg ttaaaaaaaa gaaacaatac aagattattt ggtaatgcag tatttgtttg     300
   acgctggtgt tgaacacgta ttattatctg gcaatacatg tgaagtatgc gatgggtgga     360
   gcccgattgt gctcaggatt taaatgtgaa tgtggaatgt gcaaaagttg cttcctggac     420
   ttgttgttct ggtctctcgc tttctggtgc atgaaattag aaatatacag ttcctccatg     480
   taatattttt agcttccttt tggttcaaag ctcttgtaag ttcgattgac ggctgctttg     540
   atggaaatcg atgaggtggc tcattttatc taaattctta tgagtagtat ttttgatttt     600
   cttaagttgg gaactaaaaa cacgtttttt tactaagttt tggaaactgg gcacaacgga     660
   gtcgagattt taaacattag ctccaacata catataaaaa atatgcataa atgtagaaaa     720
   taaacttatt accggaagtg cctaatgaca cacctcgagc atgctggcgg aagggagcag 780
   atgagaagct ggaccacctt aagactcaac caggaccgat gacgacattc atccacgagc 840
   cacctggaag aaaacacctg acttcgtcgt actgtcacct ttgaactaca gttgcatgtc 900
   ggagacgacc cgctgcttaa accaagtagt gtcttgacca taccagcaga catgatgaaa 960
   ggaggacatt gttgcatgta ctacaagata tgtgcatgca ataccgtgag tcatttgatt 1020
   taaggcaggg tcgattggat cgccatacca ttccgaacac accaccgtaa atggagagtg 1080
   ttgattgcac acattctgta gcaacttaaa agaatggaga gtgttagaac tagccgcttg 1140
   ccgtagatca gatcgccgtc gtacgccaac acaactataa ccgaaactag aacaaaacct 1200
   gcacgctacg agctaaaagc aagcaagctg gctggtggat cgattgttgg gctagctagc 1260
   tatggaactg atgtattctg ccgtccggct agataaatct caatcggaac tacaccaaag 1320
   tatagcacgc aaacacacga gataatctta caggagagga gatcaagcta agcagacgac 1380
   tttgtctgtg tagaaaacga ttgctttaca taacttacat ctctcaactc ctctttgacc 1440
   tcgtgtgcat cagttctcac catgaccaag tatacgtata tgatatctaa taaactatga 1500
   tgatgggtgg cgacttttca tgaaaccagc gctccatgca ttgtgctgac ttggttgaga 1560
   tggataaata attgtaatta ctactcagcc atacatcgga tgatgcatta gcgcttaagc 1620
   atattaaaaa gcaaacttga aaagtttatg caaaatacta ttcagaagcc aatagatgat 1680
   tatttagtaa tgtgctcaga ggtttcaacg tgcaagttga atgtgcaaac gttgcttcct 1740
   tgagtcgttg ttgtggtctg cctttccggg gcatgtaata agtagtttac ggtagttcct 1800
   ggagtatagc ccacatactt gggctatact tctctcagtg ataaaacttc tctctgtgta 1860
   ccatcacgag tttactcata gatttcttcc gtaactaata gtgtcaagtt atacacttat 1920
   atatacctta gttttctcta aaagaaaccg atatacctta cttgatgtcc ttctcttaat 1980
   ctagaaaggt gttaattata aatatttcca tcttgagtgt ttgtatcctc cgtatatgtt 2040
   ttgattacag gctcaaagtt tcaattgaag gttttcttga tgttttttat cccaatacat 2100
   ctgggactcc aacgggaata tttacaactt gcactgagat tcgttgtatt ctctgtctcc 2160
   tagaaatttg aggacactgt ttattttgtg aagaaatgaa taagacggac gactctaggg 2220
   cggtagttat gttttttatg gggcactgaa cctttcggcc tttctgttgt aattctgaat 2280
   atgccttatt tttctcgcaa aaaataaaga atatgccttg tttttatgca gtcgtctagc 2340
   tttgaatgtg ttccagtgtc ggttttggag cgagtaaata actttctgaa tgtaagcact 2400
   tgaacttgag ctatcttgtc gtgttggctt gaaactgccc agccctggac gagttctcgt 2460
   tcccggagaa gacgatggtg gtgctgggca gttaggaggg catcccggtg gacatcatcc 2520
   aggaggcggt ggacgtgtgc gtcgagatcc cgcagctggg cgtcgtccgg tcgctcaacg 2580
   tccgtgtcag cgccgccatc gccatctggg actacacccg ccagcagcgg gcccgctcct 2640
   cctcctcgcg gtaggtagct tcccgctctt ccttgttgga gtgtaaatca gccatgcttg 2700
   ttgttgtaag atgcatggac ctgaacatcc atggcaaaaa ctagtcatga accttagtca 2760
   ggactagatt gttagattta tgaactgaat tggtcatgac gaaaattctt tggcaagaaa 2820
   attttaactg ctcttgctct ccatcgtcca ttaattaatg tcgttgcttc ttccttcaag 2880
   tactcaacca tttggagaga aatttgacca ggcaactgta tatctggttg acgtaatcat 2940
   atgatacgta ttaggtgcgt taatgcatgg atcgacgcgc catgatttct tccttgtttg 3000
   acacaatcaa aactattgct catagaaatt gttactaaga ggtctacatc acatggagta 3060
   ccttaatttg cttctctatt tattaaaggg aaaaacgtgt atcaaagatt tgaatatggc 3120
   tgaccacatc tcaaaagttt atgcagacta atctagtcgg cagccaaaga ccatttctat 3180
   ccgttgggcg gttccacttg actatgtcca ttactcatga gttccacctg ctcgccatgc 3240
   ctatataaaa acatacatcc ccagcatgtt ccaaccatca ctcacccaac aaacatctca 3300
   ggaatatagg agaaaacaag acccaaagga gctg 3334
<210> 2
   <211> 114
   <212> DNA
   <213> Triticum sp.
<400> 2
   gtcagtcgtc ggacggtgtc cgttcatttc ctccccattt ttgtaattga ttaacttgtt 60
   atacatgctg acctcgacct gctgaataac gtccgtccat ggtttcccgt ccag 114
<210> 3
   <211> 3497
   <212> DNA
   <213> Artificial
<220>
   <223> GLP4 Promotor + WIR1a Exon/Intron
<400> 3
   cgtgcgtaaa ttaagggcat gattgagttc ctagatatgc atcctaattt ataaaataaa      60
   aggagcatat gtaattgtgt gcctgtaatc agtgaatgta tttctacctt ttaaataatc     120
   aaaaatattg ttttttatca cactgatatg atctcacatg cttttttttg gttctactgt     180
   gtcgtatttc tcataattga tcaatatttt gggttctttt ccccttattc tgaaataata     240
   tatgattagg ttaaaaaaaa gaaacaatac aagattattt ggtaatgcag tatttgtttg     300
   acgctggtgt tgaacacgta ttattatctg gcaatacatg tgaagtatgc gatgggtgga     360
   gcccgattgt gctcaggatt taaatgtgaa tgtggaatgt gcaaaagttg cttcctggac     420
   ttgttgttct ggtctctcgc tttctggtgc atgaaattag aaatatacag ttcctccatg     480
   taatattttt agcttccttt tggttcaaag ctcttgtaag ttcgattgac ggctgctttg     540
   atggaaatcg atgaggtggc tcattttatc taaattctta tgagtagtat ttttgatttt     600
   cttaagttgg gaactaaaaa cacgtttttt tactaagttt tggaaactgg gcacaacgga     660
   gtcgagattt taaacattag ctccaacata catataaaaa atatgcataa atgtagaaaa     720
   taaacttatt accggaagtg cctaatgaca cacctcgagc atgctggcgg aagggagcag     780
   atgagaagct ggaccacctt aagactcaac caggaccgat gacgacattc atccacgagc 840
   cacctggaag aaaacacctg acttcgtcgt actgtcacct ttgaactaca gttgcatgtc 900
   ggagacgacc cgctgcttaa accaagtagt gtcttgacca taccagcaga catgatgaaa 960
   ggaggacatt gttgcatgta ctacaagata tgtgcatgca ataccgtgag tcatttgatt 1020
   taaggcaggg tcgattggat cgccatacca ttccgaacac accaccgtaa atggagagtg 1080
   ttgattgcac acattctgta gcaacttaaa agaatggaga gtgttagaac tagccgcttg 1140
   ccgtagatca gatcgccgtc gtacgccaac acaactataa ccgaaactag aacaaaacct 1200
   gcacgctacg agctaaaagc aagcaagctg gctggtggat cgattgttgg gctagctagc 1260
   tatggaactg atgtattctg ccgtccggct agataaatct caatcggaac tacaccaaag 1320
   tatagcacgc aaacacacga gataatctta caggagagga gatcaagcta agcagacgac 1380
   tttgtctgtg tagaaaacga ttgctttaca taacttacat ctctcaactc ctctttgacc 1440
   tcgtgtgcat cagttctcac catgaccaag tatacgtata tgatatctaa taaactatga 1500
   tgatgggtgg cgacttttca tgaaaccagc gctccatgca ttgtgctgac ttggttgaga 1560
   tggataaata attgtaatta ctactcagcc atacatcgga tgatgcatta gcgcttaagc 1620
   atattaaaaa gcaaacttga aaagtttatg caaaatacta ttcagaagcc aatagatgat 1680
   tatttagtaa tgtgctcaga ggtttcaacg tgcaagttga atgtgcaaac gttgcttcct 1740
   tgagtcgttg ttgtggtctg cctttccggg gcatgtaata agtagtttac ggtagttcct 1800
   ggagtatagc ccacatactt gggctatact tctctcagtg ataaaacttc tctctgtgta 1860
   ccatcacgag tttactcata gatttcttcc gtaactaata gtgtcaagtt atacacttat 1920
   atatacctta gttttctcta aaagaaaccg atatacctta cttgatgtcc ttctcttaat 1980
   ctagaaaggt gttaattata aatatttcca tcttgagtgt ttgtatcctc cgtatatgtt 2040
   ttgattacag gctcaaagtt tcaattgaag gttttcttga tgttttttat cccaatacat 2100
   ctgggactcc aacgggaata tttacaactt gcactgagat tcgttgtatt ctctgtctcc 2160
   tagaaatttg aggacactgt ttattttgtg aagaaatgaa taagacggac gactctaggg 2220
   cggtagttat gttttttatg gggcactgaa cctttcggcc tttctgttgt aattctgaat 2280
   atgccttatt tttctcgcaa aaaataaaga atatgccttg tttttatgca gtcgtctagc 2340
   tttgaatgtg ttccagtgtc ggttttggag cgagtaaata actttctgaa tgtaagcact 2400
   tgaacttgag ctatcttgtc gtgttggctt gaaactgccc agccctggac gagttctcgt 2460
   tcccggagaa gacgatggtg gtgctgggca gttaggaggg catcccggtg gacatcatcc 2520
   aggaggcggt ggacgtgtgc gtcgagatcc cgcagctggg cgtcgtccgg tcgctcaacg 2580
   tccgtgtcag cgccgccatc gccatctggg actacacccg ccagcagcgg gcccgctcct 2640
   cctcctcgcg gtaggtagct tcccgctctt ccttgttgga gtgtaaatca gccatgcttg 2700
   ttgttgtaag atgcatggac ctgaacatcc atggcaaaaa ctagtcatga accttagtca 2760
   ggactagatt gttagattta tgaactgaat tggtcatgac gaaaattctt tggcaagaaa 2820
   attttaactg ctcttgctct ccatcgtcca ttaattaatg tcgttgcttc ttccttcaag 2880
   tactcaacca tttggagaga aatttgacca ggcaactgta tatctggttg acgtaatcat 2940
   atgatacgta ttaggtgcgt taatgcatgg atcgacgcgc catgatttct tccttgtttg 3000
   acacaatcaa aactattgct catagaaatt gttactaaga ggtctacatc acatggagta 3060
   ccttaatttg cttctctatt tattaaaggg aaaaacgtgt atcaaagatt tgaatatggc 3120
   tgaccacatc tcaaaagttt atgcagacta atctagtcgg cagccaaaga ccatttctat 3180
   ccgttgggcg gttccacttg actatgtcca ttactcatga gttccacctg ctcgccatgc 3240
   ctatataaaa acatacatcc ccagcatgtt ccaaccatca ctcacccaac aaacatctca 3300
   ggaatatagg agaaaacaag acccaaagga gctgatcgaa ttcctgcagg gagccacggc 3360
   cgtccacgac gccgccgcct caggtcagtc gtcggacggt gtccgttcat ttcctcccca 3420
   tttttgtaat tgattaactt gttatacatg ctgacctcga cctgctgaat aacgtccgtc 3480
   catggtttcc cgtccag 3497
<210> 4
   <211> 8841
   <212> DNA
   <213> Artificial
<220>
   <223> pGLP4GUS
<220>
   <221> promoter
   <222> (692)..(4025)
   <223> GLP4 promoter
<220>
   <221> gene
   <222> (4060)..(5871)
   <223> GUS
<220>
   <221> terminator
   <222> (5872)..(6612)
   <223> GstA1 terminator
<400> 4
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tgggtaccgg 660
   gccccccctc gacggtatcg ataagcttga tcgtgcgtaa attaagggca tgattgagtt 720
   cctagatatg catcctaatt tataaaataa aaggagcata tgtaattgtg tgcctgtaat 780
   cagtgaatgt atttctacct tttaaataat caaaaatatt gttttttatc acactgatat 840
   gatctcacat gctttttttt ggttctactg tgtcgtattt ctcataattg atcaatattt 900
   tgggttcttt tccccttatt ctgaaataat atatgattag gttaaaaaaa agaaacaata 960
   caagattatt tggtaatgca gtatttgttt gacgctggtg ttgaacacgt attattatct 1020
   ggcaatacat gtgaagtatg cgatgggtgg agcccgattg tgctcaggat ttaaatgtga 1080
   atgtggaatg tgcaaaagtt gcttcctgga cttgttgttc tggtctctcg ctttctggtg 1140
   catgaaatta gaaatataca gttcctccat gtaatatttt tagcttcctt ttggttcaaa 1200
   gctcttgtaa gttcgattga cggctgcttt gatggaaatc gatgaggtgg ctcattttat 1260
   ctaaattctt atgagtagta tttttgattt tcttaagttg ggaactaaaa acacgttttt 1320
   ttactaagtt ttggaaactg ggcacaacgg agtcgagatt ttaaacatta gctccaacat 1380
   acatataaaa aatatgcata aatgtagaaa ataaacttat taccggaagt gcctaatgac 1440
   acacctcgag catgctggcg gaagggagca gatgagaagc tggaccacct taagactcaa 1500
   ccaggaccga tgacgacatt catccacgag ccacctggaa gaaaacacct gacttcgtcg 1560
   tactgtcacc tttgaactac agttgcatgt cggagacgac ccgctgctta aaccaagtag 1620
   tgtcttgacc ataccagcag acatgatgaa aggaggacat tgttgcatgt actacaagat 1680
   atgtgcatgc aataccgtga gtcatttgat ttaaggcagg gtcgattgga tcgccatacc 1740
   attccgaaca caccaccgta aatggagagt gttgattgca cacattctgt agcaacttaa 1800
   aagaatggag agtgttagaa ctagccgctt gccgtagatc agatcgccgt cgtacgccaa 1860
   cacaactata accgaaacta gaacaaaacc tgcacgctac gagctaaaag caagcaagct 1920
   ggctggtgga tcgattgttg ggctagctag ctatggaact gatgtattct gccgtccggc 1980
   tagataaatc tcaatcggaa ctacaccaaa gtatagcacg caaacacacg agataatctt 2040
   acaggagagg agatcaagct aagcagacga ctttgtctgt gtagaaaacg attgctttac 2100
   ataacttaca tctctcaact cctctttgac ctcgtgtgca tcagttctca ccatgaccaa 2160
   gtatacgtat atgatatcta ataaactatg atgatgggtg gcgacttttc atgaaaccag 2220
   cgctccatgc attgtgctga cttggttgag atggataaat aattgtaatt actactcagc   2280
   catacatcgg atgatgcatt agcgcttaag catattaaaa agcaaacttg aaaagtttat   2340
   gcaaaatact attcagaagc caatagatga ttatttagta atgtgctcag aggtttcaac   2400
   gtgcaagttg aatgtgcaaa cgttgcttcc ttgagtcgtt gttgtggtct gcctttccgg   2460
   ggcatgtaat aagtagttta cggtagttcc tggagtatag cccacatact tgggctatac   2520
   ttctctcagt gataaaactt ctctctgtgt accatcacga gtttactcat agatttcttc   2580
   cgtaactaat agtgtcaagt tatacactta tatatacctt agttttctct aaaagaaacc   2640
   gatatacctt acttgatgtc cttctcttaa tctagaaagg tgttaattat aaatatttcc   2700
   atcttgagtg tttgtatcct ccgtatatgt tttgattaca ggctcaaagt ttcaattgaa   2760
   ggttttcttg atgtttttta tcccaataca tctgggactc caacgggaat atttacaact   2820
   tgcactgaga ttcgttgtat tctctgtctc ctagaaattt gaggacactg tttattttgt   2880
   gaagaaatga ataagacgga cgactctagg gcggtagtta tgttttttat ggggcactga   2940
   acctttcggc ctttctgttg taattctgaa tatgccttat ttttctcgca aaaaataaag   3000
   aatatgcctt gtttttatgc agtcgtctag ctttgaatgt gttccagtgt cggttttgga   3060
   gcgagtaaat aactttctga atgtaagcac ttgaacttga gctatcttgt cgtgttggct   3120
   tgaaactgcc cagccctgga cgagttctcg ttcccggaga agacgatggt ggtgctgggc   3180
   agttaggagg gcatcccggt ggacatcatc caggaggcgg tggacgtgtg cgtcgagatc   3240
   ccgcagctgg gcgtcgtccg gtcgctcaac gtccgtgtca gcgccgccat cgccatctgg   3300
   gactacaccc gccagcagcg ggcccgctcc tcctcctcgc ggtaggtagc ttcccgctct   3360
   tccttgttgg agtgtaaatc agccatgctt gttgttgtaa gatgcatgga cctgaacatc   3420
   catggcaaaa actagtcatg aaccttagtc aggactagat tgttagattt atgaactgaa   3480
   ttggtcatga cgaaaattct ttggcaagaa aattttaact gctcttgctc tccatcgtcc   3540
   attaattaat gtcgttgctt cttccttcaa gtactcaacc atttggagag aaatttgacc   3600
   aggcaactgt atatctggtt gacgtaatca tatgatacgt attaggtgcg ttaatgcatg   3660
   gatcgacgcg ccatgatttc ttccttgttt gacacaatca aaactattgc tcatagaaat 3720
   tgttactaag aggtctacat cacatggagt accttaattt gcttctctat ttattaaagg 3780
   gaaaaacgtg tatcaaagat ttgaatatgg ctgaccacat ctcaaaagtt tatgcagact 3840
   aatctagtcg gcagccaaag accatttcta tccgttgggc ggttccactt gactatgtcc 3900
   attactcatg agttccacct gctcgccatg cctatataaa aacatacatc cccagcatgt 3960
   tccaaccatc actcacccaa caaacatctc aggaatatag gagaaaacaa gacccaaagg 4020
   agctgatcga attcctgcag cccgggtggt cagtccctta tgttacgtcc tgtagaaacc 4080
   ccaacccgtg aaatcaaaaa actcgacggc ctgtgggcat tcagtctgga tcgcgaaaac 4140
   tgtggaattg atcagcgttg gtgggaaagc gcgttacaag aaagccgggc aattgctgtg 4200
   ccaggcagtt ttaacgatca gttcgccgat gcagatattc gtaattatgc gggcaacgtc 4260
   tggtatcagc gcgaagtctt tataccgaaa ggttgggcag gccagcgtat cgtgctgcgt 4320
   ttcgatgcgg tcactcatta cggcaaagtg tgggtcaata atcaggaagt gatggagcat 4380
   cagggcggct atacgccatt tgaagccgat gtcacgccgt atgttattgc cgggaaaagt 4440
   gtacgtatca ccgtttgtgt gaacaacgaa ctgaactggc agactatccc gccgggaatg 4500
   gtgattaccg acgaaaacgg caagaaaaag cagtcttact tccatgattt ctttaactat 4560
   gccggaatcc atcgcagcgt aatgctctac accacgccga acacctgggt ggacgatatc 4620
   accgtggtga cgcatgtcgc gcaagactgt aaccacgcgt ctgttgactg gcaggtggtg 4680
   gccaatggtg atgtcagcgt tgaactgcgt gatgcggatc aacaggtggt tgcaactgga 4740
   caaggcacta gcgggacttt gcaagtggtg aatccgcacc tctggcaacc gggtgaaggt 4800
   tatctctatg aactgtgcgt cacagccaaa agccagacag agtgtgatat ctacccgctt 4860
   cgcgtcggca tccggtcagt ggcagtgaag ggcgaacagt tcctgattaa ccacaaaccg 4920
   ttctacttta ctggctttgg tcgtcatgaa gatgcggact tgcgtggcaa aggattcgat 4980
   aacgtgctga tggtgcacga ccacgcatta atggactgga ttggggccaa ctcctaccgt 5040
   acctcgcatt acccttacgc tgaagagatg ctcgactggg cagatgaaca tggcatcgtg 5100
   gtgattgatg aaactgctgc tgtcggcttt aacctctctt taggcattgg tttcgaagcg 5160
   ggcaacaagc cgaaagaact gtacagcgaa gaggcagtca acggggaaac tcagcaagcg 5220
   cacttacagg cgattaaaga gctgatagcg cgtgacaaaa accacccaag cgtggtgatg 5280
   tggagtattg ccaacgaacc ggatacccgt ccgcaaggtg cacgggaata tttcgcgcca 5340
   ctggcggaag caacgcgtaa actcgacccg acgcgtccga tcacctgcgt caatgtaatg 5400
   ttctgcgacg ctcacaccga taccatcagc gatctctttg atgtgctgtg cctgaaccgt 5460
   tattacggat ggtatgtcca aagcggcgat ttggaaacgg cagagaaggt actggaaaaa 5520
   gaacttctgg cctggcagga gaaactgcat cagccgatta tcatcaccga atacggcgtg 5580
   gatacgttag ccgggctgca ctcaatgtac accgacatgt ggagtgaaga gtatcagtgt 5640
   gcatggctgg atatgtatca ccgcgtcttt gatcgcgtca gcgccgtcgt cggtgaacag 5700
   gtatggaatt tcgccgattt tgcgacctcg caaggcatat tgcgcgttgg cggtaacaag 5760
   aaagggatct tcactcgcga ccgcaaaccg aagtcggcgg cttttctgct gcaaaaacgc 5820
   tggactggca tgaacttcgg tgaaaaaccg cagcagggag gcaaacaatg agagctacta 5880
   gtatgcctac cgagtttaag ctaggtgcgc agtactaggt gtttatgcac tccgcgcggg 5940
   tgtgtgccgt gccgtgccgt gccgtgccgg tgatgattcc gttcaagaaa agagacctcc 6000
   ctcccgtggg tgggtgatca tctgcccgtc tacttccagt agtatacgct ttcagtttca 6060
   gcagcacgag gagtccgctt gcgcttctgt tcgtgcgctg gattccttgc tttaatttat 6120
   gcactagtac tagttagtaa ataaacaacg gactggtcaa tgctgctaat ccgtactgta 6180
   cgtcctatat agtagtaccc cactagagat cagtatatat atacatcttg gatacgtgtg 6240
   catttgcatg caaatatata tggacaggga taactggcac atgtgtgccc gttaggaaaa 6300
   ttacgatcgc tctccttcgg tcggtggccc gctcgctcga tcgagcgttc cttcggtcgg 6360
   tgtcccgctc gcccttcggt ccgtggcccg ctcgctcgat cgagcgttcc ttcggtcggt 6420
   gtcccgctcg ctcgatcgag cgttgaggcc agttggctcg atcgctccgc tttgatcgag 6480
   ttgtgaatgc tttcaccggt ttttttttct tctgattttt attttcagaa cggtctacta 6540
   aatagtcact acacccaata accaactcgg ccatcctacc ttgttatcca caatacgtga   6600
   acaacggaag ttgaattcta gtctacgcgg ccgcgagctc cagcttttgt tccctttagt   6660
   gagggttaat tgcgcgcttg gcgtaatcat ggtcatagct gtttcctgtg tgaaattgtt   6720
   atccgctcac aattccacac aacatacgag ccggaagcat aaagtgtaaa gcctggggtg   6780
   cctaatgagt gagctaactc acattaattg cgttgcgctc actgcccgct ttccagtcgg   6840
   gaaacctgtc gtgccagctg cattaatgaa tcggccaacg cgcggggaga ggcggtttgc   6900
   gtattgggcg ctcttccgct tcctcgctca ctgactcgct gcgctcggtc gttcggctgc   6960
   ggcgagcggt atcagctcac tcaaaggcgg taatacggtt atccacagaa tcaggggata   7020
   acgcaggaaa gaacatgtga gcaaaaggcc agcaaaaggc caggaaccgt aaaaaggccg   7080
   cgttgctggc gtttttccat aggctccgcc cccctgacga gcatcacaaa aatcgacgct   7140
   caagtcagag gtggcgaaac ccgacaggac tataaagata ccaggcgttt ccccctggaa   7200
   gctccctcgt gcgctctcct gttccgaccc tgccgcttac cggatacctg tccgcctttc   7260
   tcccttcggg aagcgtggcg ctttctcata gctcacgctg taggtatctc agttcggtgt   7320
   aggtcgttcg ctccaagctg ggctgtgtgc acgaaccccc cgttcagccc gaccgctgcg   7380
   ccttatccgg taactatcgt cttgagtcca acccggtaag acacgactta tcgccactgg   7440
   cagcagccac tggtaacagg attagcagag cgaggtatgt aggcggtgct acagagttct   7500
   tgaagtggtg gcctaactac ggctacacta gaaggacagt atttggtatc tgcgctctgc   7560
   tgaagccagt taccttcgga aaaagagttg gtagctcttg atccggcaaa caaaccaccg   7620
   ctggtagcgg tggttttttt gtttgcaagc agcagattac gcgcagaaaa aaaggatctc   7680
   aagaagatcc tttgatcttt tctacggggt ctgacgctca gtggaacgaa aactcacgtt   7740
   aagggatttt ggtcatgaga ttatcaaaaa ggatcttcac ctagatcctt ttaaattaaa   7800
   aatgaagttt taaatcaatc taaagtatat atgagtaaac ttggtctgac agttaccaat   7860
   gcttaatcag tgaggcacct atctcagcga tctgtctatt tcgttcatcc atagttgcct   7920
   gactccccgt cgtgtagata actacgatac gggagggctt accatctggc cccagtgctg   7980
   caatgatacc gcgagaccca cgctcaccgg ctccagattt atcagcaata aaccagccag 8040
   ccggaagggc cgagcgcaga agtggtcctg caactttatc cgcctccatc cagtctatta 8100
   attgttgccg ggaagctaga gtaagtagtt cgccagttaa tagtttgcgc aacgttgttg 8160
   ccattgctac aggcatcgtg gtgtcacgct cgtcgtttgg tatggcttca ttcagctccg 8220
   gttcccaacg atcaaggcga gttacatgat cccccatgtt gtgcaaaaaa gcggttagct 8280
   ccttcggtcc tccgatcgtt gtcagaagta agttggccgc agtgttatca ctcatggtta 8340
   tggcagcact gcataattct cttactgtca tgccatccgt aagatgcttt tctgtgactg 8400
   gtgagtactc aaccaagtca ttctgagaat agtgtatgcg gcgaccgagt tgctcttgcc 8460
   cggcgtcaat acgggataat accgcgccac atagcagaac tttaaaagtg ctcatcattg 8520
   gaaaacgttc ttcggggcga aaactctcaa ggatcttacc gctgttgaga tccagttcga 8580
   tgtaacccac tcgtgcaccc aactgatctt cagcatcttt tactttcacc agcgtttctg 8640
   ggtgagcaaa aacaggaagg caaaatgccg caaaaaaggg aataagggcg acacggaaat 8700
   gttgaatact catactcttc ctttttcaat attattgaag catttatcag ggttattgtc 8760
   tcatgagcgg atacatattt gaatgtattt agaaaaataa acaaataggg gttccgcgca 8820
   catttccccg aaaagtgcca c 8841
<210> 5
   <211> 8993
   <212> DNA
   <213> Artificial
<220>
   <223> pGLP4IntronGUS
<220>
   <221> promoter
   <222> (692)..(4025)
   <223> GLP4 promoter
<220>
   <221> misc_feature
   <222> (4040)..(4193)
   <223> WIR1 part of 5'CDS + intron
<220>
   <221> gene
   <222> (4212)..(6023)
   <223> GUS
<220>
   <221> terminator
   <222> (6024)..(6764)
   <223> GstA1 terminator
<400> 5
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tgggtaccgg 660
   gccccccctc gacggtatcg ataagcttga tcgtgcgtaa attaagggca tgattgagtt 720
   cctagatatg catcctaatt tataaaataa aaggagcata tgtaattgtg tgcctgtaat 780
   cagtgaatgt atttctacct tttaaataat caaaaatatt gttttttatc acactgatat 840
   gatctcacat gctttttttt ggttctactg tgtcgtattt ctcataattg atcaatattt 900
   tgggttcttt tccccttatt ctgaaataat atatgattag gttaaaaaaa agaaacaata 960
   caagattatt tggtaatgca gtatttgttt gacgctggtg ttgaacacgt attattatct 1020
   ggcaatacat gtgaagtatg cgatgggtgg agcccgattg tgctcaggat ttaaatgtga 1080
   atgtggaatg tgcaaaagtt gcttcctgga cttgttgttc tggtctctcg ctttctggtg 1140
   catgaaatta gaaatataca gttcctccat gtaatatttt tagcttcctt ttggttcaaa 1200
   gctcttgtaa gttcgattga cggctgcttt gatggaaatc gatgaggtgg ctcattttat 1260
   ctaaattctt atgagtagta tttttgattt tcttaagttg ggaactaaaa acacgttttt 1320
   ttactaagtt ttggaaactg ggcacaacgg agtcgagatt ttaaacatta gctccaacat 1380
   acatataaaa aatatgcata aatgtagaaa ataaacttat taccggaagt gcctaatgac 1440
   acacctcgag catgctggcg gaagggagca gatgagaagc tggaccacct taagactcaa 1500
   ccaggaccga tgacgacatt catccacgag ccacctggaa gaaaacacct gacttcgtcg 1560
   tactgtcacc tttgaactac agttgcatgt cggagacgac ccgctgctta aaccaagtag 1620
   tgtcttgacc ataccagcag acatgatgaa aggaggacat tgttgcatgt actacaagat 1680
   atgtgcatgc aataccgtga gtcatttgat ttaaggcagg gtcgattgga tcgccatacc 1740
   attccgaaca caccaccgta aatggagagt gttgattgca cacattctgt agcaacttaa 1800
   aagaatggag agtgttagaa ctagccgctt gccgtagatc agatcgccgt cgtacgccaa 1860
   cacaactata accgaaacta gaacaaaacc tgcacgctac gagctaaaag caagcaagct 1920
   ggctggtgga tcgattgttg ggctagctag ctatggaact gatgtattct gccgtccggc 1980
   tagataaatc tcaatcggaa ctacaccaaa gtatagcacg caaacacacg agataatctt 2040
   acaggagagg agatcaagct aagcagacga ctttgtctgt gtagaaaacg attgctttac 2100
   ataacttaca tctctcaact cctctttgac ctcgtgtgca tcagttctca ccatgaccaa 2160
   gtatacgtat atgatatcta ataaactatg atgatgggtg gcgacttttc atgaaaccag 2220
   cgctccatgc attgtgctga cttggttgag atggataaat aattgtaatt actactcagc 2280
   catacatcgg atgatgcatt agcgcttaag catattaaaa agcaaacttg aaaagtttat 2340
   gcaaaatact attcagaagc caatagatga ttatttagta atgtgctcag aggtttcaac 2400
   gtgcaagttg aatgtgcaaa cgttgcttcc ttgagtcgtt gttgtggtct gcctttccgg 2460
   ggcatgtaat aagtagttta cggtagttcc tggagtatag cccacatact tgggctatac 2520
   ttctctcagt gataaaactt ctctctgtgt accatcacga gtttactcat agatttcttc 2580
   cgtaactaat agtgtcaagt tatacactta tatatacctt agttttctct aaaagaaacc 2640
   gatatacctt acttgatgtc cttctcttaa tctagaaagg tgttaattat aaatatttcc 2700
   atcttgagtg tttgtatcct ccgtatatgt tttgattaca ggctcaaagt ttcaattgaa 2760
   ggttttcttg atgtttttta tcccaataca tctgggactc caacgggaat atttacaact 2820
   tgcactgaga ttcgttgtat tctctgtctc ctagaaattt gaggacactg tttattttgt 2880
   gaagaaatga ataagacgga cgactctagg gcggtagtta tgttttttat ggggcactga 2940
   acctttcggc ctttctgttg taattctgaa tatgccttat ttttctcgca aaaaataaag 3000
   aatatgcctt gtttttatgc agtcgtctag ctttgaatgt gttccagtgt cggttttgga 3060
   gcgagtaaat aactttctga atgtaagcac ttgaacttga gctatcttgt cgtgttggct 3120
   tgaaactgcc cagccctgga cgagttctcg ttcccggaga agacgatggt ggtgctgggc 3180
   agttaggagg gcatcccggt ggacatcatc caggaggcgg tggacgtgtg cgtcgagatc 3240
   ccgcagctgg gcgtcgtccg gtcgctcaac gtccgtgtca gcgccgccat cgccatctgg 3300
   gactacaccc gccagcagcg ggcccgctcc tcctcctcgc ggtaggtagc ttcccgctct 3360
   tccttgttgg agtgtaaatc agccatgctt gttgttgtaa gatgcatgga cctgaacatc 3420
   catggcaaaa actagtcatg aaccttagtc aggactagat tgttagattt atgaactgaa 3480
   ttggtcatga cgaaaattct ttggcaagaa aattttaact gctcttgctc tccatcgtcc 3540
   attaattaat gtcgttgctt cttccttcaa gtactcaacc atttggagag aaatttgacc 3600
   aggcaactgt atatctggtt gacgtaatca tatgatacgt attaggtgcg ttaatgcatg 3660
   gatcgacgcg ccatgatttc ttccttgttt gacacaatca aaactattgc tcatagaaat 3720
   tgttactaag aggtctacat cacatggagt accttaattt gcttctctat ttattaaagg 3780
   gaaaaacgtg tatcaaagat ttgaatatgg ctgaccacat ctcaaaagtt tatgcagact 3840
   aatctagtcg gcagccaaag accatttcta tccgttgggc ggttccactt gactatgtcc 3900
   attactcatg agttccacct gctcgccatg cctatataaa aacatacatc cccagcatgt 3960
   tccaaccatc actcacccaa caaacatctc aggaatatag gagaaaacaa gacccaaagg 4020
   agctgatcga attcctgcag ggagccacgg ccgtccacga cgccgccgcc tcaggtcagt 4080
   cgtcggacgg tgtccgttca tttcctcccc atttttgtaa ttgattaact tgttatacat 4140
   gctgacctcg acctgctgaa taacgtccgt ccatggtttc ccgtccaggc accccgggtg 4200
   gtcagtccct tatgttacgt cctgtagaaa ccccaacccg tgaaatcaaa aaactcgacg 4260
   gcctgtgggc attcagtctg gatcgcgaaa actgtggaat tgatcagcgt tggtgggaaa 4320
   gcgcgttaca agaaagccgg gcaattgctg tgccaggcag ttttaacgat cagttcgccg 4380
   atgcagatat tcgtaattat gcgggcaacg tctggtatca gcgcgaagtc tttataccga 4440
   aaggttgggc aggccagcgt atcgtgctgc gtttcgatgc ggtcactcat tacggcaaag 4500
   tgtgggtcaa taatcaggaa gtgatggagc atcagggcgg ctatacgcca tttgaagccg 4560
   atgtcacgcc gtatgttatt gccgggaaaa gtgtacgtat caccgtttgt gtgaacaacg 4620
   aactgaactg gcagactatc ccgccgggaa tggtgattac cgacgaaaac ggcaagaaaa 4680
   agcagtctta cttccatgat ttctttaact atgccggaat ccatcgcagc gtaatgctct 4740
   acaccacgcc gaacacctgg gtggacgata tcaccgtggt gacgcatgtc gcgcaagact 4800
   gtaaccacgc gtctgttgac tggcaggtgg tggccaatgg tgatgtcagc gttgaactgc 4860
   gtgatgcgga tcaacaggtg gttgcaactg gacaaggcac tagcgggact ttgcaagtgg 4920
   tgaatccgca cctctggcaa ccgggtgaag gttatctcta tgaactgtgc gtcacagcca 4980
   aaagccagac agagtgtgat atctacccgc ttcgcgtcgg catccggtca gtggcagtga 5040
   agggcgaaca gttcctgatt aaccacaaac cgttctactt tactggcttt ggtcgtcatg 5100
   aagatgcgga cttgcgtggc aaaggattcg ataacgtgct gatggtgcac gaccacgcat 5160
   taatggactg gattggggcc aactcctacc gtacctcgca ttacccttac gctgaagaga 5220
   tgctcgactg ggcagatgaa catggcatcg tggtgattga tgaaactgct gctgtcggct 5280
   ttaacctctc tttaggcatt ggtttcgaag cgggcaacaa gccgaaagaa ctgtacagcg 5340
   aagaggcagt caacggggaa actcagcaag cgcacttaca ggcgattaaa gagctgatag 5400
   cgcgtgacaa aaaccaccca agcgtggtga tgtggagtat tgccaacgaa ccggataccc 5460
   gtccgcaagg tgcacgggaa tatttcgcgc cactggcgga agcaacgcgt aaactcgacc 5520
   cgacgcgtcc gatcacctgc gtcaatgtaa tgttctgcga cgctcacacc gataccatca 5580
   gcgatctctt tgatgtgctg tgcctgaacc gttattacgg atggtatgtc caaagcggcg 5640
   atttggaaac ggcagagaag gtactggaaa aagaacttct ggcctggcag gagaaactgc 5700
   atcagccgat tatcatcacc gaatacggcg tggatacgtt agccgggctg cactcaatgt 5760
   acaccgacat gtggagtgaa gagtatcagt gtgcatggct ggatatgtat caccgcgtct 5820
   ttgatcgcgt cagcgccgtc gtcggtgaac aggtatggaa tttcgccgat tttgcgacct 5880
   cgcaaggcat attgcgcgtt ggcggtaaca agaaagggat cttcactcgc gaccgcaaac 5940
   cgaagtcggc ggcttttctg ctgcaaaaac gctggactgg catgaacttc ggtgaaaaac 6000
   cgcagcaggg aggcaaacaa tgagagctac tagtatgcct accgagttta agctaggtgc 6060
   gcagtactag gtgtttatgc actccgcgcg ggtgtgtgcc gtgccgtgcc gtgccgtgcc 6120
   ggtgatgatt ccgttcaaga aaagagacct ccctcccgtg ggtgggtgat catctgcccg 6180
   tctacttcca gtagtatacg ctttcagttt cagcagcacg aggagtccgc ttgcgcttct 6240
   gttcgtgcgc tggattcctt gctttaattt atgcactagt actagttagt aaataaacaa 6300
   cggactggtc aatgctgcta atccgtactg tacgtcctat atagtagtac cccactagag 6360
   atcagtatat atatacatct tggatacgtg tgcatttgca tgcaaatata tatggacagg 6420
   gataactggc acatgtgtgc ccgttaggaa aattacgatc gctctccttc ggtcggtggc 6480
   ccgctcgctc gatcgagcgt tccttcggtc ggtgtcccgc tcgcccttcg gtccgtggcc 6540
   cgctcgctcg atcgagcgtt ccttcggtcg gtgtcccgct cgctcgatcg agcgttgagg 6600
   ccagttggct cgatcgctcc gctttgatcg agttgtgaat gctttcaccg gttttttttt 6660
   cttctgattt ttattttcag aacggtctac taaatagtca ctacacccaa taaccaactc 6720
   ggccatccta ccttgttatc cacaatacgt gaacaacgga agttgaattc tagtctacgc 6780
   ggccgcgagc tccagctttt gttcccttta gtgagggtta attgcgcgct tggcgtaatc 6840
   atggtcatag ctgtttcctg tgtgaaattg ttatccgctc acaattccac acaacatacg 6900
   agccggaagc ataaagtgta aagcctgggg tgcctaatga gtgagctaac tcacattaat 6960
   tgcgttgcgc tcactgcccg ctttccagtc gggaaacctg tcgtgccagc tgcattaatg 7020
   aatcggccaa cgcgcgggga gaggcggttt gcgtattggg cgctcttccg cttcctcgct 7080
   cactgactcg ctgcgctcgg tcgttcggct gcggcgagcg gtatcagctc actcaaaggc 7140
   ggtaatacgg ttatccacag aatcagggga taacgcagga aagaacatgt gagcaaaagg 7200
   ccagcaaaag gccaggaacc gtaaaaaggc cgcgttgctg gcgtttttcc ataggctccg 7260
   cccccctgac gagcatcaca aaaatcgacg ctcaagtcag aggtggcgaa acccgacagg 7320
   actataaaga taccaggcgt ttccccctgg aagctccctc gtgcgctctc ctgttccgac 7380
   cctgccgctt accggatacc tgtccgcctt tctcccttcg ggaagcgtgg cgctttctca 7440
   tagctcacgc tgtaggtatc tcagttcggt gtaggtcgtt cgctccaagc tgggctgtgt 7500
   gcacgaaccc cccgttcagc ccgaccgctg cgccttatcc ggtaactatc gtcttgagtc 7560
   caacccggta agacacgact tatcgccact ggcagcagcc actggtaaca ggattagcag 7620
   agcgaggtat gtaggcggtg ctacagagtt cttgaagtgg tggcctaact acggctacac 7680
   tagaaggaca gtatttggta tctgcgctct gctgaagcca gttaccttcg gaaaaagagt 7740
   tggtagctct tgatccggca aacaaaccac cgctggtagc ggtggttttt ttgtttgcaa 7800
   gcagcagatt acgcgcagaa aaaaaggatc tcaagaagat cctttgatct tttctacggg 7860
   gtctgacgct cagtggaacg aaaactcacg ttaagggatt ttggtcatga gattatcaaa 7920
   aaggatcttc acctagatcc ttttaaatta aaaatgaagt tttaaatcaa tctaaagtat 7980
   atatgagtaa acttggtctg acagttacca atgcttaatc agtgaggcac ctatctcagc 8040
   gatctgtcta tttcgttcat ccatagttgc ctgactcccc gtcgtgtaga taactacgat 8100
   acgggagggc ttaccatctg gccccagtgc tgcaatgata ccgcgagacc cacgctcacc 8160
   ggctccagat ttatcagcaa taaaccagcc agccggaagg gccgagcgca gaagtggtcc 8220
   tgcaacttta tccgcctcca tccagtctat taattgttgc cgggaagcta gagtaagtag 8280
   ttcgccagtt aatagtttgc gcaacgttgt tgccattgct acaggcatcg tggtgtcacg 8340
   ctcgtcgttt ggtatggctt cattcagctc cggttcccaa cgatcaaggc gagttacatg 8400
   atcccccatg ttgtgcaaaa aagcggttag ctccttcggt cctccgatcg ttgtcagaag 8460
   taagttggcc gcagtgttat cactcatggt tatggcagca ctgcataatt ctcttactgt 8520
   catgccatcc gtaagatgct tttctgtgac tggtgagtac tcaaccaagt cattctgaga 8580
   atagtgtatg cggcgaccga gttgctcttg cccggcgtca atacgggata ataccgcgcc 8640
   acatagcaga actttaaaag tgctcatcat tggaaaacgt tcttcggggc gaaaactctc 8700
   aaggatctta ccgctgttga gatccagttc gatgtaaccc actcgtgcac ccaactgatc 8760
   ttcagcatct tttactttca ccagcgtttc tgggtgagca aaaacaggaa ggcaaaatgc 8820
   cgcaaaaaag ggaataaggg cgacacggaa atgttgaata ctcatactct tcctttttca 8880
   atattattga agcatttatc agggttattg tctcatgagc ggatacatat ttgaatgtat 8940
   ttagaaaaat aaacaaatag gggttccgcg cacatttccc cgaaaagtgc cac 8993
<210> 6
   <211> 5488
   <212> DNA
   <213> Artificial
<220>
   <223> pGUS
<220>
   <221> gene
   <222> (707)..(2518)
   <223> GUS
<220>
   <221> terminator
<222> (2519)..(3259)
   <223> GstA1 terminator
<400> 6
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tgggtaccgg 660
   gccccccctc gagcacattt aaatcaaccc gggtggtcag tcccttatgt tacgtcctgt 720
   agaaacccca acccgtgaaa tcaaaaaact cgacggcctg tgggcattca gtctggatcg 780
   cgaaaactgt ggaattgatc agcgttggtg ggaaagcgcg ttacaagaaa gccgggcaat 840
   tgctgtgcca ggcagtttta acgatcagtt cgccgatgca gatattcgta attatgcggg 900
   caacgtctgg tatcagcgcg aagtctttat accgaaaggt tgggcaggcc agcgtatcgt 960
   gctgcgtttc gatgcggtca ctcattacgg caaagtgtgg gtcaataatc aggaagtgat 1020
   ggagcatcag ggcggctata cgccatttga agccgatgtc acgccgtatg ttattgccgg 1080
   gaaaagtgta cgtatcaccg tttgtgtgaa caacgaactg aactggcaga ctatcccgcc 1140
   gggaatggtg attaccgacg aaaacggcaa gaaaaagcag tcttacttcc atgatttctt 1200
   taactatgcc ggaatccatc gcagcgtaat gctctacacc acgccgaaca cctgggtgga 1260
   cgatatcacc gtggtgacgc atgtcgcgca agactgtaac cacgcgtctg ttgactggca 1320
   ggtggtggcc aatggtgatg tcagcgttga actgcgtgat gcggatcaac aggtggttgc 1380
   aactggacaa ggcactagcg ggactttgca agtggtgaat ccgcacctct ggcaaccggg 1440
   tgaaggttat ctctatgaac tgtgcgtcac agccaaaagc cagacagagt gtgatatcta 1500
   cccgcttcgc gtcggcatcc ggtcagtggc agtgaagggc gaacagttcc tgattaacca 1560
   caaaccgttc tactttactg gctttggtcg tcatgaagat gcggacttgc gtggcaaagg 1620
   attcgataac gtgctgatgg tgcacgacca cgcattaatg gactggattg gggccaactc 1680
   ctaccgtacc tcgcattacc cttacgctga agagatgctc gactgggcag atgaacatgg 1740
   catcgtggtg attgatgaaa ctgctgctgt cggctttaac ctctctttag gcattggttt 1800
   cgaagcgggc aacaagccga aagaactgta cagcgaagag gcagtcaacg gggaaactca 1860
   gcaagcgcac ttacaggcga ttaaagagct gatagcgcgt gacaaaaacc acccaagcgt 1920
   ggtgatgtgg agtattgcca acgaaccgga tacccgtccg caaggtgcac gggaatattt 1980
   cgcgccactg gcggaagcaa cgcgtaaact cgacccgacg cgtccgatca cctgcgtcaa 2040
   tgtaatgttc tgcgacgctc acaccgatac catcagcgat ctctttgatg tgctgtgcct 2100
   gaaccgttat tacggatggt atgtccaaag cggcgatttg gaaacggcag agaaggtact 2160
   ggaaaaagaa cttctggcct ggcaggagaa actgcatcag ccgattatca tcaccgaata 2220
   cggcgtggat acgttagccg ggctgcactc aatgtacacc gacatgtgga gtgaagagta 2280
   tcagtgtgca tggctggata tgtatcaccg cgtctttgat cgcgtcagcg ccgtcgtcgg 2340
   tgaacaggta tggaatttcg ccgattttgc gacctcgcaa ggcatattgc gcgttggcgg 2400
   taacaagaaa gggatcttca ctcgcgaccg caaaccgaag tcggcggctt ttctgctgca 2460
   aaaacgctgg actggcatga acttcggtga aaaaccgcag cagggaggca aacaatgaga 2520
   gctactagta tgcctaccga gtttaagcta ggtgcgcagt actaggtgtt tatgcactcc 2580
   gcgcgggtgt gtgccgtgcc gtgccgtgcc gtgccggtga tgattccgtt caagaaaaga 2640
   gacctccctc ccgtgggtgg gtgatcatct gcccgtctac ttccagtagt atacgctttc 2700
   agtttcagca gcacgaggag tccgcttgcg cttctgttcg tgcgctggat tccttgcttt 2760
   aatttatgca ctagtactag ttagtaaata aacaacggac tggtcaatgc tgctaatccg 2820
   tactgtacgt cctatatagt agtaccccac tagagatcag tatatatata catcttggat 2880
   acgtgtgcat ttgcatgcaa atatatatgg acagggataa ctggcacatg tgtgcccgtt 2940
   aggaaaatta cgatcgctct ccttcggtcg gtggcccgct cgctcgatcg agcgttcctt 3000
   cggtcggtgt cccgctcgcc cttcggtccg tggcccgctc gctcgatcga gcgttccttc 3060
   ggtcggtgtc ccgctcgctc gatcgagcgt tgaggccagt tggctcgatc gctccgcttt 3120
   gatcgagttg tgaatgcttt caccggtttt tttttcttct gatttttatt ttcagaacgg 3180
   tctactaaat agtcactaca cccaataacc aactcggcca tcctaccttg ttatccacaa 3240
   tacgtgaaca acggaagttg aattctagtc tacgcggccg cgagctccag cttttgttcc 3300
   ctttagtgag ggttaattgc gcgcttggcg taatcatggt catagctgtt tcctgtgtga 3360
   aattgttatc cgctcacaat tccacacaac atacgagccg gaagcataaa gtgtaaagcc 3420
   tggggtgcct aatgagtgag ctaactcaca ttaattgcgt tgcgctcact gcccgctttc 3480
   cagtcgggaa acctgtcgtg ccagctgcat taatgaatcg gccaacgcgc ggggagaggc 3540
   ggtttgcgta ttgggcgctc ttccgcttcc tcgctcactg actcgctgcg ctcggtcgtt 3600
   cggctgcggc gagcggtatc agctcactca aaggcggtaa tacggttatc cacagaatca 3660
   ggggataacg caggaaagaa catgtgagca aaaggccagc aaaaggccag gaaccgtaaa 3720
   aaggccgcgt tgctggcgtt tttccatagg ctccgccccc ctgacgagca tcacaaaaat 3780
   cgacgctcaa gtcagaggtg gcgaaacccg acaggactat aaagatacca ggcgtttccc 3840
   cctggaagct ccctcgtgcg ctctcctgtt ccgaccctgc cgcttaccgg atacctgtcc 3900
   gcctttctcc cttcgggaag cgtggcgctt tctcatagct cacgctgtag gtatctcagt 3960
   tcggtgtagg tcgttcgctc caagctgggc tgtgtgcacg aaccccccgt tcagcccgac 4020
   cgctgcgcct tatccggtaa ctatcgtctt gagtccaacc cggtaagaca cgacttatcg 4080
   ccactggcag cagccactgg taacaggatt agcagagcga ggtatgtagg cggtgctaca 4140
   gagttcttga agtggtggcc taactacggc tacactagaa ggacagtatt tggtatctgc 4200
   gctctgctga agccagttac cttcggaaaa agagttggta gctcttgatc cggcaaacaa 4260
   accaccgctg gtagcggtgg tttttttgtt tgcaagcagc agattacgcg cagaaaaaaa 4320
   ggatctcaag aagatccttt gatcttttct acggggtctg acgctcagtg gaacgaaaac 4380
   tcacgttaag ggattttggt catgagatta tcaaaaagga tcttcaccta gatcctttta 4440
   aattaaaaat gaagttttaa atcaatctaa agtatatatg agtaaacttg gtctgacagt 4500
   taccaatgct taatcagtga ggcacctatc tcagcgatct gtctatttcg ttcatccata 4560
   gttgcctgac tccccgtcgt gtagataact acgatacggg agggcttacc atctggcccc 4620
   agtgctgcaa tgataccgcg agacccacgc tcaccggctc cagatttatc agcaataaac 4680
   cagccagccg gaagggccga gcgcagaagt ggtcctgcaa ctttatccgc ctccatccag 4740
   tctattaatt gttgccggga agctagagta agtagttcgc cagttaatag tttgcgcaac 4800
   gttgttgcca ttgctacagg catcgtggtg tcacgctcgt cgtttggtat ggcttcattc 4860
   agctccggtt cccaacgatc aaggcgagtt acatgatccc ccatgttgtg caaaaaagcg 4920
   gttagctcct tcggtcctcc gatcgttgtc agaagtaagt tggccgcagt gttatcactc 4980
   atggttatgg cagcactgca taattctctt actgtcatgc catccgtaag atgcttttct 5040
   gtgactggtg agtactcaac caagtcattc tgagaatagt gtatgcggcg accgagttgc 5100
   tcttgcccgg cgtcaatacg ggataatacc gcgccacata gcagaacttt aaaagtgctc 5160
   atcattggaa aacgttcttc ggggcgaaaa ctctcaagga tcttaccgct gttgagatcc 5220
   agttcgatgt aacccactcg tgcacccaac tgatcttcag catcttttac tttcaccagc 5280
   gtttctgggt gagcaaaaac aggaaggcaa aatgccgcaa aaaagggaat aagggcgaca 5340
   cggaaatgtt gaatactcat actcttcctt tttcaatatt attgaagcat ttatcagggt 5400
   tattgtctca tgagcggata catatttgaa tgtatttaga aaaataaaca aataggggtt 5460
   ccgcgcacat ttccccgaaa agtgccac 5488
<210> 7
   <211> 5646
   <212> DNA
   <213> Artificial
<220>
   <223> pIntronGUS
<220>
   <221> misc_feature
   <222> (694)..(846)
   <223> WIR1 part of 5'CDS + intron
<220>
   <221> gene
   <222> (865)..(2676)
   <223> GUS
<220>
   <221> terminator
   <222> (2677)..(3417)
   <223> GstA1 terminator
<400> 7
   ctaaattgta agcgttaata ttttgttaaa attcgcgtta aatttttgtt aaatcagctc      60
   attttttaac caataggccg aaatcggcaa aatcccttat aaatcaaaag aatagaccga      120
   gatagggttg agtgttgttc cagtttggaa caagagtcca ctattaaaga acgtggactc     180
   caacgtcaaa gggcgaaaaa ccgtctatca gggcgatggc ccactacgtg aaccatcacc     240
   ctaatcaagt tttttggggt cgaggtgccg taaagcacta aatcggaacc ctaaagggag     300
   cccccgattt agagcttgac ggggaaagcc ggcgaacgtg gcgagaaagg aagggaagaa     360
   agcgaaagga gcgggcgcta gggcgctggc aagtgtagcg gtcacgctgc gcgtaaccac     420
   cacacccgcc gcgcttaatg cgccgctaca gggcgcgtcc cattcgccat tcaggctgcg     480
   caactgttgg gaagggcgat cggtgcgggc ctcttcgcta ttacgccagc tggcgaaagg     540
   gggatgtgct gcaaggcgat taagttgggt aacgccaggg ttttcccagt cacgacgttg     600
   taaaacgacg gccagtgagc gcgcgtaata cgactcacta tagggcgaat tgggtaccgg     660
   gccccccctc gagctcattt aaatcctctg cagggagcca cggccgtcca cgacgccgcc     720
   gcctcaggtc agtcgtcgga cggtgtccgt tcatttcctc cccatttttg taattgatta     780
   acttgttata catgctgacc tcgacctgct gaataacgtc cgtccatggt ttcccgtcca     840
   ggcaccccgg gtggtcagtc ccttatgtta cgtcctgtag aaaccccaac ccgtgaaatc     900
   aaaaaactcg acggcctgtg ggcattcagt ctggatcgcg aaaactgtgg aattgatcag     960
   cgttggtggg aaagcgcgtt acaagaaagc cgggcaattg ctgtgccagg cagttttaac 1020
   gatcagttcg ccgatgcaga tattcgtaat tatgcgggca acgtctggta tcagcgcgaa 1080
   gtctttatac cgaaaggttg ggcaggccag cgtatcgtgc tgcgtttcga tgcggtcact 1140
   cattacggca aagtgtgggt caataatcag gaagtgatgg agcatcaggg cggctatacg 1200
   ccatttgaag ccgatgtcac gccgtatgtt attgccggga aaagtgtacg tatcaccgtt 1260
   tgtgtgaaca acgaactgaa ctggcagact atcccgccgg gaatggtgat taccgacgaa 1320
   aacggcaaga aaaagcagtc ttacttccat gatttcttta actatgccgg aatccatcgc 1380
   agcgtaatgc tctacaccac gccgaacacc tgggtggacg atatcaccgt ggtgacgcat 1440
   gtcgcgcaag actgtaacca cgcgtctgtt gactggcagg tggtggccaa tggtgatgtc 1500
   agcgttgaac tgcgtgatgc ggatcaacag gtggttgcaa ctggacaagg cactagcggg 1560
   actttgcaag tggtgaatcc gcacctctgg caaccgggtg aaggttatct ctatgaactg 1620
   tgcgtcacag ccaaaagcca gacagagtgt gatatctacc cgcttcgcgt cggcatccgg 1680
   tcagtggcag tgaagggcga acagttcctg attaaccaca aaccgttcta ctttactggc 1740
   tttggtcgtc atgaagatgc ggacttgcgt ggcaaaggat tcgataacgt gctgatggtg 1800
   cacgaccacg cattaatgga ctggattggg gccaactcct accgtacctc gcattaccct 1860
   tacgctgaag agatgctcga ctgggcagat gaacatggca tcgtggtgat tgatgaaact 1920
   gctgctgtcg gctttaacct ctctttaggc attggtttcg aagcgggcaa caagccgaaa 1980
   gaactgtaca gcgaagaggc agtcaacggg gaaactcagc aagcgcactt acaggcgatt 2040
   aaagagctga tagcgcgtga caaaaaccac ccaagcgtgg tgatgtggag tattgccaac 2100
   gaaccggata cccgtccgca aggtgcacgg gaatatttcg cgccactggc ggaagcaacg 2160
   cgtaaactcg acccgacgcg tccgatcacc tgcgtcaatg taatgttctg cgacgctcac 2220
   accgatacca tcagcgatct ctttgatgtg ctgtgcctga accgttatta cggatggtat 2280
   gtccaaagcg gcgatttgga aacggcagag aaggtactgg aaaaagaact tctggcctgg 2340
   caggagaaac tgcatcagcc gattatcatc accgaatacg gcgtggatac gttagccggg 2400
   ctgcactcaa tgtacaccga catgtggagt gaagagtatc agtgtgcatg gctggatatg 2460
   tatcaccgcg tctttgatcg cgtcagcgcc gtcgtcggtg aacaggtatg gaatttcgcc 2520
   gattttgcga cctcgcaagg catattgcgc gttggcggta acaagaaagg gatcttcact 2580
   cgcgaccgca aaccgaagtc ggcggctttt ctgctgcaaa aacgctggac tggcatgaac 2640
   ttcggtgaaa aaccgcagca gggaggcaaa caatgagagc tactagtatg cctaccgagt 2700
   ttaagctagg tgcgcagtac taggtgttta tgcactccgc gcgggtgtgt gccgtgccgt 2760
   gccgtgccgt gccggtgatg attccgttca agaaaagaga cctccctccc gtgggtgggt 2820
   gatcatctgc ccgtctactt ccagtagtat acgctttcag tttcagcagc acgaggagtc 2880
   cgcttgcgct tctgttcgtg cgctggattc cttgctttaa tttatgcact agtactagtt 2940
   agtaaataaa caacggactg gtcaatgctg ctaatccgta ctgtacgtcc tatatagtag 3000
   taccccacta gagatcagta tatatataca tcttggatac gtgtgcattt gcatgcaaat 3060
   atatatggac agggataact ggcacatgtg tgcccgttag gaaaattacg atcgctctcc 3120
   ttcggtcggt ggcccgctcg ctcgatcgag cgttccttcg gtcggtgtcc cgctcgccct 3180
   tcggtccgtg gcccgctcgc tcgatcgagc gttccttcgg tcggtgtccc gctcgctcga 3240
   tcgagcgttg aggccagttg gctcgatcgc tccgctttga tcgagttgtg aatgctttca 3300
   ccggtttttt tttcttctga tttttatttt cagaacggtc tactaaatag tcactacacc 3360
   caataaccaa ctcggccatc ctaccttgtt atccacaata cgtgaacaac ggaagttgaa 3420
   ttctagtcta cgcggccgcg agctccagct tttgttccct ttagtgaggg ttaattgcgc 3480
   gcttggcgta atcatggtca tagctgtttc ctgtgtgaaa ttgttatccg ctcacaattc 3540
   cacacaacat acgagccgga agcataaagt gtaaagcctg gggtgcctaa tgagtgagct 3600
   aactcacatt aattgcgttg cgctcactgc ccgctttcca gtcgggaaac ctgtcgtgcc 3660
   agctgcatta atgaatcggc caacgcgcgg ggagaggcgg tttgcgtatt gggcgctctt 3720
   ccgcttcctc gctcactgac tcgctgcgct cggtcgttcg gctgcggcga gcggtatcag 3780
   ctcactcaaa ggcggtaata cggttatcca cagaatcagg ggataacgca ggaaagaaca 3840
   tgtgagcaaa aggccagcaa aaggccagga accgtaaaaa ggccgcgttg ctggcgtttt 3900
   tccataggct ccgcccccct gacgagcatc acaaaaatcg acgctcaagt cagaggtggc 3960
   gaaacccgac aggactataa agataccagg cgtttccccc tggaagctcc ctcgtgcgct 4020
   ctcctgttcc gaccctgccg cttaccggat acctgtccgc ctttctccct tcgggaagcg 4080
   tggcgctttc tcatagctca cgctgtaggt atctcagttc ggtgtaggtc gttcgctcca 4140
   agctgggctg tgtgcacgaa ccccccgttc agcccgaccg ctgcgcctta tccggtaact 4200
   atcgtcttga gtccaacccg gtaagacacg acttatcgcc actggcagca gccactggta 4260
   acaggattag cagagcgagg tatgtaggcg gtgctacaga gttcttgaag tggtggccta 4320
   actacggcta cactagaagg acagtatttg gtatctgcgc tctgctgaag ccagttacct 4380
   tcggaaaaag agttggtagc tcttgatccg gcaaacaaac caccgctggt agcggtggtt 4440
   tttttgtttg caagcagcag attacgcgca gaaaaaaagg atctcaagaa gatcctttga 4500
   tcttttctac ggggtctgac gctcagtgga acgaaaactc acgttaaggg attttggtca 4560
   tgagattatc aaaaaggatc ttcacctaga tccttttaaa ttaaaaatga agttttaaat 4620
   caatctaaag tatatatgag taaacttggt ctgacagtta ccaatgctta atcagtgagg 4680
   cacctatctc agcgatctgt ctatttcgtt catccatagt tgcctgactc cccgtcgtgt 4740
   agataactac gatacgggag ggcttaccat ctggccccag tgctgcaatg ataccgcgag 4800
   acccacgctc accggctcca gatttatcag caataaacca gccagccgga agggccgagc 4860
   gcagaagtgg tcctgcaact ttatccgcct ccatccagtc tattaattgt tgccgggaag 4920
   ctagagtaag tagttcgcca gttaatagtt tgcgcaacgt tgttgccatt gctacaggca 4980
   tcgtggtgtc acgctcgtcg tttggtatgg cttcattcag ctccggttcc caacgatcaa 5040
   ggcgagttac atgatccccc atgttgtgca aaaaagcggt tagctccttc ggtcctccga 5100
   tcgttgtcag aagtaagttg gccgcagtgt tatcactcat ggttatggca gcactgcata 5160
   attctcttac tgtcatgcca tccgtaagat gcttttctgt gactggtgag tactcaacca 5220
   agtcattctg agaatagtgt atgcggcgac cgagttgctc ttgcccggcg tcaatacggg 5280
   ataataccgc gccacatagc agaactttaa aagtgctcat cattggaaaa cgttcttcgg 5340
   ggcgaaaact ctcaaggatc ttaccgctgt tgagatccag ttcgatgtaa cccactcgtg 5400
   cacccaactg atcttcagca tcttttactt tcaccagcgt ttctgggtga gcaaaaacag 5460
   gaaggcaaaa tgccgcaaaa aagggaataa gggcgacacg gaaatgttga atactcatac 5520
   tcttcctttt tcaatattat tgaagcattt atcagggtta ttgtctcatg agcggataca 5580
   tatttgaatg tatttagaaa aataaacaaa taggggttcc gcgcacattt ccccgaaaag 5640
   tgccac 5646
<210> 8
   <211> 20
   <212> DNA
   <213> Artificial
<220>
   <223> forward primer for promoter
<400> 8
   cgtgcgtaaa ttaagggcat 20
<210> 9
   <211> 18
   <212> DNA
   <213> Artificial
<220>
   <223> reverse primer for promoter
<400> 9
   cagctccttt gggtcttg 18
<210> 10
   <211> 20
   <212> DNA
   <213> Artificial
<220>
   <223> oligonucleotide
<400> 10
   tcgagcacat ttaaatcaac 20
<210> 11
   <211> 20
   <212> DNA
   <213> Artificial
<220>
   <223> oligonucleotide
<400> 11
   ccgggttgat ttaaatgtgc 20
<210> 12
   <211> 24
   <212> DNA
   <213> Artificial
<220>
   <223> oligonucleotide
<400> 12
   tcgagctcat ttaaatcctc tgca 24
<210> 13
   <211> 16
   <212> DNA
   <213> Artificial
<220>
   <223> oligonucleotide
<400> 13
   gaggatttaa atgagc 16
<210> 14
   <211> 20
   <212> DNA
   <213> Artificial
<220>
   <223> oligonucleotide
<400> 14
   ggatttgtca cgtccaacct 20
<210> 15
   <211> 20
   <212> DNA
   <213> Artificial
<220>
   <223> oligonucleotide
<400> 15
   attggcaatt gtgatagccc 20
<210> 16
   <211> 2953
   <212> DNA
   <213> Hordeum vulgare
<220>
   <221> promoter
   <222> (1)..(2953)
<400> 16
   aaatgtgaat gtggaatgtg caaaagttgc ttcctggact tgttgttctg gtctctcgct 60
   ttctggtgca tgaaattaga aatatacagt tcctccatgt aatattttta gcttcctttt 120
   ggttcaaagc tcttgtaagt tcgattgacg gctgctttga tggaaatcga tgaggtggct 180
   cattttatct aaattcttat gagtagtatt tttgattttc ttaagttggg aactaaaaac 240
   acgttttttt actaagtttt ggaaactggg cacaacggag tcgagatttt aaacattagc 300
   tccaacatac atataaaaaa tatgcataaa tgtagaaaat aaacttatta ccggaagtgc 360
   ctaatgacac acctcgagca tgctggcgga agggagcaga tgagaagctg gaccacctta 420
   agactcaacc aggaccgatg acgacattca tccacgagcc acctggaaga aaacacctga 480
   cttcgtcgta ctgtcacctt tgaactacag ttgcatgtcg gagacgaccc gctgcttaaa 540
   ccaagtagtg tcttgaccat accagcagac atgatgaaag gaggacattg ttgcatgtac 600
   tacaagatat gtgcatgcaa taccgtgagt catttgattt aaggcagggt cgattggatc 660
   gccataccat tccgaacaca ccaccgtaaa tggagagtgt tgattgcaca cattctgtag 720
   caacttaaaa gaatggagag tgttagaact agccgcttgc cgtagatcag atcgccgtcg 780
   tacgccaaca caactataac cgaaactaga acaaaacctg cacgctacga gctaaaagca 840
   agcaagctgg ctggtggatc gattgttggg ctagctagct atggaactga tgtattctgc 900
   cgtccggcta gataaatctc aatcggaact acaccaaagt atagcacgca aacacacgag 960
   ataatcttac aggagaggag atcaagctaa gcagacgact ttgtctgtgt agaaaacgat 1020
   tgctttacat aacttacatc tctcaactcc tctttgacct cgtgtgcatc agttctcacc 1080
   atgaccaagt atacgtatat gatatctaat aaactatgat gatgggtggc gacttttcat 1140
   gaaaccagcg ctccatgcat tgtgctgact tggttgagat ggataaataa ttgtaattac 1200
   tactcagcca tacatcggat gatgcattag cgcttaagca tattaaaaag caaacttgaa 1260
   aagtttatgc aaaatactat tcagaagcca atagatgatt atttagtaat gtgctcagag 1320
   gtttcaacgt gcaagttgaa tgtgcaaacg ttgcttcctt gagtcgttgt tgtggtctgc 1380
   ctttccgggg catgtaataa gtagtttacg gtagttcctg gagtatagcc cacatacttg 1440
   ggctatactt ctctcagtga taaaacttct ctctgtgtac catcacgagt ttactcatag 1500
   atttcttccg taactaatag tgtcaagtta tacacttata tataccttag ttttctctaa 1560
   aagaaaccga tataccttac ttgatgtcct tctcttaatc tagaaaggtg ttaattataa 1620
   atatttccat cttgagtgtt tgtatcctcc gtatatgttt tgattacagg ctcaaagttt 1680
   caattgaagg ttttcttgat gttttttatc ccaatacatc tgggactcca acgggaatat 1740
   ttacaacttg cactgagatt cgttgtattc tctgtctcct agaaatttga ggacactgtt 1800
   tattttgtga agaaatgaat aagacggacg actctagggc ggtagttatg ttttttatgg 1860
   ggcactgaac ctttcggcct ttctgttgta attctgaata tgccttattt ttctcgcaaa 1920
   aaataaagaa tatgccttgt ttttatgcag tcgtctagct ttgaatgtgt tccagtgtcg 1980
   gttttggagc gagtaaataa ctttctgaat gtaagcactt gaacttgagc tatcttgtcg 2040
   tgttggcttg aaactgccca gccctggacg agttctcgtt cccggagaag acgatggtgg 2100
   tgctgggcag ttaggagggc atcccggtgg acatcatcca ggaggcggtg gacgtgtgcg 2160
   tcgagatccc gcagctgggc gtcgtccggt cgctcaacgt ccgtgtcagc gccgccatcg 2220
   ccatctggga ctacacccgc cagcagcggg cccgctcctc ctcctcgcgg taggtagctt 2280
   cccgctcttc cttgttggag tgtaaatcag ccatgcttgt tgttgtaaga tgcatggacc 2340
   tgaacatcca tggcaaaaac tagtcatgaa ccttagtcag gactagattg ttagatttat 2400
   gaactgaatt ggtcatgacg aaaattcttt ggcaagaaaa ttttaactgc tcttgctctc 2460
   catcgtccat taattaatgt cgttgcttct tccttcaagt actcaaccat ttggagagaa 2520
   atttgaccag gcaactgtat atctggttga cgtaatcata tgatacgtat taggtgcgtt 2580
   aatgcatgga tcgacgcgcc atgatttctt ccttgtttga cacaatcaaa actattgctc 2640
   atagaaattg ttactaagag gtctacatca catggagtac cttaatttgc ttctctattt 2700
   attaaaggga aaaacgtgta tcaaagattt gaatatggct gaccacatct caaaagttta 2760
   tgcagactaa tctagtcggc agccaaagac catttctatc cgttgggcgg ttccacttga 2820
   ctatgtccat tactcatgag ttccacctgc tcgccatgcc tatataaaaa catacatccc 2880
   cagcatgttc caaccatcac tcacccaaca aacatctcag gaatatagga gaaaacaaga 2940
   cccaaaggag ctg 2953
<210> 17
   <211> 3116
   <212> DNA
   <213> Hordeum vulgare
<220>
   <221> misc_feature
   <222> (1) .. (3116)
   <223> Promotor + WIR1a-Exon/Intron
<400> 17
   aaatgtgaat gtggaatgtg caaaagttgc ttcctggact tgttgttctg gtctctcgct 60
   ttctggtgca tgaaattaga aatatacagt tcctccatgt aatattttta gcttcctttt 120
   ggttcaaagc tcttgtaagt tcgattgacg gctgctttga tggaaatcga tgaggtggct    180
   cattttatct aaattcttat gagtagtatt tttgattttc ttaagttggg aactaaaaac    240
   acgttttttt actaagtttt ggaaactggg cacaacggag tcgagatttt aaacattagc    300
   tccaacatac atataaaaaa tatgcataaa tgtagaaaat aaacttatta ccggaagtgc    360
   ctaatgacac acctcgagca tgctggcgga agggagcaga tgagaagctg gaccacctta    420
   agactcaacc aggaccgatg acgacattca tccacgagcc acctggaaga aaacacctga    480
   cttcgtcgta ctgtcacctt tgaactacag ttgcatgtcg gagacgaccc gctgcttaaa    540
   ccaagtagtg tcttgaccat accagcagac atgatgaaag gaggacattg ttgcatgtac    600
   tacaagatat gtgcatgcaa taccgtgagt catttgattt aaggcagggt cgattggatc    660
   gccataccat tccgaacaca ccaccgtaaa tggagagtgt tgattgcaca cattctgtag    720
   caacttaaaa gaatggagag tgttagaact agccgcttgc cgtagatcag atcgccgtcg    780
   tacgccaaca caactataac cgaaactaga acaaaacctg cacgctacga gctaaaagca    840
   agcaagctgg ctggtggatc gattgttggg ctagctagct atggaactga tgtattctgc    900
   cgtccggcta gataaatctc aatcggaact acaccaaagt atagcacgca aacacacgag    960
   ataatcttac aggagaggag atcaagctaa gcagacgact ttgtctgtgt agaaaacgat   1020
   tgctttacat aacttacatc tctcaactcc tctttgacct cgtgtgcatc agttctcacc   1080
   atgaccaagt atacgtatat gatatctaat aaactatgat gatgggtggc gacttttcat   1140
   gaaaccagcg ctccatgcat tgtgctgact tggttgagat ggataaataa ttgtaattac   1200
   tactcagcca tacatcggat gatgcattag cgcttaagca tattaaaaag caaacttgaa   1260
   aagtttatgc aaaatactat tcagaagcca atagatgatt atttagtaat gtgctcagag   1320
   gtttcaacgt gcaagttgaa tgtgcaaacg ttgcttcctt gagtcgttgt tgtggtctgc   1380
   ctttccgggg catgtaataa gtagtttacg gtagttcctg gagtatagcc cacatacttg   1440
   ggctatactt ctctcagtga taaaacttct ctctgtgtac catcacgagt ttactcatag   1500
   atttcttccg taactaatag tgtcaagtta tacacttata tataccttag ttttctctaa   1560
   aagaaaccga tataccttac ttgatgtcct tctcttaatc tagaaaggtg ttaattataa 1620
   atatttccat cttgagtgtt tgtatcctcc gtatatgttt tgattacagg ctcaaagttt 1680
   caattgaagg ttttcttgat gttttttatc ccaatacatc tgggactcca acgggaatat 1740
   ttacaacttg cactgagatt cgttgtattc tctgtctcct agaaatttga ggacactgtt 1800
   tattttgtga agaaatgaat aagacggacg actctagggc ggtagttatg ttttttatgg 1860
   ggcactgaac ctttcggcct ttctgttgta attctgaata tgccttattt ttctcgcaaa 1920
   aaataaagaa tatgccttgt ttttatgcag tcgtctagct ttgaatgtgt tccagtgtcg 1980
   gttttggagc gagtaaataa ctttctgaat gtaagcactt gaacttgagc tatcttgtcg 2040
   tgttggcttg aaactgccca gccctggacg agttctcgtt cccggagaag acgatggtgg 2100
   tgctgggcag ttaggagggc atcccggtgg acatcatcca ggaggcggtg gacgtgtgcg 2160
   tcgagatccc gcagctgggc gtcgtccggt cgctcaacgt ccgtgtcagc gccgccatcg 2220
   ccatctggga ctacacccgc cagcagcggg cccgctcctc ctcctcgcgg taggtagctt 2280
   cccgctcttc cttgttggag tgtaaatcag ccatgcttgt tgttgtaaga tgcatggacc 2340
   tgaacatcca tggcaaaaac tagtcatgaa ccttagtcag gactagattg ttagatttat 2400
   gaactgaatt ggtcatgacg aaaattcttt ggcaagaaaa ttttaactgc tcttgctctc 2460
   catcgtccat taattaatgt cgttgcttct tccttcaagt actcaaccat ttggagagaa 2520
   atttgaccag gcaactgtat atctggttga cgtaatcata tgatacgtat taggtgcgtt 2580
   aatgcatgga tcgacgcgcc atgatttctt ccttgtttga cacaatcaaa actattgctc 2640
   atagaaattg ttactaagag gtctacatca catggagtac cttaatttgc ttctctattt 2700
   attaaaggga aaaacgtgta tcaaagattt gaatatggct gaccacatct caaaagttta 2760
   tgcagactaa tctagtcggc agccaaagac catttctatc cgttgggcgg ttccacttga 2820
   ctatgtccat tactcatgag ttccacctgc tcgccatgcc tatataaaaa catacatccc 2880
   cagcatgttc caaccatcac tcacccaaca aacatctcag gaatatagga gaaaacaaga 2940
   cccaaaggag ctgatcgaat tcctgcaggg agccacggcc gtccacgacg ccgccgcctc 3000
   aggtcagtcg tcggacggtg tccgttcatt tcctccccat ttttgtaatt gattaacttg 3060
   ttatacatgc tgacctcgac ctgctgaata acgtccgtcc atggtttccc gtccag 3116
<210> 18
   <211> 16420
   <212> DNA
   <213> Artificial
<220>
   <223> Plasmid p6UGLP4GUS
<400> 18
   tctagtagtc tgcagtgcag cgtgacccgg tcgtgcccct ctctagagat aatgagcatt      60
   gcatgtctaa gttataaaaa attaccacat attttttttg tcacacttgt ttgaagtgca      120
   gtttatctat ctttatacat atatttaaac tttactctac gaataatata atctatagta      180
   ctacaataat atcagtgttt tagagaatca tataaatgaa cagttagaca tggtctaaag      240
   gacaattgag tattttgaca acaggactct acagttttat ctttttagtg tgcatgtgtt      300
   ctcctttttt tttgcaaata gcttcaccta tataatactt catccatttt attagtacat      360
   ccatttaggg tttagggtta atggttttta tagactaatt tttttagtac atctatttta      420
   ttctatttta gcctctaaat taagaaaact aaaactctat tttagttttt ttatttaata      480
   atttagatat aaaatagaat aaaataaagt gactaaaaat taaacaaata ccctttaaga      540
   aattaaaaaa actaaggaaa catttttctt gtttcgagta gataatgcca gcctgttaaa      600
   cgccgtcgat cgacgagtct aacggacacc aaccagcgaa ccagcagcgt cgcgtcgggc      660
   caagcgaagc agacggcacg gcatctctgt cgctgcctct ggacccctct cgagagttcc      720
   gctccaccgt tggacttgct ccgctgtcgg catccagaaa ttgcgtggcg gagcggcaga      780
   cgtgagccgg cacggcaggc ggcctcctcc tcctctcacg gcaccggcag ctacggggga      840
   ttcctttccc accgctcctt cgctttccct tcctcgcccg ccgtaataaa tagacacccc      900
   ctccacaccc tctttcccca acctcgtgtt gttcggagcg cacacacaca caaccagatc      960
   tcccccaaat ccacccgtcg gcacctccgc ttcaaggtac gccgctcgtc ctcccccccc     1020
   ccccctctct accttctcta gatcggcgtt ccggtccatg gttagggccc ggtagttcta 1080
   cttctgttca tgtttgtgtt agatccgtgt ttgtgttaga tccgtgctgc tagcgttcgt 1140
   acacggatgc gacctgtacg tcagacacgt tctgattgct aacttgccag tgtttctctt 1200
   tggggaatcc tgggatggct ctagccgttc cgcagacggg atcgatctag gataggtata 1260
   catgttgatg tgggttttac tgatgcatat acatgatggc atatgcagca tctattcata 1320
   tgctctaacc ttgagtacct atctattata ataaacaagt atgttttata attattttga 1380
   tcttgatata cttggatgat ggcatatgca gcagctatat gtggattttt ttagccctgc 1440
   cttcatacgc tatttatttg cttggtactg tttcttttgt cgatgctcac cctgttgttt 1500
   ggtgttactt ctgcaggtcg agggccccgg ggggcaataa gatatgaaaa agcctgaact 1560
   caccgcgacg tctgtcgaga agtttctgat cgaaaagttc gacagcgtct ccgacctgat 1620
   gcagctctcg gagggcgaag aatctcgtgc tttcagcttc gatgtaggag ggcgtggata 1680
   tgtcctgcgg gtaaatagct gcgccgatgg tttctacaaa gatcgttatg tttatcggca 1740
   ctttgcatcg gccgcgctcc cgattccgga agtgcttgac attggggaat tcagcgagag 1800
   cctgacctat tgcatctccc gccgtgcaca gggtgtcacg ttgcaagacc tgcctgaaac 1860
   cgaactgccc gctgttctgc agccggtcgc ggaggccatg gatgcgatcg ctgcggccga 1920
   tcttagccag acgagcgggt tcggcccatt cggaccgcaa ggaatcggtc aatacactac 1980
   atggcgtgat ttcatatgcg cgattgctga tccccatgtg tatcactggc aaactgtgat 2040
   ggacgacacc gtcagtgcgt ccgtcgcgca ggctctcgat gagctgatgc tttgggccga 2100
   ggactgcccc gaagtccggc acctcgtgca cgcggatttc ggctccaaca atgtcctgac 2160
   ggacaatggc cgcataacag cggtcattga ctggagcgag gcgatgttcg gggattccca 2220
   atacgaggtc gccaacatct tcttctggag gccgtggttg gcttgtatgg agcagcagac 2280
   gcgctacttc gagcggaggc atccggagct tgcaggatcg ccgcggctcc gggcgtatat 2340
   gctccgcatt ggtcttgacc aactctatca gagcttggtt gacggcaatt tcgatgatgc 2400
   agcttgggcg cagggtcgat gcgacgcaat cgtccgatcc ggagccggga ctgtcgggcg 2460
   tacacaaatc gcccgcagaa gcgcggccgt ctggaccgat ggctgtgtag aagtactcgc 2520
   cgatagtgga aaccgacgcc ccagcactcg tccgagggca aaggaataga gtagatgccg 2580
   accggagtcc gcaaaaatca ccagtctctc tctacaaatc tatctctctc tatttttctc 2640
   cagaataatg tgtgagtagt tcccagataa gggaattagg gttcttatag ggtttcgctc 2700
   atgtgttgag catataagaa acccttagta tgtatttgta tttgtaaaat acttctatca 2760
   ataaaatttc taattcctaa aaccaaaatc cagtgacctg caggcatgca agctgatcca 2820
   ctagaggcca tggcggccgc gtagactaga attcaacttc cgttgttcac gtattgtgga 2880
   taacaaggta ggatggccga gttggttatt gggtgtagtg actatttagt agaccgttct 2940
   gaaaataaaa atcagaagaa aaaaaaaccg gtgaaagcat tcacaactcg atcaaagcgg 3000
   agcgatcgag ccaactggcc tcaacgctcg atcgagcgag cgggacaccg accgaaggaa 3060
   cgctcgatcg agcgagcggg ccacggaccg aagggcgagc gggacaccga ccgaaggaac 3120
   gctcgatcga gcgagcgggc caccgaccga aggagagcga tcgtaatttt cctaacgggc 3180
   acacatgtgc cagttatccc tgtccatata tatttgcatg caaatgcaca cgtatccaag 3240
   atgtatatat atactgatct ctagtggggt actactatat aggacgtaca gtacggatta 3300
   gcagcattga ccagtccgtt gtttatttac taactagtac tagtgcataa attaaagcaa 3360
   ggaatccagc gcacgaacag aagcgcaagc ggactcctcg tgctgctgaa actgaaagcg 3420
   tatactactg gaagtagacg ggcagatgat cacccaccca cgggagggag gtctcttttc 3480
   ttgaacggaa tcatcaccgg cacggcacgg cacggcacgg cacacacccg cgcggagtgc 3540
   ataaacacct agtactgcgc acctagctta aactcggtag gcatactagt agctctcatt 3600
   gtttgcctcc ctgctgcggt ttttcaccga agttcatgcc agtccagcgt ttttgcagca 3660
   gaaaagccgc cgacttcggt ttgcggtcgc gagtgaagat ccctttcttg ttaccgccaa 3720
   cgcgcaatat gccttgcgag gtcgcaaaat cggcgaaatt ccatacctgt tcaccgacga 3780
   cggcgctgac gcgatcaaag acgcggtgat acatatccag ccatgcacac tgatactctt 3840
   cactccacat gtcggtgtac attgagtgca gcccggctaa cgtatccacg ccgtattcgg 3900
   tgatgataat cggctgatgc agtttctcct gccaggccag aagttctttt tccagtacct 3960
   tctctgccgt ttccaaatcg ccgctttgga cataccatcc gtaataacgg ttcaggcaca 4020
   gcacatcaaa gagatcgctg atggtatcgg tgtgagcgtc gcagaacatt acattgacgc 4080
   aggtgatcgg acgcgtcggg tcgagtttac gcgttgcttc cgccagtggc gcgaaatatt 4140
   cccgtgcacc ttgcggacgg gtatccggtt cgttggcaat actccacatc accacgcttg 4200
   ggtggttttt gtcacgcgct atcagctctt taatcgcctg taagtgcgct tgctgagttt 4260
   ccccgttgac tgcctcttcg ctgtacagtt ctttcggctt gttgcccgct tcgaaaccaa 4320
   tgcctaaaga gaggttaaag ccgacagcag cagtttcatc aatcaccacg atgccatgtt 4380
   catctgccca gtcgagcatc tcttcagcgt aagggtaatg cgaggtacgg taggagttgg 4440
   ccccaatcca gtccattaat gcgtggtcgt gcaccatcag cacgttatcg aatcctttgc 4500
   cacgcaagtc cgcatcttca tgacgaccaa agccagtaaa gtagaacggt ttgtggttaa 4560
   tcaggaactg ttcgcccttc actgccactg accggatgcc gacgcgaagc gggtagatat 4620
   cacactctgt ctggcttttg gctgtgacgc acagttcata gagataacct tcacccggtt 4680
   gccagaggtg cggattcacc acttgcaaag tcccgctagt gccttgtcca gttgcaacca 4740
   cctgttgatc cgcatcacgc agttcaacgc tgacatcacc attggccacc acctgccagt 4800
   caacagacgc gtggttacag tcttgcgcga catgcgtcac cacggtgata tcgtccaccc 4860
   aggtgttcgg cgtggtgtag agcattacgc tgcgatggat tccggcatag ttaaagaaat 4920
   catggaagta agactgcttt ttcttgccgt tttcgtcggt aatcaccatt cccggcggga 4980
   tagtctgcca gttcagttcg ttgttcacac aaacggtgat acgtacactt ttcccggcaa 5040
   taacatacgg cgtgacatcg gcttcaaatg gcgtatagcc gccctgatgc tccatcactt 5100
   cctgattatt gacccacact ttgccgtaat gagtgaccgc atcgaaacgc agcacgatac 5160
   gctggcctgc ccaacctttc ggtataaaga cttcgcgctg ataccagacg ttgcccgcat 5220
   aattacgaat atctgcatcg gcgaactgat cgttaaaact gcctggcaca gcaattgccc 5280
   ggctttcttg taacgcgctt tcccaccaac gctgatcaat tccacagttt tcgcgatcca 5340
   gactgaatgc ccacaggccg tcgagttttt tgatttcacg ggttggggtt tctacaggac   5400
   gtaacataag ggactgacca cccgggctgc aggaattcga tcagctcctt tgggtcttgt   5460
   tttctcctat attcctgaga tgtttgttgg gtgagtgatg gttggaacat gctggggatg   5520
   tatgttttta tataggcatg gcgagcaggt ggaactcatg agtaatggac atagtcaagt   5580
   ggaaccgccc aacggataga aatggtcttt ggctgccgac tagattagtc tgcataaact   5640
   tttgagatgt ggtcagccat attcaaatct ttgatacacg tttttccctt taataaatag   5700
   agaagcaaat taaggtactc catgtgatgt agacctctta gtaacaattt ctatgagcaa   5760
   tagttttgat tgtgtcaaac aaggaagaaa tcatggcgcg tcgatccatg cattaacgca   5820
   cctaatacgt atcatatgat tacgtcaacc agatatacag ttgcctggtc aaatttctct   5880
   ccaaatggtt gagtacttga aggaagaagc aacgacatta attaatggac gatggagagc   5940
   aagagcagtt aaaatttcct tgccaaagaa ttttcgtcat gaccaattca gttcataaat   6000
   ctaacaatct agtcctgact aaggttcatg actagttttt gccatggatg ttcaggtcca   6060
   tgcatcttac aacaacaagc atggctgatt tacactccaa caaggaagag cgggaagcta   6120
   cctaccgcga ggaggaggag cgggcccgct gctggcgggt gtagtcccag atggcgatgg   6180
   cggcgctgac acggacgttg agcgaccgga cgacgcccag ctgcgggatc tcgacgcaca   6240
   cgtccaccgc ctcctggatg atgtccaccg ggatgccctc ctaactgccc agcaccacca   6300
   tcgtcttctc cgggaacgag aactcgtcca gggctgggca gtttcaagcc aacacgacaa   6360
   gatagctcaa gttcaagtgc ttacattcag aaagttattt actcgctcca aaaccgacac   6420
   tggaacacat tcaaagctag acgactgcat aaaaacaagg catattcttt attttttgcg   6480
   agaaaaataa ggcatattca gaattacaac agaaaggccg aaaggttcag tgccccataa   6540
   aaaacataac taccgcccta gagtcgtccg tcttattcat ttcttcacaa aataaacagt   6600
   gtcctcaaat ttctaggaga cagagaatac aacgaatctc agtgcaagtt gtaaatattc   6660
   ccgttggagt cccagatgta ttgggataaa aaacatcaag aaaaccttca attgaaactt   6720
   tgagcctgta atcaaaacat atacggagga tacaaacact caagatggaa atatttataa   6780
   ttaacacctt tctagattaa gagaaggaca tcaagtaagg tatatcggtt tcttttagag 6840
   aaaactaagg tatatataag tgtataactt gacactatta gttacggaag aaatctatga 6900
   gtaaactcgt gatggtacac agagagaagt tttatcactg agagaagtat agcccaagta 6960
   tgtgggctat actccaggaa ctaccgtaaa ctacttatta catgccccgg aaaggcagac 7020
   cacaacaacg actcaaggaa gcaacgtttg cacattcaac ttgcacgttg aaacctctga 7080
   gcacattact aaataatcat ctattggctt ctgaatagta ttttgcataa acttttcaag 7140
   tttgcttttt aatatgctta agcgctaatg catcatccga tgtatggctg agtagtaatt 7200
   acaattattt atccatctca accaagtcag cacaatgcat ggagcgctgg tttcatgaaa 7260
   agtcgccacc catcatcata gtttattaga tatcatatac gtatacttgg tcatggtgag 7320
   aactgatgca cacgaggtca aagaggagtt gagagatgta agttatgtaa agcaatcgtt 7380
   ttctacacag acaaagtcgt ctgcttagct tgatctcctc tcctgtaaga ttatctcgtg 7440
   tgtttgcgtg ctatactttg gtgtagttcc gattgagatt tatctagccg gacggcagaa 7500
   tacatcagtt ccatagctag ctagcccaac aatcgatcca ccagccagct tgcttgcttt 7560
   tagctcgtag cgtgcaggtt ttgttctagt ttcggttata gttgtgttgg cgtacgacgg 7620
   cgatctgatc tacggcaagc ggctagttct aacactctcc attcttttaa gttgctacag 7680
   aatgtgtgca atcaacactc tccatttacg gtggtgtgtt cggaatggta tggcgatcca 7740
   atcgaccctg ccttaaatca aatgactcac ggtattgcat gcacatatct tgtagtacat 7800
   gcaacaatgt cctcctttca tcatgtctgc tggtatggtc aagacactac ttggtttaag 7860
   cagcgggtcg tctccgacat gcaactgtag ttcaaaggtg acagtacgac gaagtcaggt 7920
   gttttcttcc aggtggctcg tggatgaatg tcgtcatcgg tcctggttga gtcttaaggt 7980
   ggtccagctt ctcatctgct cccttccgcc agcatgctcg aggtgtgtca ttaggcactt 8040
   ccggtaataa gtttattttc tacatttatg catatttttt atatgtatgt tggagctaat 8100
   gtttaaaatc tcgactccgt tgtgcccagt ttccaaaact tagtaaaaaa acgtgttttt 8160
   agttcccaac ttaagaaaat caaaaatact actcataaga atttagataa aatgagccac 8220
   ctcatcgatt tccatcaaag cagccgtcaa tcgaacttac aagagctttg aaccaaaagg 8280
   aagctaaaaa tattacatgg aggaactgta tatttctaat ttcatgcacc agaaagcgag 8340
   agaccagaac aacaagtcca ggaagcaact tttgcacatt ccacattcac atttaaatcc 8400
   tgagcacaat cgggctccac ccatcgcata cttcacatgt attgccagat aataatacgt 8460
   gttcaacacc agcgtcaaac aaatactgca ttaccaaata atcttgtatt gtttcttttt 8520
   tttaacctaa tcatatatta tttcagaata aggggaaaag aacccaaaat attgatcaat 8580
   tatgagaaat acgacacagt agaaccaaaa aaaagcatgt gagatcatat cagtgtgata 8640
   aaaaacaata tttttgatta tttaaaaggt agaaatacat tcactgatta caggcacaca 8700
   attacatatg ctccttttat tttataaatt aggatgcata tctaggaact caatcatgcc 8760
   cttaatttac gcacgatcaa gcttgaattc ctgcagcccg ggggatccac tagtaaggcc 8820
   ttaagggcca gatcttgggc ccggtacccg atcagattgt cgtttcccgc cttcggttta 8880
   aactatcagt gtttgacagg atatattggc gggtaaacct aagagaaaag agcgtttatt 8940
   agaataatcg gatatttaaa agggcgtgaa aaggtttatc cgttcgtcca tttgtatgtg 9000
   catgccaacc acagggttcc cctcgggagt gcttggcatt ccgtgcgata atgacttctg 9060
   ttcaaccacc caaacgtcgg aaagcctgac gacggagcag cattccaaaa agatcccttg 9120
   gctcgtctgg gtcggctaga aggtcgagtg ggctgctgtg gcttgatccc tcaacgcggt 9180
   cgcggacgta gcgcagcgcc gaaaaatcct cgatcgcaaa tccgacgctg tcgaaaagcg 9240
   tgatctgctt gtcgctcttt cggccgacgt cctggccagt catcacgcgc caaagttccg 9300
   tcacaggatg atctggcgcg agttgctgga tctcgccttc aatccgggtc tgtggcggga 9360
   actccacgaa aatatccgaa cgcagcaaga tatcgcggtg catctcggtc ttgcctgggc 9420
   agtcgccgcc gacgccgttg atgtggacgc cgaaaaggat ctaggtgaag atcctttttg 9480
   ataatctcat gaccaaaatc ccttaacgtg agttttcgtt ccactgagcg tcagaccccg 9540
   tagaaaagat caaaggatct tcttgagatc ctttttttct gcgcgtaatc tgctgcttgc 9600
   aaacaaaaaa accaccgcta ccagcggtgg tttgtttgcc ggatcaagag ctaccaactc 9660
   tttttccgaa ggtaactggc ttcagcagag cgcagatacc aaatactgtt cttctagtgt 9720
   agccgtagtt aggccaccac ttcaagaact ctgtagcacc gcctacatac ctcgctctgc 9780
   taatcctgtt accagtggct gctgccagtg gcgataagtc gtgtcttacc gggttggact 9840
   caagacgata gttaccggat aaggcgcagc ggtcgggctg aacggggggt tcgtgcacac 9900
   agcccagctt ggagcgaacg acctacaccg aactgagata cctacagcgt gagctatgag 9960
   aaagcgccac gcttcccgaa gggagaaagg cggacaggta tccggtaagc ggcagggtcg 10020
   gaacaggaga gcgcacgagg gagcttccag ggggaaacgc ctggtatctt tatagtcctg 10080
   tcgggtttcg ccacctctga cttgagcgtc gatttttgtg atgctcgtca ggggggcgga 10140
   gcctatggaa aaacgccagc aacgcggcct ttttacggtt cctggccttt tgctggcctt 10200
   ttgctcacat gttctttcct gcgttatccc ctgattctgt ggataaccga ttaccgcctt 10260
   tgagtgagct gataccgctc gccgcagccg aacgaccgag cgcagcgagt cagtgagcga 10320
   ggaagcggaa gagcgcctga tgcggtattt tctccttacg catctgtgcg gtatttcaca 10380
   ccgcatatgg tgcactctca gtacaatctg ctctgatgcc gcatagttaa gccagtatac 10440
   actccgctat cgctacgtga ctgggtcatg gctgcgcccc gacacccgcc aacacccgct 10500
   gacgcgccct gacgggcttg tctgctcccg gcatccgctt acagacaagc tgtgaccgtc 10560
   tccgggagct gcatgtgtca gaggttttca ccgtcatcac cgaaacgcgc gaggcagggg 10620
   tacgtcgagg tcgatccaac ccctccgctg ctatagtgca gtcggcttct gacgttcagt 10680
   gcagccgtct tctgaaaacg acatgtcgca caagtcctaa gttacgcgac aggctgccgc 10740
   cctgcccttt tcctggcgtt ttcttgtcgc gtgttttagt cgcataaagt agaatacttg 10800
   cgactagaac cggagacatt acgccatgaa caagagcgcc gccgctggcc tgctgggcta 10860
   tgcccgcgtc agcaccgacg accaggactt gaccaaccaa cgggccgaac tgcacgcggc 10920
   cggctgcacc aagctgtttt ccgagaagat caccggcacc aggcgcgacc gcccggagct 10980
   ggccaggatg cttgaccacc tacgccctgg cgacgttgtg acagtgacca ggctagaccg 11040
   cctggcccgc agcacccgcg acctactgga cattgccgag cgcatccagg aggccggcgc 11100
   gggcctgcgt agcctggcag agccgtgggc cgacaccacc acgccggccg gccgcatggt 11160
   gttgaccgtg ttcgccggca ttgccgagtt cgagcgttcc ctaatcatcg accgcacccg 11220
   gagcgggcgc gaggccgcca aggcgcgagg cgtgaagttt ggcccccgcc ctaccctcac 11280
   cccggcacag atcgcgcacg cccgcgagct gatcgaccag gaaggccgca ccgtgaaaga 11340
   ggcggctgca ctgcttggcg tgcatcgctc gaccctgtac cgcgcacttg agcgcagcga 11400
   ggaagtgacg cccaccgagg ccaggcggcg cggtgccttc cgtgaggacg cattgaccga 11460
   ggccgacgcc ctggcggccg ccgagaatga acgccaagag gaacaagcat gaaaccgcac 11520
   caggacggcc aggacgaacc gtttttcatt accgaagaga tcgaggcgga gatgatcgcg 11580
   gccgggtacg tgttcgagcc gcccgcgcac gtctcaaccg tgcggctgca tgaaatcctg 11640
   gccggtttgt ctgatgccaa gctcgcggcc tggccggcga gcttggccgc tgaagaaacc 11700
   gagcgccgcc gtctaaaaag gtgatgtgta tttgagtaaa acagcttgcg tcatgcggtc 11760
   gctgcgtata tgatgcgatg agtaaataaa caaatacgca aggggaacgc atgaaggtta 11820
   tcgctgtact taaccagaaa ggcgggtcag gcaagacgac catcgcaacc catctagccc 11880
   gcgccctgca actcgccggg gccgatgttc tgttagtcga ttccgatccc cagggcagtg 11940
   cccgcgattg ggcggccgtg cgggaagatc aaccgctaac cgttgtcggc atcgaccgcc 12000
   cgacgattga ccgcgacgtg aaggccatcg gccggcgcga cttcgtagtg atcgacggag 12060
   cgccccaggc ggcggacttg gctgtgtccg cgatcaaggc agccgacttc gtgctgattc 12120
   cggtgcagcc aagcccttac gacatatggg ccaccgccga cctggtggag ctggttaagc 12180
   agcgcattga ggtcacggat ggaaggctac aagcggcctt tgtcgtgtcg cgggcgatca 12240
   aaggcacgcg catcggcggt gaggttgccg aggcgctggc cgggtacgag ctgcccattc 12300
   ttgagtcccg tatcacgcag cgcgtgagct acccaggcac tgccgccgcc ggcacaaccg 12360
   ttcttgaatc agaacccgag ggcgacgctg cccgcgaggt ccaggcgctg gccgctgaaa 12420
   ttaaatcaaa actcatttga gttaatgagg taaagagaaa atgagcaaaa gcacaaacac 12480
   gctaagtgcc ggccgtccga gcgcacgcag cagcaaggct gcaacgttgg ccagcctggc 12540
   agacacgcca gccatgaagc gggtcaactt tcagttgccg gcggaggatc acaccaagct 12600
   gaagatgtac gcggtacgcc aaggcaagac cattaccgag ctgctatctg aatacatcgc 12660
   gcagctacca gagtaaatga gcaaatgaat aaatgagtag atgaatttta gcggctaaag 12720
   gaggcggcat ggaaaatcaa gaacaaccag gcaccgacgc cgtggaatgc cccatgtgtg 12780
   gaggaacggg cggttggcca ggcgtaagcg gctgggttgt ctgccggccc tgcaatggca 12840
   ctggaacccc caagcccgag gaatcggcgt gagcggtcgc aaaccatccg gcccggtaca 12900
   aatcggcgcg gcgctgggtg atgacctggt ggagaagttg aaggcggcgc aggccgccca 12960
   gcggcaacgc atcgaggcag aagcacgccc cggtgaatcg tggcaagcgg ccgctgatcg 13020
   aatccgcaaa gaatcccggc aaccgccggc agccggtgcg ccgtcgatta ggaagccgcc 13080
   caagggcgac gagcaaccag attttttcgt tccgatgctc tatgacgtgg gcacccgcga 13140
   tagtcgcagc atcatggacg tggccgtttt ccgtctgtcg aagcgtgacc gacgagctgg 13200
   cgaggtgatc cgctacgagc ttccagacgg gcacgtagag gtttccgcag ggccggccgg 13260
   catggcgagt gtgtgggatt acgacctggt actgatggcg gtttcccatc taaccgaatc 13320
   catgaaccga taccgggaag ggaagggaga caagcccggc cgcgtgttcc gtccacacgt 13380
   tgcggacgta ctcaagttct gccggcgagc cgatggcgga aagcagaaag acgacctggt 13440
   agaaacctgc attcggttaa acaccacgca cgttgccatg cagcgtacga agaaggccaa 13500
   gaacggccgc ctggtgacgg tatccgaggg tgaagccttg attagccgct acaagatcgt 13560
   aaagagcgaa accgggcggc cggagtacat cgagatcgag ctagctgatt ggatgtaccg 13620
   cgagatcaca gaaggcaaga acccggacgt gctgacggtt caccccgatt actttttgat 13680
   cgatcccggc atcggccgtt ttctctaccg cctggcacgc cgcgccgcag gcaaggcaga 13740
   agccagatgg ttgttcaaga cgatctacga acgcagtggc agcgccggag agttcaagaa 13800
   gttctgtttc accgtgcgca agctgatcgg gtcaaatgac ctgccggagt acgatttgaa 13860
   ggaggaggcg gggcaggctg gcccgatcct agtcatgcgc taccgcaacc tgatcgaggg 13920
   cgaagcatcc gccggttcct aatgtacgga gcagatgcta gggcaaattg ccctagcagg 13980
   ggaaaaaggt cgaaaaggtc tctttcctgt ggatagcacg tacattggga acccaaagcc 14040
   gtacattggg aaccggaacc cgtacattgg gaacccaaag ccgtacattg ggaaccggtc 14100
   acacatgtaa gtgactgata taaaagagaa aaaaggcgat ttttccgcct aaaactcttt 14160
   aaaacttatt aaaactctta aaacccgcct ggcctgtgca taactgtctg gccagcgcac 14220
   agccgaagag ctgcaaaaag cgcctaccct tcggtcgctg cgctccctac gccccgccgc 14280
   ttcgcgtcgg cctatcgcgg ccgctggccg ctcaaaaatg gctggcctac ggccaggcaa 14340
   tctaccaggg cgcggacaag ccgcgccgtc gccactcgac cgccggcgcc cacatcaagg 14400
   caccggtggg tatgcctgac gatgcgtgga gaccgaaacc ttgcgctcgt tcgccagcca 14460
   ggacagaaat gcctcgactt cgctgctgcc caaggttgcc gggtgacgca caccgtggaa 14520
   acggatgaag gcacgaaccc agtggacata agcctgttcg gttcgtaagc tgtaatgcaa 14580
   gtagcgtatg cgctcacgca actggtccag aaccttgacc gaacgcagcg gtggtaacgg 14640
   cgcagtggcg gttttcatgg cttgttatga ctgttttttt ggggtacagt ctatgcctcg 14700
   ggcatccaag cagcaagcgc gttacgccgt gggtcgatgt ttgatgttat ggagcagcaa 14760
   cgatgttacg cagcagggca gtcgccctaa aacaaagtta aacatcatga gggaagcggt 14820
   gatcgccgaa gtatcgactc aactatcaga ggtagttggc gtcatcgagc gccatctcga 14880
   accgacgttg ctggccgtac atttgtacgg ctccgcagtg gatggcggcc tgaagccaca 14940
   cagtgatatt gatttgctgg ttacggtgac cgtaaggctt gatgaaacaa cgcggcgagc 15000
   tttgatcaac gaccttttgg aaacttcggc ttcccctgga gagagcgaga ttctccgcgc 15060
   tgtagaagtc accattgttg tgcacgacga catcattccg tggcgttatc cagctaagcg 15120
   cgaactgcaa tttggagaat ggcagcgcaa tgacattctt gcaggtatct tcgagccagc 15180
   cacgatcgac attgatctgg ctatcttgct gacaaaagca agagaacata gcgttgcctt 15240
   ggtaggtcca gcggcggagg aactctttga tccggttcct gaacaggatc tatttgaggc 15300
   gctaaatgaa accttaacgc tatggaactc gccgcccgac tgggctggcg atgagcgaaa 15360
   tgtagtgctt acgttgtccc gcatttggta cagcgcagta accggcaaaa tcgcgccgaa 15420
   ggatgtcgct gccgactggg caatggagcg cctgccggcc cagtatcagc ccgtcatact  15480
   tgaagctaga caggcttatc ttggacaaga agaagatcgc ttggcctcgc gcgcagatca  15540
   gttggaagaa tttgtccact acgtgaaagg cgagatcacc aaggtagtcg gcaaataatg  15600
   tctaacaatt cgttcaagcc gacgccgctt cgcggcgcgg cttaactcaa gcgttagatg  15660
   cactaagcac ataattgctc acagccaaac tatcaggtca agtctgcttt tattattttt  15720
   aagcgtgcat aataagccct acacaaattg ggagatatat catgaaaggc tggctttttc  15780
   ttgttatcgc aatagttggc gaagtaatcg caacatagct tgcttggtcg ttccgcgtga  15840
   acgtcggctc gattgtacct gcgttcaaat actttgcgat cgtgttgcgc gcctgcccgg  15900
   tgcgtcggct gatctcacgg atcgactgct tctctcgcaa cgccatccga cggatgatgt  15960
   ttaaaagtcc catgtggatc actccgttgc cccgtcgctc accgtgttgg ggggaaggtg  16020
   cacatggctc agttctcaat ggaaattatc tgcctaaccg gctcagttct gcgtagaaac  16080
   caacatgcaa gctccaccgg gtgcaaagcg gcagcggcgg caggatatat tcaattgtaa  16140
   atggcttcat gtccgggaaa tctacatgga tcagcaatga gtatgatggt caatatggag  16200
   aaaaagaaag agtaattacc aatttttttt caattcaaaa atgtagatgt ccgcagcgtt  16260
   attataaaat gaaagtacat tttgataaaa cgacaaatta cgatccgtcg tatttatagg  16320
   cgaaagcaat aaacaaatta ttctaattcg gaaatcttta tttcgacgtg tctacattca  16380
   cgtccaaatg ggggcttaga tgagaaactt cacgatcggc                         16420
<210> 19
   <211> 16572
   <212> DNA
   <213> Artificial
<220>
   <223> Plasmid p6UGLP4IntronGUS
<400> 19
   tctagtagtc tgcagtgcag cgtgacccgg tcgtgcccct ctctagagat aatgagcatt      60
   gcatgtctaa gttataaaaa attaccacat attttttttg tcacacttgt ttgaagtgca    120
   gtttatctat ctttatacat atatttaaac tttactctac gaataatata atctatagta   180
   ctacaataat atcagtgttt tagagaatca tataaatgaa cagttagaca tggtctaaag   240
   gacaattgag tattttgaca acaggactct acagttttat ctttttagtg tgcatgtgtt   300
   ctcctttttt tttgcaaata gcttcaccta tataatactt catccatttt attagtacat   360
   ccatttaggg tttagggtta atggttttta tagactaatt tttttagtac atctatttta   420
   ttctatttta gcctctaaat taagaaaact aaaactctat tttagttttt ttatttaata   480
   atttagatat aaaatagaat aaaataaagt gactaaaaat taaacaaata ccctttaaga   540
   aattaaaaaa actaaggaaa catttttctt gtttcgagta gataatgcca gcctgttaaa   600
   cgccgtcgat cgacgagtct aacggacacc aaccagcgaa ccagcagcgt cgcgtcgggc   660
   caagcgaagc agacggcacg gcatctctgt cgctgcctct ggacccctct cgagagttcc   720
   gctccaccgt tggacttgct ccgctgtcgg catccagaaa ttgcgtggcg gagcggcaga   780
   cgtgagccgg cacggcaggc ggcctcctcc tcctctcacg gcaccggcag ctacggggga   840
   ttcctttccc accgctcctt cgctttccct tcctcgcccg ccgtaataaa tagacacccc   900
   ctccacaccc tctttcccca acctcgtgtt gttcggagcg cacacacaca caaccagatc   960
   tcccccaaat ccacccgtcg gcacctccgc ttcaaggtac gccgctcgtc ctcccccccc  1020
   ccccctctct accttctcta gatcggcgtt ccggtccatg gttagggccc ggtagttcta  1080
   cttctgttca tgtttgtgtt agatccgtgt ttgtgttaga tccgtgctgc tagcgttcgt  1140
   acacggatgc gacctgtacg tcagacacgt tctgattgct aacttgccag tgtttctctt  1200
   tggggaatcc tgggatggct ctagccgttc cgcagacggg atcgatctag gataggtata  1260
   catgttgatg tgggttttac tgatgcatat acatgatggc atatgcagca tctattcata  1320
   tgctctaacc ttgagtacct atctattata ataaacaagt atgttttata attattttga  1380
   tcttgatata cttggatgat ggcatatgca gcagctatat gtggattttt ttagccctgc  1440
   cttcatacgc tatttatttg cttggtactg tttcttttgt cgatgctcac cctgttgttt  1500
   ggtgttactt ctgcaggtcg agggccccgg ggggcaataa gatatgaaaa agcctgaact 1560
   caccgcgacg tctgtcgaga agtttctgat cgaaaagttc gacagcgtct ccgacctgat 1620
   gcagctctcg gagggcgaag aatctcgtgc tttcagcttc gatgtaggag ggcgtggata 1680
   tgtcctgcgg gtaaatagct gcgccgatgg tttctacaaa gatcgttatg tttatcggca 1740
   ctttgcatcg gccgcgctcc cgattccgga agtgcttgac attggggaat tcagcgagag 1800
   cctgacctat tgcatctccc gccgtgcaca gggtgtcacg ttgcaagacc tgcctgaaac 1860
   cgaactgccc gctgttctgc agccggtcgc ggaggccatg gatgcgatcg ctgcggccga 1920
   tcttagccag acgagcgggt tcggcccatt cggaccgcaa ggaatcggtc aatacactac 1980
   atggcgtgat ttcatatgcg cgattgctga tccccatgtg tatcactggc aaactgtgat 2040
   ggacgacacc gtcagtgcgt ccgtcgcgca ggctctcgat gagctgatgc tttgggccga 2100
   ggactgcccc gaagtccggc acctcgtgca cgcggatttc ggctccaaca atgtcctgac 2160
   ggacaatggc cgcataacag cggtcattga ctggagcgag gcgatgttcg gggattccca 2220
   atacgaggtc gccaacatct tcttctggag gccgtggttg gcttgtatgg agcagcagac 2280
   gcgctacttc gagcggaggc atccggagct tgcaggatcg ccgcggctcc gggcgtatat 2340
   gctccgcatt ggtcttgacc aactctatca gagcttggtt gacggcaatt tcgatgatgc 2400
   agcttgggcg cagggtcgat gcgacgcaat cgtccgatcc ggagccggga ctgtcgggcg 2460
   tacacaaatc gcccgcagaa gcgcggccgt ctggaccgat ggctgtgtag aagtactcgc 2520
   cgatagtgga aaccgacgcc ccagcactcg tccgagggca aaggaataga gtagatgccg 2580
   accggagtcc gcaaaaatca ccagtctctc tctacaaatc tatctctctc tatttttctc 2640
   cagaataatg tgtgagtagt tcccagataa gggaattagg gttcttatag ggtttcgctc 2700
   atgtgttgag catataagaa acccttagta tgtatttgta tttgtaaaat acttctatca 2760
   ataaaatttc taattcctaa aaccaaaatc cagtgacctg caggcatgca agctgatcca 2820
   ctagaggcca tggcggccgc gtagactaga attcaacttc cgttgttcac gtattgtgga 2880
   taacaaggta ggatggccga gttggttatt gggtgtagtg actatttagt agaccgttct 2940
   gaaaataaaa atcagaagaa aaaaaaaccg gtgaaagcat tcacaactcg atcaaagcgg 3000
   agcgatcgag ccaactggcc tcaacgctcg atcgagcgag cgggacaccg accgaaggaa 3060
   cgctcgatcg agcgagcggg ccacggaccg aagggcgagc gggacaccga ccgaaggaac 3120
   gctcgatcga gcgagcgggc caccgaccga aggagagcga tcgtaatttt cctaacgggc 3180
   acacatgtgc cagttatccc tgtccatata tatttgcatg caaatgcaca cgtatccaag 3240
   atgtatatat atactgatct ctagtggggt actactatat aggacgtaca gtacggatta 3300
   gcagcattga ccagtccgtt gtttatttac taactagtac tagtgcataa attaaagcaa 3360
   ggaatccagc gcacgaacag aagcgcaagc ggactcctcg tgctgctgaa actgaaagcg 3420
   tatactactg gaagtagacg ggcagatgat cacccaccca cgggagggag gtctcttttc 3480
   ttgaacggaa tcatcaccgg cacggcacgg cacggcacgg cacacacccg cgcggagtgc 3540
   ataaacacct agtactgcgc acctagctta aactcggtag gcatactagt agctctcatt 3600
   gtttgcctcc ctgctgcggt ttttcaccga agttcatgcc agtccagcgt ttttgcagca 3660
   gaaaagccgc cgacttcggt ttgcggtcgc gagtgaagat ccctttcttg ttaccgccaa 3720
   cgcgcaatat gccttgcgag gtcgcaaaat cggcgaaatt ccatacctgt tcaccgacga 3780
   cggcgctgac gcgatcaaag acgcggtgat acatatccag ccatgcacac tgatactctt 3840
   cactccacat gtcggtgtac attgagtgca gcccggctaa cgtatccacg ccgtattcgg 3900
   tgatgataat cggctgatgc agtttctcct gccaggccag aagttctttt tccagtacct 3960
   tctctgccgt ttccaaatcg ccgctttgga cataccatcc gtaataacgg ttcaggcaca 4020
   gcacatcaaa gagatcgctg atggtatcgg tgtgagcgtc gcagaacatt acattgacgc 4080
   aggtgatcgg acgcgtcggg tcgagtttac gcgttgcttc cgccagtggc gcgaaatatt 4140
   cccgtgcacc ttgcggacgg gtatccggtt cgttggcaat actccacatc accacgcttg 4200
   ggtggttttt gtcacgcgct atcagctctt taatcgcctg taagtgcgct tgctgagttt 4260
   ccccgttgac tgcctcttcg ctgtacagtt ctttcggctt gttgcccgct tcgaaaccaa 4320
   tgcctaaaga gaggttaaag ccgacagcag cagtttcatc aatcaccacg atgccatgtt 4380
   catctgccca gtcgagcatc tcttcagcgt aagggtaatg cgaggtacgg taggagttgg 4440
   ccccaatcca gtccattaat gcgtggtcgt gcaccatcag cacgttatcg aatcctttgc 4500
   cacgcaagtc cgcatcttca tgacgaccaa agccagtaaa gtagaacggt ttgtggttaa 4560
   tcaggaactg ttcgcccttc actgccactg accggatgcc gacgcgaagc gggtagatat 4620
   cacactctgt ctggcttttg gctgtgacgc acagttcata gagataacct tcacccggtt 4680
   gccagaggtg cggattcacc acttgcaaag tcccgctagt gccttgtcca gttgcaacca 4740
   cctgttgatc cgcatcacgc agttcaacgc tgacatcacc attggccacc acctgccagt 4800
   caacagacgc gtggttacag tcttgcgcga catgcgtcac cacggtgata tcgtccaccc 4860
   aggtgttcgg cgtggtgtag agcattacgc tgcgatggat tccggcatag ttaaagaaat 4920
   catggaagta agactgcttt ttcttgccgt tttcgtcggt aatcaccatt cccggcggga 4980
   tagtctgcca gttcagttcg ttgttcacac aaacggtgat acgtacactt ttcccggcaa 5040
   taacatacgg cgtgacatcg gcttcaaatg gcgtatagcc gccctgatgc tccatcactt 5100
   cctgattatt gacccacact ttgccgtaat gagtgaccgc atcgaaacgc agcacgatac 5160
   gctggcctgc ccaacctttc ggtataaaga cttcgcgctg ataccagacg ttgcccgcat 5220
   aattacgaat atctgcatcg gcgaactgat cgttaaaact gcctggcaca gcaattgccc 5280
   ggctttcttg taacgcgctt tcccaccaac gctgatcaat tccacagttt tcgcgatcca 5340
   gactgaatgc ccacaggccg tcgagttttt tgatttcacg ggttggggtt tctacaggac 5400
   gtaacataag ggactgacca cccggggtgc ctggacggga aaccatggac ggacgttatt 5460
   cagcaggtcg aggtcagcat gtataacaag ttaatcaatt acaaaaatgg ggaggaaatg 5520
   aacggacacc gtccgacgac tgacctgagg cggcggcgtc gtggacggcc gtggctccct 5580
   gcaggaattc gatcagctcc tttgggtctt gttttctcct atattcctga gatgtttgtt 5640
   gggtgagtga tggttggaac atgctgggga tgtatgtttt tatataggca tggcgagcag 5700
   gtggaactca tgagtaatgg acatagtcaa gtggaaccgc ccaacggata gaaatggtct 5760
   ttggctgccg actagattag tctgcataaa cttttgagat gtggtcagcc atattcaaat 5820
   ctttgataca cgtttttccc tttaataaat agagaagcaa attaaggtac tccatgtgat 5880
   gtagacctct tagtaacaat ttctatgagc aatagttttg attgtgtcaa acaaggaaga 5940
   aatcatggcg cgtcgatcca tgcattaacg cacctaatac gtatcatatg attacgtcaa 6000
   ccagatatac agttgcctgg tcaaatttct ctccaaatgg ttgagtactt gaaggaagaa 6060
   gcaacgacat taattaatgg acgatggaga gcaagagcag ttaaaatttt cttgccaaag 6120
   aattttcgtc atgaccaatt cagttcataa atctaacaat ctagtcctga ctaaggttca 6180
   tgactagttt ttgccatgga tgttcaggtc catgcatctt acaacaacaa gcatggctga 6240
   tttacactcc aacaaggaag agcgggaagc tacctaccgc gaggaggagg agcgggcccg 6300
   ctgctggcgg gtgtagtccc agatggcgat ggcggcgctg acacggacgt tgagcgaccg 6360
   gacgacgccc agctgcggga tctcgacgca cacgtccacc gcctcctgga tgatgtccac 6420
   cgggatgccc tcctaactgc ccagcaccac catcgtcttc tccgggaacg agaactcgtc 6480
   cagggctggg cagtttcaag ccaacacgac aagatagctc aagttcaagt gcttacattc 6540
   agaaagttat ttactcgctc caaaaccgac actggaacac attcaaagct agacgactgc 6600
   ataaaaacaa ggcatattct ttattttttg cgagaaaaat aaggcatatt cagaattaca 6660
   acagaaaggc cgaaaggttc agtgccccat aaaaaacata actaccgccc tagagtcgtc 6720
   cgtcttattc atttcttcac aaaataaaca gtgtcctcaa atttctagga gacagagaat 6780
   acaacgaatc tcagtgcaag ttgtaaatat tcccgttgga gtcccagatg tattgggata 6840
   aaaaacatca agaaaacctt caattgaaac tttgagcctg taatcaaaac atatacggag 6900
   gatacaaaca ctcaagatgg aaatatttat aattaacacc tttctagatt aagagaagga 6960
   catcaagtaa ggtatatcgg tttcttttag agaaaactaa ggtatatata agtgtataac 7020
   ttgacactat tagttacgga agaaatctat gagtaaactc gtgatggtac acagagagaa 7080
   gttttatcac tgagagaagt atagcccaag tatgtgggct atactccagg aactaccgta 7140
   aactacttat tacatgcccc ggaaaggcag accacaacaa cgactcaagg aagcaacgtt 7200
   tgcacattca acttgcacgt tgaaacctct gagcacatta ctaaataatc atctattggc 7260
   ttctgaatag tattttgcat aaacttttca agtttgcttt ttaatatgct taagcgctaa 7320
   tgcatcatcc gatgtatggc tgagtagtaa ttacaattat ttatccatct caaccaagtc 7380
   agcacaatgc atggagcgct ggtttcatga aaagtcgcca cccatcatca tagtttatta 7440
   gatatcatat acgtatactt ggtcatggtg agaactgatg cacacgaggt caaagaggag 7500
   ttgagagatg taagttatgt aaagcaatcg ttttctacac agacaaagtc gtctgcttag 7560
   cttgatctcc tctcctgtaa gattatctcg tgtgtttgcg tgctatactt tggtgtagtt 7620
   ccgattgaga tttatctagc cggacggcag aatacatcag ttccatagct agctagccca 7680
   acaatcgatc caccagccag cttgcttgct tttagctcgt agcgtgcagg ttttgttcta 7740
   gtttcggtta tagttgtgtt ggcgtacgac ggcgatctga tctacggcaa gcggctagtt 7800
   ctaacactct ccattctttt aagttgctac agaatgtgtg caatcaacac tctccattta 7860
   cggtggtgtg ttcggaatgg tatggcgatc caatcgaccc tgccttaaat caaatgactc 7920
   acggtattgc atgcacatat cttgtagtac atgcaacaat gtcctccttt catcatgtct 7980
   gctggtatgg tcaagacact acttggttta agcagcgggt cgtctccgac atgcaactgt 8040
   agttcaaagg tgacagtacg acgaagtcag gtgttttctt ccaggtggct cgtggatgaa 8100
   tgtcgtcatc ggtcctggtt gagtcttaag gtggtccagc ttctcatctg ctcccttccg 8160
   ccagcatgct cgaggtgtgt cattaggcac ttccggtaat aagtttattt tctacattta 8220
   tgcatatttt ttatatgtat gttggagcta atgtttaaaa tctcgactcc gttgtgccca 8280
   gtttccaaaa cttagtaaaa aaacgtgttt ttagttccca acttaagaaa atcaaaaata 8340
   ctactcataa gaatttagat aaaatgagcc acctcatcga tttccatcaa agcagccgtc 8400
   aatcgaactt acaagagctt tgaaccaaaa ggaagctaaa aatattacat ggaggaactg 8460
   tatatttcta atttcatgca ccagaaagcg agagaccaga acaacaagtc caggaagcaa 8520
   cttttgcaca ttccacattc acatttaaat cctgagcaca atcgggctcc acccatcgca 8580
   tacttcacat gtattgccag ataataatac gtgttcaaca ccagcgtcaa acaaatactg 8640
   cattaccaaa taatcttgta ttgtttcttt tttttaacct aatcatatat tatttcagaa 8700
   taaggggaaa agaacccaaa atattgatca attatgagaa atacgacaca gtagaaccaa 8760
   aaaaaagcat gtgagatcat atcagtgtga taaaaaacaa tatttttgat tatttaaaag 8820
   gtagaaatac attcactgat tacaggcaca caattacata tgctcctttt attttataaa 8880
   ttaggatgca tatctaggaa ctcaatcatg cccttaattt acgcacgatc aagcttgaat 8940
   tcctgcagcc cgggggatcc actagtaagg ccttaagggc cagatcttgg gcccggtacc 9000
   cgatcagatt gtcgtttccc gccttcggtt taaactatca gtgtttgaca ggatatattg 9060
   gcgggtaaac ctaagagaaa agagcgttta ttagaataat cggatattta aaagggcgtg 9120
   aaaaggttta tccgttcgtc catttgtatg tgcatgccaa ccacagggtt cccctcggga 9180
   gtgcttggca ttccgtgcga taatgacttc tgttcaacca cccaaacgtc ggaaagcctg 9240
   acgacggagc agcattccaa aaagatccct tggctcgtct gggtcggcta gaaggtcgag 9300
   tgggctgctg tggcttgatc cctcaacgcg gtcgcggacg tagcgcagcg ccgaaaaatc 9360
   ctcgatcgca aatccgacgc tgtcgaaaag cgtgatctgc ttgtcgctct ttcggccgac 9420
   gtcctggcca gtcatcacgc gccaaagttc cgtcacagga tgatctggcg cgagttgctg 9480
   gatctcgcct tcaatccggg tctgtggcgg gaactccacg aaaatatccg aacgcagcaa 9540
   gatatcgcgg tgcatctcgg tcttgcctgg gcagtcgccg ccgacgccgt tgatgtggac 9600
   gccgaaaagg atctaggtga agatcctttt tgataatctc atgaccaaaa tcccttaacg 9660
   tgagttttcg ttccactgag cgtcagaccc cgtagaaaag atcaaaggat cttcttgaga 9720
   tccttttttt ctgcgcgtaa tctgctgctt gcaaacaaaa aaaccaccgc taccagcggt 9780
   ggtttgtttg ccggatcaag agctaccaac tctttttccg aaggtaactg gcttcagcag 9840
   agcgcagata ccaaatactg ttcttctagt gtagccgtag ttaggccacc acttcaagaa 9900
   ctctgtagca ccgcctacat acctcgctct gctaatcctg ttaccagtgg ctgctgccag 9960
   tggcgataag tcgtgtctta ccgggttgga ctcaagacga tagttaccgg ataaggcgca 10020
   gcggtcgggc tgaacggggg gttcgtgcac acagcccagc ttggagcgaa cgacctacac 10080
   cgaactgaga tacctacagc gtgagctatg agaaagcgcc acgcttcccg aagggagaaa 10140
   ggcggacagg tatccggtaa gcggcagggt cggaacagga gagcgcacga gggagcttcc 10200
   agggggaaac gcctggtatc tttatagtcc tgtcgggttt cgccacctct gacttgagcg 10260
   tcgatttttg tgatgctcgt caggggggcg gagcctatgg aaaaacgcca gcaacgcggc 10320
   ctttttacgg ttcctggcct tttgctggcc ttttgctcac atgttctttc ctgcgttatc 10380
   ccctgattct gtggataacc gattaccgcc tttgagtgag ctgataccgc tcgccgcagc 10440
   cgaacgaccg agcgcagcga gtcagtgagc gaggaagcgg aagagcgcct gatgcggtat 10500
   tttctcctta cgcatctgtg cggtatttca caccgcatat ggtgcactct cagtacaatc 10560
   tgctctgatg ccgcatagtt aagccagtat acactccgct atcgctacgt gactgggtca 10620
   tggctgcgcc ccgacacccg ccaacacccg ctgacgcgcc ctgacgggct tgtctgctcc 10680
   cggcatccgc ttacagacaa gctgtgaccg tctccgggag ctgcatgtgt cagaggtttt 10740
   caccgtcatc accgaaacgc gcgaggcagg ggtacgtcga ggtcgatcca acccctccgc 10800
   tgctatagtg cagtcggctt ctgacgttca gtgcagccgt cttctgaaaa cgacatgtcg 10860
   cacaagtcct aagttacgcg acaggctgcc gccctgccct tttcctggcg ttttcttgtc 10920
   gcgtgtttta gtcgcataaa gtagaatact tgcgactaga accggagaca ttacgccatg 10980
   aacaagagcg ccgccgctgg cctgctgggc tatgcccgcg tcagcaccga cgaccaggac 11040
   ttgaccaacc aacgggccga actgcacgcg gccggctgca ccaagctgtt ttccgagaag 11100
   atcaccggca ccaggcgcga ccgcccggag ctggccagga tgcttgacca cctacgccct 11160
   ggcgacgttg tgacagtgac caggctagac cgcctggccc gcagcacccg cgacctactg 11220
   gacattgccg agcgcatcca ggaggccggc gcgggcctgc gtagcctggc agagccgtgg 11280
   gccgacacca ccacgccggc cggccgcatg gtgttgaccg tgttcgccgg cattgccgag 11340
   ttcgagcgtt ccctaatcat cgaccgcacc cggagcgggc gcgaggccgc caaggcgcga 11400
   ggcgtgaagt ttggcccccg ccctaccctc accccggcac agatcgcgca cgcccgcgag 11460
   ctgatcgacc aggaaggccg caccgtgaaa gaggcggctg cactgcttgg cgtgcatcgc 11520
   tcgaccctgt accgcgcact tgagcgcagc gaggaagtga cgcccaccga ggccaggcgg 11580
   cgcggtgcct tccgtgagga cgcattgacc gaggccgacg ccctggcggc cgccgagaat 11640
   gaacgccaag aggaacaagc atgaaaccgc accaggacgg ccaggacgaa ccgtttttca 11700
   ttaccgaaga gatcgaggcg gagatgatcg cggccgggta cgtgttcgag ccgcccgcgc 11760
   acgtctcaac cgtgcggctg catgaaatcc tggccggttt gtctgatgcc aagctcgcgg 11820
   cctggccggc gagcttggcc gctgaagaaa ccgagcgccg ccgtctaaaa aggtgatgtg 11880
   tatttgagta aaacagcttg cgtcatgcgg tcgctgcgta tatgatgcga tgagtaaata 11940
   aacaaatacg caaggggaac gcatgaaggt tatcgctgta cttaaccaga aaggcgggtc 12000
   aggcaagacg accatcgcaa cccatctagc ccgcgccctg caactcgccg gggccgatgt 12060
   tctgttagtc gattccgatc cccagggcag tgcccgcgat tgggcggccg tgcgggaaga 12120
   tcaaccgcta accgttgtcg gcatcgaccg cccgacgatt gaccgcgacg tgaaggccat 12180
   cggccggcgc gacttcgtag tgatcgacgg agcgccccag gcggcggact tggctgtgtc 12240
   cgcgatcaag gcagccgact tcgtgctgat tccggtgcag ccaagccctt acgacatatg 12300
   ggccaccgcc gacctggtgg agctggttaa gcagcgcatt gaggtcacgg atggaaggct 12360
   acaagcggcc tttgtcgtgt cgcgggcgat caaaggcacg cgcatcggcg gtgaggttgc 12420
   cgaggcgctg gccgggtacg agctgcccat tcttgagtcc cgtatcacgc agcgcgtgag 12480
   ctacccaggc actgccgccg ccggcacaac cgttcttgaa tcagaacccg agggcgacgc 12540
   tgcccgcgag gtccaggcgc tggccgctga aattaaatca aaactcattt gagttaatga 12600
   ggtaaagaga aaatgagcaa aagcacaaac acgctaagtg ccggccgtcc gagcgcacgc 12660
   agcagcaagg ctgcaacgtt ggccagcctg gcagacacgc cagccatgaa gcgggtcaac 12720
   tttcagttgc cggcggagga tcacaccaag ctgaagatgt acgcggtacg ccaaggcaag 12780
   accattaccg agctgctatc tgaatacatc gcgcagctac cagagtaaat gagcaaatga 12840
   ataaatgagt agatgaattt tagcggctaa aggaggcggc atggaaaatc aagaacaacc 12900
   aggcaccgac gccgtggaat gccccatgtg tggaggaacg ggcggttggc caggcgtaag 12960
   cggctgggtt gtctgccggc cctgcaatgg cactggaacc cccaagcccg aggaatcggc 13020
   gtgagcggtc gcaaaccatc cggcccggta caaatcggcg cggcgctggg tgatgacctg 13080
   gtggagaagt tgaaggcggc gcaggccgcc cagcggcaac gcatcgaggc agaagcacgc 13140
   cccggtgaat cgtggcaagc ggccgctgat cgaatccgca aagaatcccg gcaaccgccg 13200
   gcagccggtg cgccgtcgat taggaagccg cccaagggcg acgagcaacc agattttttc 13260
   gttccgatgc tctatgacgt gggcacccgc gatagtcgca gcatcatgga cgtggccgtt 13320
   ttccgtctgt cgaagcgtga ccgacgagct ggcgaggtga tccgctacga gcttccagac 13380
   gggcacgtag aggtttccgc agggccggcc ggcatggcga gtgtgtggga ttacgacctg 13440
   gtactgatgg cggtttccca tctaaccgaa tccatgaacc gataccggga agggaaggga 13500
   gacaagcccg gccgcgtgtt ccgtccacac gttgcggacg tactcaagtt ctgccggcga 13560
   gccgatggcg gaaagcagaa agacgacctg gtagaaacct gcattcggtt aaacaccacg 13620
   cacgttgcca tgcagcgtac gaagaaggcc aagaacggcc gcctggtgac ggtatccgag 13680
   ggtgaagcct tgattagccg ctacaagatc gtaaagagcg aaaccgggcg gccggagtac 13740
   atcgagatcg agctagctga ttggatgtac cgcgagatca cagaaggcaa gaacccggac 13800
   gtgctgacgg ttcaccccga ttactttttg atcgatcccg gcatcggccg ttttctctac 13860
   cgcctggcac gccgcgccgc aggcaaggca gaagccagat ggttgttcaa gacgatctac 13920
   gaacgcagtg gcagcgccgg agagttcaag aagttctgtt tcaccgtgcg caagctgatc 13980
   gggtcaaatg acctgccgga gtacgatttg aaggaggagg cggggcaggc tggcccgatc 14040
   ctagtcatgc gctaccgcaa cctgatcgag ggcgaagcat ccgccggttc ctaatgtacg 14100
   gagcagatgc tagggcaaat tgccctagca ggggaaaaag gtcgaaaagg tctctttcct 14160
   gtggatagca cgtacattgg gaacccaaag ccgtacattg ggaaccggaa cccgtacatt 14220
   gggaacccaa agccgtacat tgggaaccgg tcacacatgt aagtgactga tataaaagag 14280
   aaaaaaggcg atttttccgc ctaaaactct ttaaaactta ttaaaactct taaaacccgc 14340
   ctggcctgtg cataactgtc tggccagcgc acagccgaag agctgcaaaa agcgcctacc 14400
   cttcggtcgc tgcgctccct acgccccgcc gcttcgcgtc ggcctatcgc ggccgctggc 14460
   cgctcaaaaa tggctggcct acggccaggc aatctaccag ggcgcggaca agccgcgccg 14520
   tcgccactcg accgccggcg cccacatcaa ggcaccggtg ggtatgcctg acgatgcgtg 14580
   gagaccgaaa ccttgcgctc gttcgccagc caggacagaa atgcctcgac ttcgctgctg 14640
   cccaaggttg ccgggtgacg cacaccgtgg aaacggatga aggcacgaac ccagtggaca 14700
   taagcctgtt cggttcgtaa gctgtaatgc aagtagcgta tgcgctcacg caactggtcc 14760
   agaaccttga ccgaacgcag cggtggtaac ggcgcagtgg cggttttcat ggcttgttat 14820
   gactgttttt ttggggtaca gtctatgcct cgggcatcca agcagcaagc gcgttacgcc 14880
   gtgggtcgat gtttgatgtt atggagcagc aacgatgtta cgcagcaggg cagtcgccct 14940
   aaaacaaagt taaacatcat gagggaagcg gtgatcgccg aagtatcgac tcaactatca 15000
   gaggtagttg gcgtcatcga gcgccatctc gaaccgacgt tgctggccgt acatttgtac 15060
   ggctccgcag tggatggcgg cctgaagcca cacagtgata ttgatttgct ggttacggtg 15120
   accgtaaggc ttgatgaaac aacgcggcga gctttgatca acgacctttt ggaaacttcg 15180
   gcttcccctg gagagagcga gattctccgc gctgtagaag tcaccattgt tgtgcacgac 15240
   gacatcattc cgtggcgtta tccagctaag cgcgaactgc aatttggaga atggcagcgc 15300
   aatgacattc ttgcaggtat cttcgagcca gccacgatcg acattgatct ggctatcttg 15360
   ctgacaaaag caagagaaca tagcgttgcc ttggtaggtc cagcggcgga ggaactcttt 15420
   gatccggttc ctgaacagga tctatttgag gcgctaaatg aaaccttaac gctatggaac 15480
   tcgccgcccg actgggctgg cgatgagcga aatgtagtgc ttacgttgtc ccgcatttgg 15540
   tacagcgcag taaccggcaa aatcgcgccg aaggatgtcg ctgccgactg ggcaatggag 15600
   cgcctgccgg cccagtatca gcccgtcata cttgaagcta gacaggctta tcttggacaa 15660
   gaagaagatc gcttggcctc gcgcgcagat cagttggaag aatttgtcca ctacgtgaaa 15720
   ggcgagatca ccaaggtagt cggcaaataa tgtctaacaa ttcgttcaag ccgacgccgc 15780
   ttcgcggcgc ggcttaactc aagcgttaga tgcactaagc acataattgc tcacagccaa 15840
   actatcaggt caagtctgct tttattattt ttaagcgtgc ataataagcc ctacacaaat 15900
   tgggagatat atcatgaaag gctggctttt tcttgttatc gcaatagttg gcgaagtaat  15960
   cgcaacatag cttgcttggt cgttccgcgt gaacgtcggc tcgattgtac ctgcgttcaa  16020
   atactttgcg atcgtgttgc gcgcctgccc ggtgcgtcgg ctgatctcac ggatcgactg  16080
   cttctctcgc aacgccatcc gacggatgat gtttaaaagt cccatgtgga tcactccgtt  16140
   gccccgtcgc tcaccgtgtt gggggGaagg tgcacatggc tcagttctca atggaaatta  16200
   tctgcctaac cggctcagtt ctgcgtagaa accaacatgc aagctccacc gggtgcaaag  16260
   cggcagcggc ggcaggatat attcaattgt aaatggcttc atgtccggga aatctacatg  16320
   gatcagcaat gagtatgatg gtcaatatgg agaaaaagaa agagtaatta ccaatttttt  16380
   ttcaattcaa aaatgtagat gtccgcagcg ttattataaa atgaaagtac attttgataa  16440
   aacgacaaat tacgatccgt cgtatttata ggcgaaagca ataaacaaat tattctaatt  16500
   cggaaatctt tatttcgacg tgtctacatt cacgtccaaa tgggggctta gatgagaaac  16560
   ttcacgatcg gc                                                      16572